# EUROPEAN PATENT SPECIFICATION

(11) **EP 3 236 970 B1**
(45) Date of publication and mention of the grant of the patent: **16.12.2020**
(21) Application number: 15828387.9
(22) Date of filing: 22.12.2015
(51) Int. Cl.: A61K 31/555, A61P 3/10, A61P 11/06, A61P 17/02, A61P 17/06, A61P 19/02, A61P 25/02, A61P 29/00, A61P 31/00, A61P 31/04, A61P 31/12, A61P 31/22, A61P 35/00, A61P 37/02, C07D 259/00

(54) **NEW METAL COMPLEXES OF NOCARDAMINE AND THEIR USE IN PHARMACEUTICAL COMPOSITIONS**
NEUE METALLKOMPLEXE VON NOCARDAMIN UND DEREN VERWENDUNG IN PHARMAZEUTISCHEN ZUSAMMENSETZUNGEN
NOUVEAUX COMPLEXES MÉTALLIQUES DE NOCARDAMINE ET LEUR UTILISATION DANS DES COMPOSITIONS PHARMACEUTIQUES

(30) Priority: 22.12.2014 US 201462095212 P
(43) Date of publication of application: 01.11.2017
(73) Proprietor: Chevion, Mordechai, 9076705 Mevassert Zion (IL); Vinokur, Vladimir, 8429001 Beer Sheva (IL); Eliashar, Ron, 9083600 Har-Adar (IL); Berenshtein, Eduard, 9757411 Jerusalem (IL)
(72) Inventor: Chevion, Mordechai, 9076705 Mevassert Zion (IL); Vinokur, Vladimir, 8429001 Beer Sheva (IL); Eliashar, Ron, 9083600 Har-Adar (IL); Berenshtein, Eduard, 9757411 Jerusalem (IL)
(74) Representative: Engstle, Verena
(86) International application number: PCT/IL2015/051243
(87) International publication number: WO 2016/103260

(56) References cited:
- WO-A1-2004/060490
- WO-A1-2008/101909
- WO-A2-2011/021203
- DE-A1-102010 028 742
- US-A- 5 051 523
- US-A1- 2008 085 866
- MILOS PETRIK ET AL: "Preclinical evaluation of twoGa-siderophores as potential radiopharmaceuticals forinfection imaging", EUROPEAN JOURNAL OF NUCLEAR MEDICINE AND MOLECULAR IMAGING, SPRINGER, BERLIN, DE, vol. 39, no. 7, 24 April 2012 (2012-04-24) , pages 1175-1183, XP035067508, ISSN: 1619-7089, DOI: 10.1007/S00259-012-2110-3
- FAULKNER K M ET AL: "Characterization of Mn(III) Complexes of Linear and Cyclic Desferrioxamines as Mimics of Superoxide Dismutase Activity", ARCHIVES OF BIOCHEMISTRY AND BIOPHYSICS, ACADEMIC PRESS, US, vol. 310, no. 2, 1 May 1994 (1994-05-01), pages 341-346, XP024752748, ISSN: 0003-9861, DOI: 10.1006/ABBI.1994.1176 [retrieved on 1994-05-01] cited in the application
- ALEXEY OBOLENSKY ET AL: "Zinc desferrioxamine attenuates retinal degeneration in the rd10 mouse model of retinitis pigmentosa", FREE RADICAL BIOLOGY AND MEDICINE, ELSEVIER INC, US, vol. 51, no. 8, 15 July 2011 (2011-07-15), pages 1482-1491, XP028327478, ISSN: 0891-5849, DOI: 10.1016/J.FREERADBIOMED.2011.07.014 [retrieved on 2011-07-23]
- DHUNGANA S ET AL.: J.BIOL.INORG.CHEM., vol. 6, 2001, pages 810-818,

## Description

### FIELD OF THE INVENTION

The invention relates to novel complexes of nocardamine (desferrioxamine E), with a metal or a combination of metals and uses thereof in at least one of modulating pro-and anti-inflammatory cytokines, acting as an anti-biotic and chelating of labile and redox-active iron, within and out of cells. More specifically, the invention provides compositions, methods and kits for treating conditions associated with inflammation, infection and iron overload.

### BACKGROUND REFERENCES

References considered to be relevant as background to the presently disclosed subject matter are listed below:
1. Ferrero-Milani L et al. Clin Exp Immunol. 2007; 147(2): 227-235
2. Andersen CJ. Nutrients. 2015; 7(9):7889-913
3. Diaz-Gonsalez F et al. Eur J Immunol. 2015; 45(3):679-86
4. Nohl H et al. Free. Radic. Biol. Med. 1991; 11(6):581-8
5. Konijn AM, Baillieres Clin. Haematol. 1994; 7(4):829-49
6. Goldoni P et al. J. Med. Microbiol. 1991; 34(2):113-8
7. Gutteridge JM et al. Biochem. J. 1979; 184(2):469-72
8. Dhungana S et al. J.Biol.Inorg.Chem. 2001; 6:810-818
9. Banin E et al. Proc. Natl. Acad. Sci. USA. 2008; 105(43): 16761-16766
10. Obolensky A et al. Free Radic. Biol. Med. 2011; 51(8):1482-91
11. US 5,075,469
12. WO 2011/021203
13. WO 2004060490
14. US 5,051,523
15 Faulkner KM et al. Arch Biochem Biophys. 1994; 310(2):341-6.
16 EP2129762
17 US 5,618,838
18 Meiwes et al. Appl Microbiol Biotechnol 1990 32:505-510
19 Goebeler M et al. Arch. Dermatol. Res. 1991; 283:246-250

Acknowledgement of the above references herein is not to be inferred as meaning that these are in any way relevant to the patentability of the presently disclosed subject matter.

### BACKGROUND OF THE INVENTION

Inflammation is a part of the complicated biological response of body tissues to stimuli, such as pathogens, damaged cells, or irritants. Inflammation is a protective response that involves immune cells, blood vessels, and molecular mediators (1). The purpose of inflammation is to eliminate the initial cause of cell injury, clear out necrotic cells and tissues, which had been damaged from the original insult and the inflammatory process, and to initiate tissue repair. The classical physiological signs of acute inflammation are pain, heat, redness, swelling, and loss of function. Inflammation is a generic response, and therefore it is considered as a mechanism of innate immunity. Inflammation can be classified as either acute or chronic. Acute inflammation is the initial response of the body to harmful stimuli and is achieved by increased mobilization of leukocytes, especially granulocytes, from the blood into the injured tissues. A series of biochemical events propagate and promote the inflammatory response, involving the local vascular system, the immune system, and various cells within the injured tissue. Chronic inflammation is a prolonged inflammation, which leads to a progressive shift in the type of cells present at the site of inflammation and is characterized by simultaneous destruction and healing of the tissue involved in the inflammatory process (2).

Many bioactive compounds carry anti-inflammatory properties, acting via various mechanisms. One of the mechanisms may involve direct interruption of cyclooxygenase activity, thereby counteracting production of pro-inflammatory molecules - the prostaglandins. Other bioactive molecules suppress the activity of pro-inflammatory cytokines or interfere with L-selectin function in neutrophils during the inflammatory response (3).

Compounds that modulate the immune response, specifically via modulation of pro- and anti-inflammatory cytokines and any other immuno-modulators are therefore valuable as tools for modulating an immune response and controlling related disorders.

Nocardamine - a siderophore (CAS # 26605-16-3), is involved in iron transport to bacterial, fungal and plant cells. Iron, is an essential element that plays key roles in biological systems. Iron is an abundant metal in the human body. In healthy adults the total amount of iron is 3 to 4 grams, most of which is located within hemoglobin - the oxygen transporting protein and the major protein component in red blood cells. A fraction of body iron is located in the muscle, as an oxygen binding protein - the myoglobin. About 1% of the body's iron is bound to a variety of iron-containing enzymes and a plethora of iron-dependent redox proteins including the proteins involved in respiration and electron transport. On the other hand, part of the "labile" and redox-active iron pool, serves as a key participant in the formation of reactive oxygen-derived species (ROS), including the hydroxyl radical via the Fenton reaction, thus catalyzing tissue-induced injury, such as the development of inflammatory conditions (4). Indeed, there is a direct association between excessive tissue iron and several pathological and inflammatory phenomena in different organs and tissues (5). Thus, iron sequestration may have bacteriostatic and bactericidal effects on pathogenic microorganisms (6, 7).

Iron is required for cellular proliferation, including microbial growth. In microorganisms iron acquisition is mediated by a family of low molecular weight ferric ion-selective ligands - the siderophores, shuttling the iron into the cell via a receptor-mediated mechanism, equivalent to cell-bound receptors and the associated intracellular utilization components.

The formation of complexes of siderophores with metal ions may contribute to increased solubility of the metals, and to the enhanced capacity of transition metal ions to pass across cellular membranes and infiltrate, as a complex, into cells and tissues. In addition, these transition metal ions could enhance anti-inflammatory and/or antibiotic activities (8, 9). Some metals display anti-inflammatory activity by themselves, however, a synergistic effect could be demonstrated when they are administered as a complex with a siderophore (10).

US 5,075,469 (11) concerns the pharmaceutical compositions used for treatment of iron-mediated damage. The compositions contain a zinc complex of desferrioxamine B or penicillamine, in treatment of ischemic damage, thallasaemia and haemochromatosis.

WO 2011/021203 (12), that is a previous publication of the inventors concerns the pharmaceutical compositions comprising DFO-B metal complexes, used for preventing, treating, ameliorating or inhibiting different immune-related disorders including asthma, diabetes mellitus types II and I, and psoriasis.

WO 2004/060490 (13) concerns the use of desferrioxamine B-metal complex for the topical treatment and/or prevention of damage to a tissue exposed to warfare agents, e.g. mustard gas-induced skin burns and damages.

US 5,051,523 (14) concerns formation of nocardamine (desferrioxamine E) complex with manganese and the radioactive isotope Ga-67. The latter is applicable to PET scan in cancer diagnostics and in localization of inflammatory process in sarcoidosis or tuberculosis, but not in treatment.

Manganese complex with nocardamine has been shown to mimic the superoxide dismutase activity. Such complex may be applied as a therapy for a line of diseases, presumably mediated by superoxide radical ion, but not affected by increased levels of hydrogen-peroxidase, which is a product of the dismutation reaction, e.g. ischemia-reperfusion injury and several other inflammatory processes (15).

EP2129762 (16) discloses detergents and cleaning agents comprising metal-siderophore complexes, spesifically, DFO-E metal complexes and their use as bleach catalysts.

Thus, there is a need to develop efficient anti-inflammatory compounds that may be applicable for a wide variety of diseases, the pathogenesis of those involves several modes of action of the novel metal-nocardamine complexes, as anti-inflammatory compounds, antibiotics and iron chelators.

### SUMMARY OF THE INVENTION

According to one aspect, the invention provides an effective amount of at least one complex of nocardamine (desferrioxamine E) with at least one metal, any combination/s of said complexes, any pharmaceutical composition/s of the complexes comprising any carrier/s, matrix or vehicle/s, for use in a method for modulating cytokine levels in a subject, wherein said subject is suffering from a disorder associated with at least one of elevated level/s of at least one pro-inflammatory cytokine and reduced level/s of at least one anti-inflammatory cytokine, and wherein said disorder is at least one of inflammatory, infectious, autoimmune, proliferative, ischemic, metabolic, neuro-degenerative disorder, spinal cord injury and trauma, and acute or chronic wound or injury, wherein said complex is a complex of nocardamine with Zn²⁺ion or a complex of nocardamine with Ga³⁺ion. Therefore, the invention provides a composition comprising at least one complex of nocardamine (desferrioxamine E) with at least one metal ion, thus Zn²⁺ or Ga³⁺. In yet some further embodiments, the composition of the invention may optionally further comprises at least one of diluent/s, carrier/s, excipient/s.

In another aspect of the invention said modulation is at least one of reduction or elevation of cytokine levels in said subject.

In yet another aspect of the invention said cytokine is at least one of: at least one pro-inflammatory cytokine and at least one anti-inflammatory cytokine.

In yet another aspect of the invention the complex or composition is used for at least one of:
(a) reducing the level of at least one pro-inflammatory cytokine in said subject, wherein said pro-inflammatory cytokine is at least one of IL-1α, IL-6, TNF-α and IL-17; and
(b) elevating the level of at least one anti-inflammatory cytokine in said subject, wherein said anti-inflammatory cytokine each independently is at least one of IL-13, IL-4, and IL-10.

In yet another aspect, the invention relates to a kit comprising: (i) at least one metal selected from zinc, gallium, aluminium, gold, cobalt, molybdenum, vanadium, or any combinations of said complexes, in any form of salts, esters and amides thereof, or a pharmaceutically acceptable derivative thereof and a pharmaceutically acceptable carrier or diluent, wherein at least one of said metals is a zinc ion (Zn²⁺); and (ii) nocardamine.

These and other aspects of the invention will become apparent as the description proceeds.

The scope of the invention is defined by the claims. Any subject-matter falling outside the scope of the claims is provided for information purposes only. Any references in the description to methods of treatment refer to the compounds, pharmaceutical compositions or medicaments of the present invention for use in a method of treatment of the human (or animal) body.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:

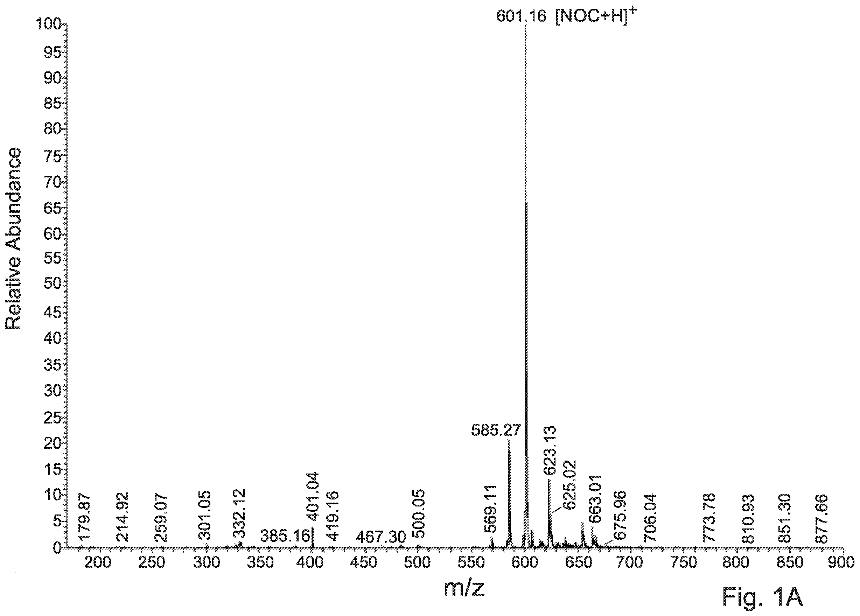

### Figure 1A-1C. Comparative mass-spectrometric analysis of nocardamine, zinc-nocardamine complex and gallium-nocardamine complex

**Figure 1A** shows mass-spectra of nocardamine.

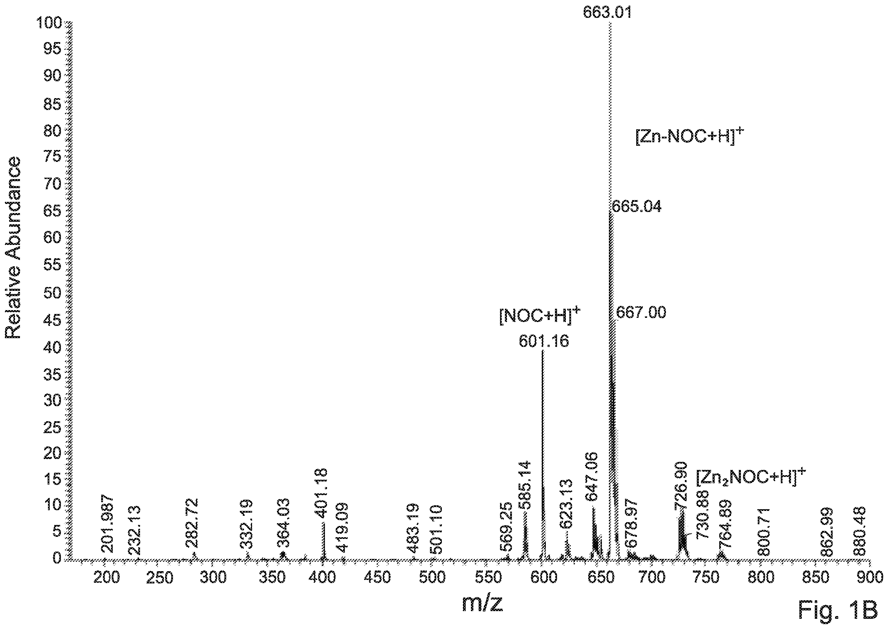

**Figure 1B** shows mass-spectrum of zinc-nocardamine complex.
   Zinc-nocardamine complex, prepared and purified to 89.8% as outlined in experimental procedures, was studied using mass spectroscopy. After direct injection (Ion source: electrospray ionization (positive); vaporizer temperature: 200°C; capillary temperature 100°C; flow: 0.005 ml/min (100% water); injection volume: 5 µl), a representative spectrum is shown.

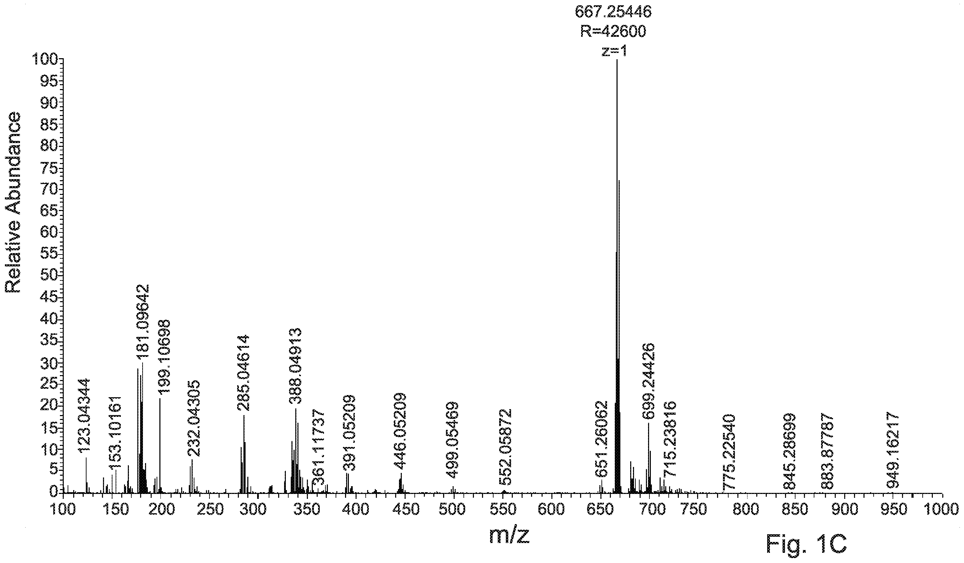

**Figure 1C** shows mass-spectrum of gallium-nocardamine complex.
   Gallium-nocardamine complex, was prepared from Gallium Chloride (>99.999% pure) solution (according to the manufacturer detailed information) and nocardamine (>98% pure) as outlined in experimental procedures. The complex was studied using mass spectroscopy. After direct injection (Ion source: electrospray ionization (positive); vaporizer temperature: 200°C; capillary temperature 100°C; flow: 0.005 ml/min (100% water); injection volume: 5 µl), a representative spectrum is shown.
**Figure 2A-2B****. Treatment of croton oil-induced irritant contact dermatitis (ICD) model in mice ears**
**Figure 2A**. Two groups of mice were used, both were exposed to croton oil. At 3 hours after the exposure one group was treated with ointment containing Vaseline-only (vehicle only), while the second group was not treated. The Figure shows the delta in ear thickness over time after the exposure to the irritant. Means ± SEM are shown.
**Figure 2B**. Four groups of mice were used. Three were treated with ointments containing either nocardamine (0.5%) (Group 1), zinc-nocardamine (0.5%) (Group 2), or betamethasone valerate (0.1%) (Group 3), as compared to non treated mice (Group 4). Means ± SEM are shown.
**Figure 3****. Treatment of murine model of ICD with zinc-nocardamine ointment: a dose-response**
   Balb/c female mice at 12 weeks of age were used. Thirty two mice were divided into 4 groups, eight mice per group as following: Group 1 - untreated control; Group 2 - treated with zinc-nocardamine 0.1%; Group 3 - treated with zinc-nocardamine 0.2%; Group 4 - treated with zinc-nocardamine 0.5%. The fingertip of the respective ointment was applied on the exposed ears at 3 hours after the exposure to the irritant. Means ± SEM are shown.

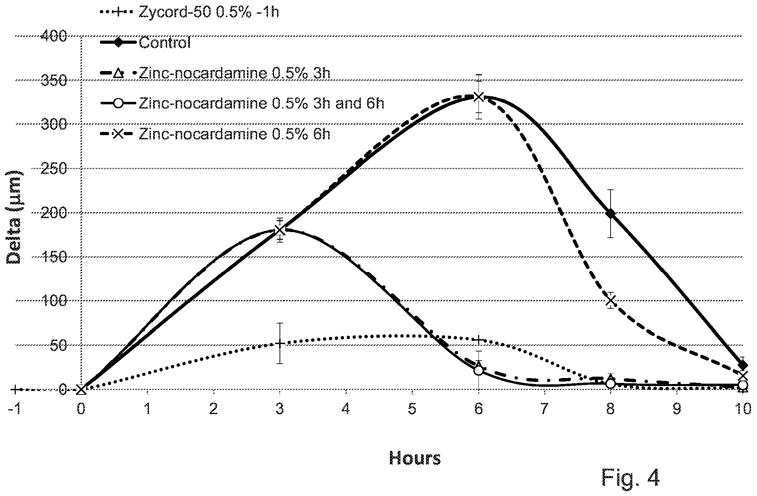

**Figure 4****. Treatment with or prevention of inflammatory process by zinc-nocardamine complex-containing ointment using the murine model of ICD**
   Balb/c female mice at 12 weeks of age were used. Forty mice were divided into 5 groups, eight mice per group; Group 1 - mice treated with zinc-nocardamine (0.5% ointment) at 1 hour prior to the exposure to the irritant; Group 2 - untreated control; Group 3 - treated with zinc-nocardamine (0.5% ointment) at 3 hours after the exposure to the irritant; Group 4 - treated with zinc-nocardamine (0.5% ointment) at 3 and 6 hours after the exposure to the irritant; Group 5 - treated with zinc-nocardamine (0.5% ointment) at 6 hours after the exposure to the irritant. Means ± SEM are shown.

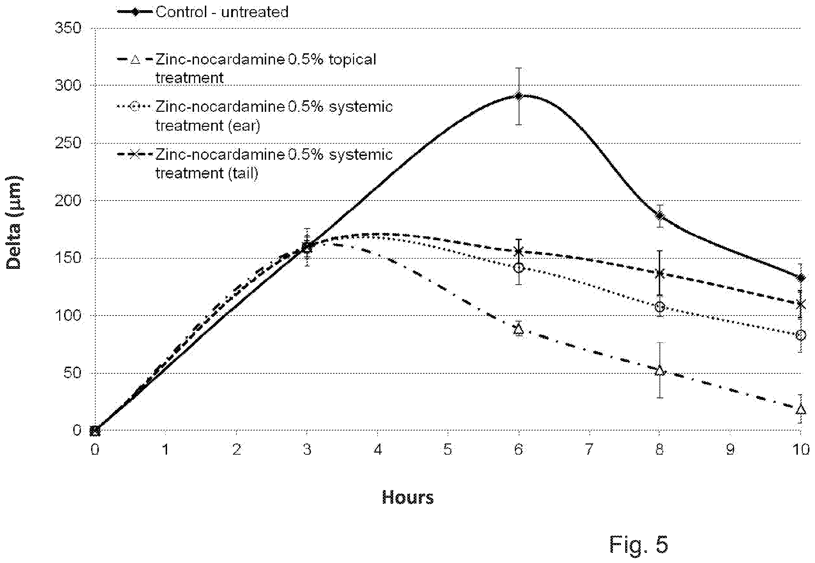

**Figure 5****. Systemic effect of topically applied zinc-nocardamine on murine model of ICD**
   Balb/c female mice at 12 weeks of age were used. Twenty mice were divided into 4 groups, five mice per group as following: Group 1 - untreated control; Group 2 - the right ear was exposed to croton oil and treated with zinc-nocardamine 0.5% at 3 hours after the exposure; Group 3 - both ears were exposed to croton oil, and only the right ear was treated with zinc-nocardamine 0.5% at 3 hours after the exposure; Group 4 - the right ear was exposed to croton oil, and a fingertip of zinc-nocardamine 0.5% ointment was applied on the middle part of the tail at 3 hours after the exposure. Means ± SEM are shown.

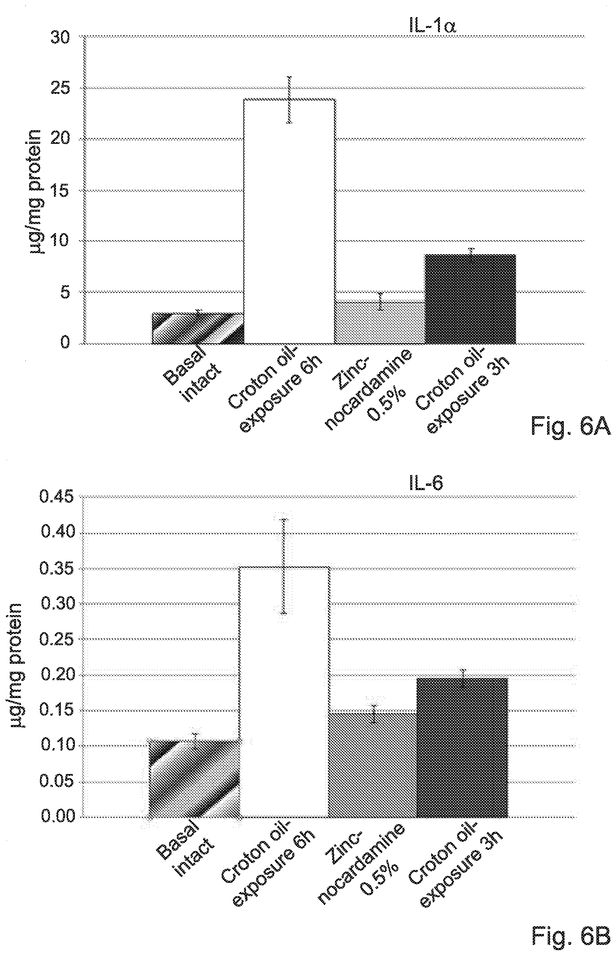

**Figure 6A-6D****. Effect of treatment with zinc-nocardamine of ICD on the levels of pro-inflammatory cytokines**
**Figure 6A** shows the levels of IL-1α in ears of mice exposed to croton oil.
**Figure 6B** shows the levels of IL-6 in ears of mice exposed to croton oil.

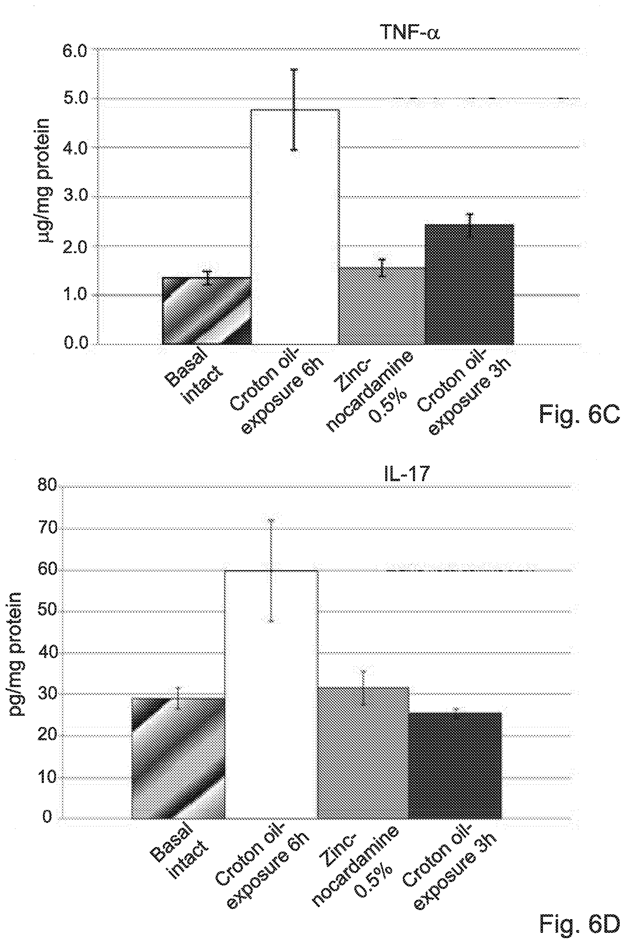

**Figure 6C** shows the levels of TNF-α in ears of mice exposed to croton oil.
**Figure 6D** shows the levels of IL-17 in ears of mice exposed to croton oil.
   Means ± SEM are shown.
   The left bar in each panel of the Figure (6A - 6D) shows the level of the corresponding cytokine before the exposure to the irritant (t = 0), the second bar from the left shows the level of the corresponding cytokine at t = 6 h after the exposure to the irritant. The second bar from the right shows the level of the corresponding cytokine at t =6 h after the exposure to the irritant, but these ears were treated with the ointment, once, at t = 3 h after the exposure to the irritant. The right bar shows the level of the corresponding cytokine at t = 3 h after the exposure to the irritant.

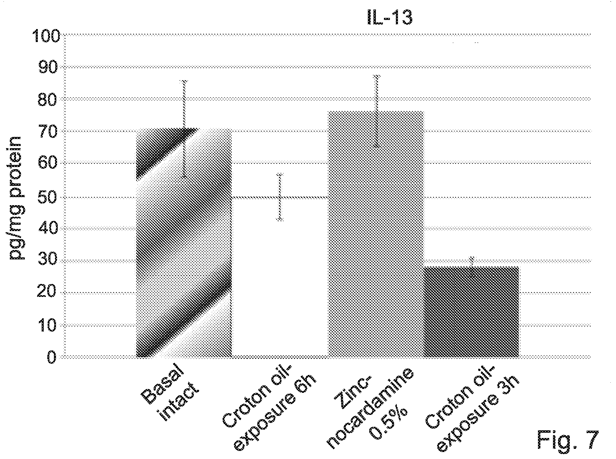

**Figure 7****. Effect of zinc-nocardamine treatment of ICD on the level of anti-inflammatory cytokine IL-13**
   The left bar shows the level of IL-13 before the exposure to the irritant (t = 0), the second bar from the left shows the level of the IL-13 at t = 6 h after the exposure to the irritant. The second bar from the right shows the level of IL-13 at t = 6 h after the exposure to the irritant (while the treatment with the complex of the invention was conducted at t = 3 h after the exposure. The right bar shows the level of IL-13 at t = 3 h after the exposure to the irritant (without treatment). Means ± SEM are shown.

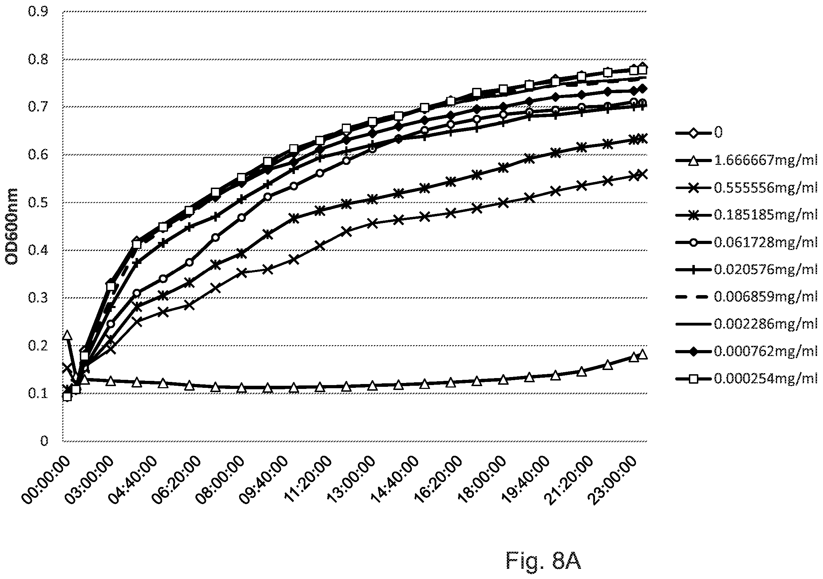

**Figure 8A-8C****: Anti-bacterial effect of zinc-nocardamine complex on bacterial growth**
**Figure 8A** shows the effect of zinc-nocardamine on a pathogenic *Escherichia coli.*

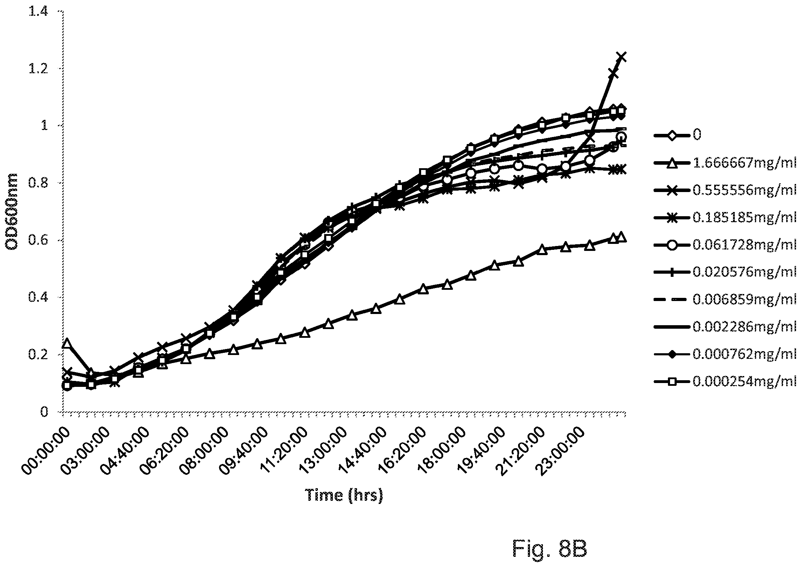

**Figure 8B** shows the effect of zinc-nocardamine on *Pseudomonas aeruginosa.*

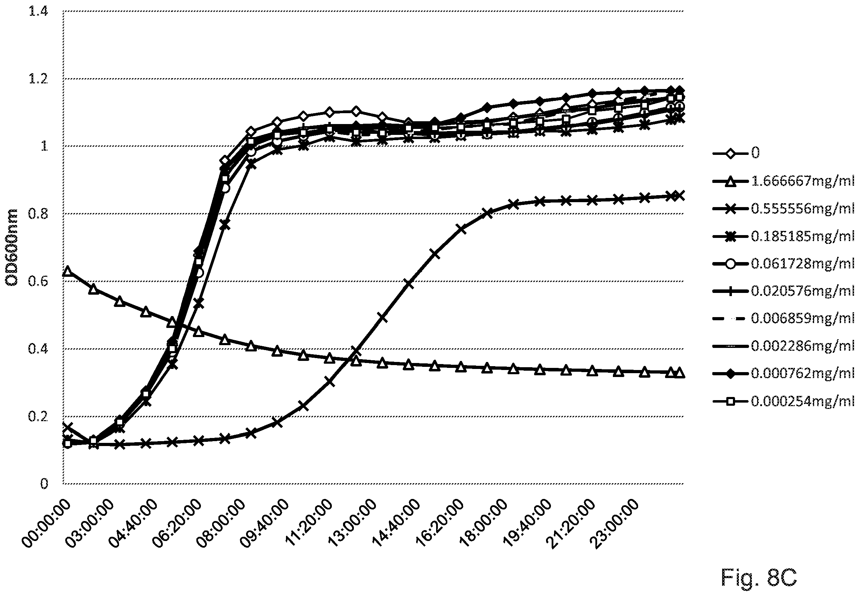

**Figure 8C** shows the effect of zinc-nocardamine on *Staphylococcus aureus.*

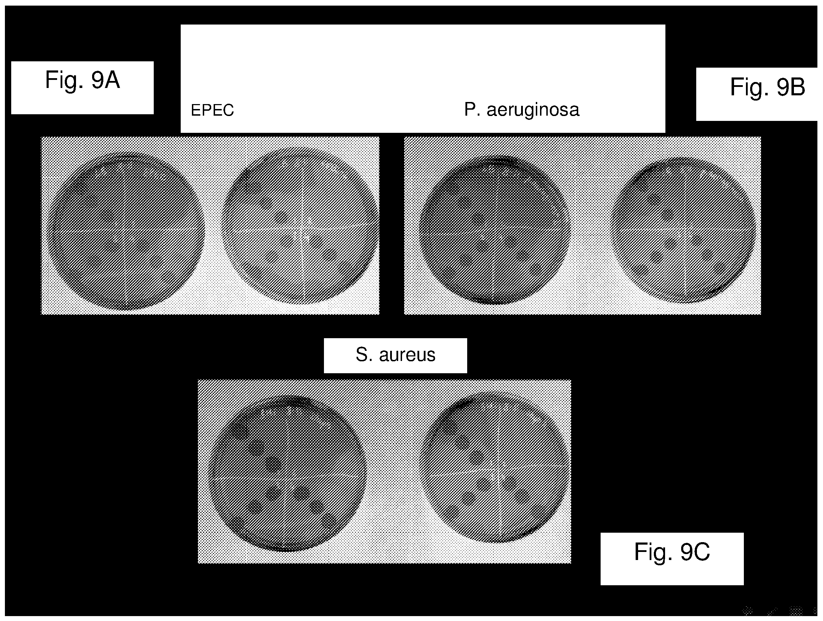

**Figure 9A-9C****. Bactericidal activity of zinc-nocardamine on** *Escherichia coli,* ***Pseudomonas aeruginosa,* and *Staphylococcus aureus***
   Bactericidal activity of the **zinc-nocardamine** complex was evaluated for the following bacteria: **Figure 9A****.** *E. coli;* **Figure 9B****.** *P. aeruginosa;* and **Figure 9C****.** *S. aureus* grown on Petri dishes with appropriate medium.
   The concentrations of zinc-nocardamine, in four quadrants (1 - 4) of a Petri dish, were set as follows: 1 - 0 mg/ml; 2 - 5 mg/ml; 3 - 1.7 mg/ml; 4 - 0.5 mg/ml.

### DETAILED DESCRIPTION OF THE INVENTION

The inventors report herein, for the first time, that a complex of a siderophore (nocardamine) with zinc ions exhibits high anti-inflammatory activity. They also reveal the mechanism underlying this activity. As shown in Examples 2-7, this anti-inflammatory complex was tested in irritant contact dermatitis (ICD) model, where local inflammatory response is elicited by application of croton oil on a mouse ear. Treatment with a fingertip of ointment containing the relatively small amount of zinc-nocardamine complex, rapidly diminishes the inflammation generated in the ICD model.

Although the anti-inflammatory effect of metal-desferrioxamine B complexes was previously shown by the present inventors (12), the anti-inflammatory effect of metal-nocardamine complexes as shown by the present invention is unexpected. More specifically, it was surprisingly found by the inventors that a metal complex of nocardamine may rapidly penetrate into cells, in contrast with the teachings of Dhungana et al. (8). The crystal structures of the ferric complexes of desferrioxamine B and nocardamine were compared to each other. In the desferrioxamine B complex a pendant pentane chain with protonated primary amino group at an end of the molecule was found in its coiled structure. Such a chain cannot be detected in iron-nocardamine, due to its closed cyclic structure and the lack of primary amino group. Also, this chain was found to be pointing away from the amide connecting ring and facing the surface of the molecule, that contains carbonyl groups. Lack of this feature in nocardamine is expected to impair membrane transport processes (8). Therefore, the fact that nocardamine efficiently penetrates to cells is unexpected. Thus, nocardamine should not have been chosen for the preparation of a drug which infiltrates into cells, exhibiting properties of prompt chelation of intracellular iron activity, anti-biotic activity and anti-inflammatory activity. Still further, desferrioxamine B is a highly hydrophilic molecule with water solubility of about 50 mg/ml. On the other hand, nocardamine is much more lipophilic compound, described as soluble in water with heating. There are other siderophores that carry chemical properties that are more similar to those of desferrioxamine B. Thus the choice of nocardamine is against the natural choice of a scholar in the field. Furthermore, nocardamine, unlike desferrioxamine B, was reported to change the morphology of insect *Bombyx mori* (silkworm) ovary-derived cultured cell line cells into spindle-like configuration. These cells with altered configuration remained attached to the culture flask much stronger than the normal ones. The mechanism of the alteration remained unclear, but this feature in ovarian cells has been interpreted as an indicator of neoplastic process, leading in turn, to ovarian cancer. In the light of these reported risks of nocardamine, the safe use and anti-cancerous application of the metal-nocardamine complexes of the invention are surprising. Moreover, as disclosed in Examples 7, the metal-nocardamine complexes of the invention clearly demonstrate an unexpected anti-bacterial effect. More specifically, in a number of previous publications, the present inventors have shown that a clear structural change in the spatial structure of desferrioxamine B, from a linear structure in its apo-state, into a ball-shaped, coiled structure occurs upon the binding of a metal ion (e.g., ferric iron, gallium and zinc). This change upon metal binding plays a pivotal role in facilitating the intracellular entrance of this complex and thereby providing its anti-microbial effect (17). While the linear structure of the (apo-) siderophore is not recognized by the siderophore receptors of target microorganism, the more spherical zinc-siderophore complex which partially mimics the iron-bound form siderophore, binds to the receptor, is being imported into the microorganism cell, but it does not release the (apo-) siderophore from its zinc complex, thus, poisons the iron import machinery of the organism resulting in a bacteriostatic and bactericidal effects. Therefore, the effective anti-microbial properties of the metal-nocardamine complexes of the invention are unexpected. Also, the present invention now surprisingly shows an unexpected anti-inflammatory effect of the metal-nocardamine complexes.
Thus, in a first aspect, the present disclosure provides compositions comprising at least one metal-chelating complex and specifically a complex of a cyclic siderophore, desferrioxamine E (also denoted as nocardamine or DFO-E), with a metal. In some optional embodiments, the compositions of the invention may further comprise at least one of diluent/s, carrier/s and excipient/s. It should be appreciated that in certain embodiments, the compositions of the invention may comprise several different metal-DFO E complexes or any combinations thereof.
The term *"chelation"* relates to a particular way, by which ions and molecules bind metal ions. According to the International Union of Pure and Applied Chemistry (IUPAC), chelation involves the formation or presence of two or more separate coordinate bonds between a polydentate (multiple bonded) ligand and a single central atom. Usually these ligands are organic compounds, and are called *chelants, chelators, chelating agents,* or *sequestering agents.* The chelate effect describes the enhanced affinity of chelating ligands for a metal ion compared to the affinity of a collection of similar nonchelating (monodentate) ligands for the same metal.

Nocardamine is a siderophore (CAS# 26605-16-3) that is synthesized by several microorganisms, including the generally recognized as safe (GRAS) bacterium *Streptomyces olivaceus.* Nocardamine is a cyclic trihydroxamate molecule with spacers of -(CH₂)₅- and -(CH₂)₂- between the individual hydroxamate groups. Nocardamine interacts non-covalently with transition metals and post-transition metals, forming complexes of a stoichiometry of 1:1. The ferric (Fe³⁺) complex shows a very high stability constant, and is structurally characterized by a distorted octahedron.

In yet some specific embodiments, the nocardamine of the compositions of the invention is as presented in the structure of Formula I:

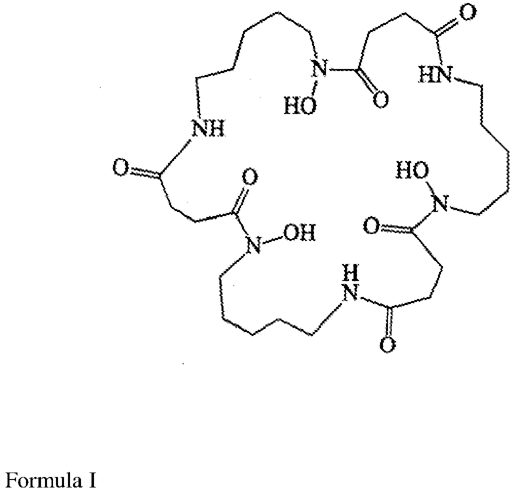

As detailed herein, nocardamine is a chelating agent. It comprises several hydroxamic acid groups, which deprotonate to hydroxamates that bind to metal ion to form a complex.

As used herein the term ***"complex"*** (also known as ***complex ion*** or ***coordination complexes***) refers to a chemical compound composed of a ligand having multiple binding sites (multidentate ligand) and a metal ion. The ligand being a chelating agent is in association with at least one metal ion.

As used herein, the term ***"association"*** refers to the chemical or physical force which holds two entities together (i.e. the ligand and the metal ion). Such force may be any type of chemical or physical bonding interaction known to a person skilled in the art. Non-limiting examples of such association interactions are ionic bonding, covalent bonding, coordination bonding, complexation, hydrogen bonding, van der Waals bonding, hydrophobicity-hydrophilicity interactions, etc.

In some embodiments, the association may be via covalent bonding. In other specific embodiments, the association may be via coordinative bonding. As used herein the term *coordinative bonding* or *coordinate bond* refers to a type of covalent bond in which two shared electrons originate from the same atom (known as dative bond). In the context of the present disclosure, the association between the ligand and the metal comprises association via multiple atoms in the ligand to several sites on the metal. Thus, the association may be viewed as comprising more than one bond, at times two covalent bonds, at times one covalent bond and one coordinate bond, at times two coordinate bonds. The number of bonds is known as the complex's coordination number. The bonds may include single (sigma bond) and/or double (pi) bond and may be dipolar bonds.

It should be understood to a person skilled in the art that in some cases the associative interactions between two atoms or two chemical entities may involve more than one type of chemical and/or physical interactions.

When referring to a *metal* it is to be understood as including any one or more of the elements commonly known as, transition metals, post transition metal/s, lanthanide/s, actinide/s and metalloids.

In some embodiments, the metal may act as a Lewis acid by accepting electron pairs from a donor Lewis bases (i.e. a chelating agent), thus forming a complex or complex ions. It should be noted that the donating electrons may be viewed as an electron pair donated into an empty metal orbital. In the context of the present disclosure, the metal may be referred as a "central metal".

At least one metal is selected from the group consisting of zinc, gallium, aluminum, silver, gold, cobalt, molybdenum, vanadium, including the vanadyl group, or any ionic forms thereof. The metal may be found in the human body, namely may be an endogenous, non-toxic metal.

In accordance with the present invention, at least one metal is a metal ion selected from the group consisting of Zn²⁺, Ga³⁺. In accordance with the present disclosure, at least one metal ion may be selected from Al³⁺, Ag¹⁺, Au³⁺, Au¹⁺, Co³⁺, V⁴⁺, V⁵⁺, [VO]²⁺, Mo⁴⁺, Mo⁶⁺.

As detailed herein, upon administration of the complex into a tissue or an organism, since the association between the ligand and the metal is reversible, the metal ion may be replaced by a different metal ion from an endogenous source and in turn may bind to another cellular component or could be released into the circulation in a ligand- free/unbound form. When referring to a ligand-free/unbound form it should be noted that the metal ion is free from or unbound to the ligand (i.e. nocardamine).

It was suggested, that the release of the metal ion from the complex and the concomitant replacement by a different metal ion is derived from the differences in the dissociation constant of each one of the two metal ions from the ligand (i.e. nocardamine). In other words, replacement will be favorable in case the differences in the dissociation constants are such that dissociation of the complex and release of the metal ion is thermodynamically favorable as the association constant of the 'new' metal with the ligand (i.e., nocardamine) is higher than for the original metal ion. Without being bound by theory, it was suggested that a complex of desferrioxamine E and Zn²⁺ will have the tendency to release the metal ion (i.e. Zn²⁺) and to bind Fe³⁺. In some embodiments, the free unbound form of the metal ion may have a physiological effect, for example an antioxidant effect. It is to be understood that the designations nocardamine, desferrioxamine E or DFO E, relate to the same compound, and will be used interchanging throughout the document. In some further embodiments, this compound is disclosed by Formula I, herein before.

In some other embodiments, the metal ion is a redox inert metal. In other words, the metal ion does not participate in oxidation-reduction reactions in the body.

In some embodiments the at least one metal ion is Zn²⁺. In yet some further embodiments the at least one metal ion is Ga³⁺.

Still further, the composition of the invention may comprise at least one complex of nocardamine with Zn²⁺. In some specific embodiments, the complex of the compositions of the invention may have the structure as presented by Formula II or similar, where any two hydroxamate groups (out of the 3 present in nocardamine) are bound to the zinc metal.

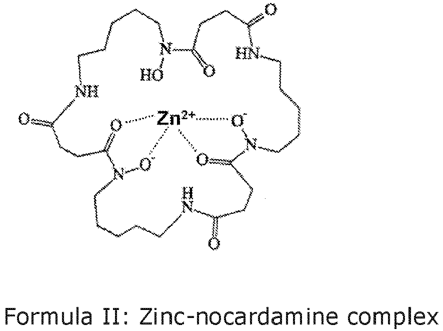

In yet some other embodiments, the composition of the invention may comprise at least one complex of nocardamine with Ga³⁺. Still further, in certain embodiments, the complex of nocardamine with Ga³⁺ may be presented by Formula III. It should be noted that Formula III shows the structure of gallium-nocardamine complex, which is similar to the corresponding iron complex, where the 3 hydroxamate groups are bound to the metal ion.

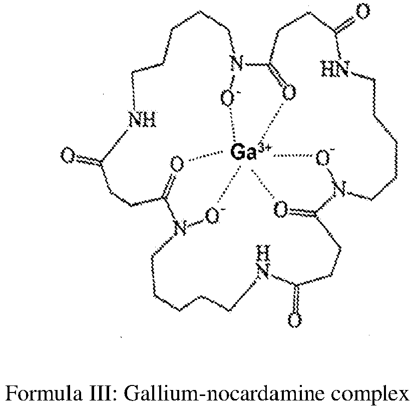

It should be appreciated that the composition of the invention may comprise at least one complex of metal- nocardamine. In yet some other embodiments, the compositions of the invention may comprise at least 2, 3, 4, 5, 6, 7, 8, 9, 10, 20, 30, 50, 50, 60, 70, 80, 90, 100 or more complexes as described by the invention. Still further, it must be appreciated that these complexes or any combinations thereof may be presented in the compositions of the invention at any ratio, for example, 1:1, to 0,0001-100,000 or more.

In yet some further embodiments, the compositions of the invention may comprise at least one complex of nocardamine with Ga³⁺ and at least one complex of nocardamine with Zn²⁺. It should be noted that in certain embodiments such combinations may exist in said composition in any ratio, for example a ratio ranging between about 1:1 to 0.0001:100,000 to 100,000:0.0001. More specifically, 0.1:1, 0.2:1, 0.3:1, 0.4:1, 0.5:1, 0.6:1, 0.7:1, 0.8:1, 0.9:1, 1:1, 1:2, 1:3, 1:4, 1:5, 1:6, 1:7, 1:8, 1:9, 1:10, or more.

By one embodiment either the metal ion or the nocardamine are in the form of a salt with an acid, and/or the complex comprises an acid, in addition to the siderophores and the metal ion. The acid may be selected from non limiting examples, being 1-hydroxy-2-naphthoic acid, 2,2-dichloroacetic acid, 2-oxoglutaric acid, 4-acetamidobenzoic acid, 4-aminosalicylic acid, acetic acid, alkanesulphonic acid, alkenesulphonic acid, alkynesulphonic acid with any substitutions, adipic acid, ascorbic acid, aspartic acid, benzoic acid, camphoric acid, camphor-10-sulfonic acid, decanoic acid, hexanoic acid, octanoic acid, carbonic acid, cinnamic acid, citric acid, cyclamic acid, dodecylsulfuric acid, formic acid, fumaric acid, galactaric acid, gentisic acid, glucoheptonic acid, gluconic acid, glucuronic acid, glutamic acid, glutaric acid, glycerophosphoric acid, glycolic acid, hippuric acid, hydrobromic acid, hydrochloric acid, isobutyric acid, lactic acid, lactobionic acid, lauric acid, maleic acid, malic acid, malonic acid, mandelic acid, methanesulfonic acid or its derivates, or a substituted hydrocarbyl sulphonic acid, for example a hydroxy-, alkoxy-, acyloxy-, alkoxycarbonyl-, halogen-, aromatic- or amino-substituted alkylsulphonic acid, nicotinic acid, nitric acid, oleic acid, oxalic acid, palmitic acid, pamoic acid, phosphoric acid, propionic acid, pyroglutamic acid, salicylic acid, sebacic acid, seleninic acid, selenonic acid, or any seleninic or selenonic analogue of the previously mentioned sulfonic compounds, stearic acid, succinic acid, sulfuric acid, tartaric acid, thiocyanic acid, undecylenic acid.

Metal-nocardamine complexes can be applied as is, or conjugated with sugars, starches, amino-acids, polyethylene-glycol, or polyglycerol-based compounds.

Metal-nocardamine complexes can be applied as is, or conjugated with hydrazines, hydroxylamines, amines, halides, aliphatic, aromatic, heterocyclic compounds or any other pharmaceutically acceptable groups.

In another embodiment, the composition of the invention may be formulated in a pharmaceutical composition. More specifically, the composition of the invention comprises as an active ingredient at least one of the metal-nocardamine complexes of the invention as described above, or any combinations thereof, and at least one pharmaceutically acceptable carrier/s, diluent/s, excipient/s.

As used herein *"pharmaceutically acceptable carrier"* includes any and all solvents, dispersion media, coatings and the like. The use of such media and agents for pharmaceutical active substances is well known in the art. Except as any conventional media or agent is incompatible with the active ingredient, its use in the therapeutic composition is contemplated.

In some embodiments the pharmaceutical composition of the invention is suitable for systemic administration. The pharmaceutical composition of the invention can be administered and dosed by the methods of the invention, in accordance with good medical practice. More specifically, the compositions used in the methods and kits of the invention, described herein after, may be adapted for administration by systemic, parenteral, intraperitoneal, transdermal, oral (including buccal or sublingual), rectal, topical (including buccal or sublingual), vaginal, intranasal and any other appropriate routes. Such formulations may be prepared by any method known in the art of pharmacy, for example by bringing into association the active ingredient with the carrier(s) or excipient(s).

The phrases *"systemic administration", "administered systemically"* as used herein mean the administration of a compound, drug or other material other than directly into the central blood system, such that it enters the patient's system and, thus, is subject to metabolism and other like processes. The phrases *"parenteral administration"* and *"administered parenterally"* as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

Systemic administration includes parenteral injection by intravenous bolus injection, by intravenous infusion, by sub-cutaneous, intramuscular, intraperitoneal injections or by suppositories, by patches, or by any other clinically accepted method, including tablets, pills, lozenges, pastilles, capsules, drinkable preparations, ointment, cream, paste, encapsulated gel, patches, boluses, or sprayable aerosol or vapors containing these complexes and combinations thereof, when applied in an acceptable carrier. Alternatively, to any pulmonary delivery as by oral inhalation such as by using liquid nebulizers, aerosol-based metered dose inhalers (MDI's), or dry powder dispersion devices.

In other embodiments the pharmaceutical composition is adapted for topical administration. By *"topical administration"* it is meant that the pharmaceutical composition and the carrier may be adapted to any mode of topical administration including: epicutaneous, oral, bronchoalveolar lavage, ophtalmic administration, enema, nasal administration, administration to the ear, administration by inhalation.

Regardless of the route of administration selected, the compounds of the present invention, which may be used in a suitable hydrated form, and/or the pharmaceutical compositions of the present invention, are formulated into pharmaceutically-acceptable dosage forms by conventional methods known to those of skill in the art.

Pharmaceutical compositions used to treat subjects in need thereof according to the invention generally comprise a buffering agent, an agent who adjusts the osmolarity thereof, and optionally, one or more pharmaceutically acceptable carriers, excipients and/or additives as known in the art. Supplementary active ingredients can also be incorporated into the compositions. The carrier can be solvent or dispersion medium containing, for example, water, ethanol, and suitable mixtures thereof. The proper fluidity can be maintained, for example, by the use of a coating, such as lecithin, by the maintenance of the required particle size in the case of dispersion and by the use of surfactants.

In some embodiments the pharmaceutical composition comprising desferrioxamine E with a metal, as the active ingredient, can be administered either alone or in combination with other metal-nocardamine complex/s.
Of particular relevance are formulations of the complexes or any compositions of the invention adapted for use as nano- or micro-particles. Nanoscale drug delivery systems using liposomes and nanoparticles are emerging technologies for the rational drug delivery, which offers improved pharmacokinetic properties, controlled and sustained release of drugs and, more importantly, lower systemic toxicity. A particularly desired solution allows for externally triggered release of encapsulated compounds. Externally controlled release can be accomplished if drug delivery vehicles, such as liposomes or polyelectrolyte multilayer capsules, incorporate nanoparticle (NP) actuators. More specifically, controlled drug delivery systems (DDS) have several advantages compared to the traditional forms of drugs. A drug is transported to the site of action, hence, its influence on vital tissues and undesirable side effects can be minimized. Accumulation of therapeutic compounds in the target site increases and, consequently, the required doses of drugs are lower. This form of therapy is especially important when there is a discrepancy between the dose or the concentration of a drug and its therapeutic results or toxic effects. Cell-specific targeting can be accomplished by attaching drugs to specially designed carriers. Various nanostructures, including liposomes, polymers, dendrimers, silicon or carbon materials, and magnetic nanoparticles, have been tested as carriers in drug delivery systems. It should be therefore appreciated that the complexes of the invention or any compositions thereof may be formulated in any of the nano- or micro-particles disclosed herein.

A further aspect of the disclosure relates to the method for modulating cytokine levels in a cell. More specifically, the method may comprise the step of contacting said cell with an effective amount of at least one complex of nocardamine with at least one metal or any derivatives thereof, compositions or any vehicle, matrix, nano- or micro-particle comprising the same. Thus, in some embodiments the metal- nocardamine complexes of the invention modulate cytokine levels in a cell. As indicated herein after, modulating as used herein may be either increasing or decreasing the levels of cytokines in a cell or in a subject as described herein after.

Still further, in some embodiments, for modulating the cytokine levels in a cell, the complexes of the invention may be contacted with the cell. The term "contacting" means, to bring, put, incubate or mix together. As such, a first item is contacted with a second item when the two items are brought or put together, e.g., by touching them to each other or combining them. In the context of the present invention, the term "contacting" includes all measures or steps, which allow interaction between the complexes of the invention and the cells or subjects to be modulated, as specified herein after. The metal of the complexes used by the methods may be selected from the group consisting of lanthanide/s, actinide/s, post-transition metal/s or transition metal/s. The metal of the complexes used by the methods of the invention may be selected from the group consisting of zinc and gallium, or in a further disclosure from the group consisting of aluminum, silver, gold, cobalt, molybdenum, vanadium or any ionic forms thereof.

Further the metal ion of the complexes used by the methods may be selected from the group consisting of Zn²⁺ and Ga³⁺, or in a further disclosure from the group consisting of Al³⁺, Ag⁺ and Au³⁺, Co³⁺, vanadium and molybdenum.

According to the invention, the metal ion of the complexes used by the methods may be Zn²⁺. The methods may use at least one complex of nocardamine with Zn²⁺ as described herein before.

In alternative, the metal ion of the complexes used by the methods may be Ga³⁺. In some further embodiments, the methods may use at least one complex of Desferoxamine E with Ga³⁺.

As indicated above, the present disclosure provides methods for modulating cytokine levels in a cell. The term modulation as used herein refers to reduction or alternatively, to elevation of cytokine levels in said cell.

In yet more specific embodiments, the metal-nocardamine complexes of the invention may lead to decrease, reduction, elimination, attenuation or inhibition of the cytokine levels of at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or about 100% in a cell contacted with, or in a subject administered with the complexes of the invention as compared with a cell or a subject not treated with the complexes of the invention. Alternatively, in some embodiments, modulation may relate to increase, elevation, enhancement or augmentation of the cytokine levels of at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 200%, 300%, 400%, 500%, 600%, 700%, 800%, 900% or about 1000%, in a cell or in a subject contacted or administered with the complexes of the invention as compared with a cell or a subject not treated with the complexes of the invention.

Specifically, the method of modulation of cytokines relates to a modulation of at least one pro-inflammatory cytokine and at least one anti-inflammatory cytokine.

"Cytokines" are a category of small proteins (about 5 to 20 kDa), released by cells and have a specific effect on the interactions between cells, on communications between cells or on the behavior of cells. Cytokines can also be involved in autocrine signaling. Cytokines include chemokines, interferons, interleukins, lymphokines, tumor necrosis factor but generally not hormones or growth factors (despite some terminologic overlap). Cytokines are produced by a broad range of cells, including immune cells like macrophages, B lymphocytes, T lymphocytes and mast cells, as well as endothelial cells, fibroblasts, and various stromal cells; a given cytokine may be produced by more than one type of cell. It should be noted that in some embodiments, the methods of the invention may be applicable in modulating the levels of any cytokines as described above.

Still further, in some other embodiments, the methods may be used for modulating the levels of pro-inflammatory cytokines in a cell. "Pro-inflammatory cytokines" are cytokines which promote systemic inflammation. Due to their pro-inflammatory action, they initiate the pathologic process or aggravate it by producing fever, edema, inflammation, tissue destruction, and in some cases, even shock and death. Non-limiting examples for pro-inflammatory cytokines include IL 1-α, IL1-I3, IL6, and TNF-α, members of the IL20 family, IL33 LIF, IFN-y, OSM, CNTF, TGF-I3, GM-CSF, IL11, IL12, IL17, IL18, IL8 and a variety of other chemokines that chemo-attract inflammatory cells.

In several embodiments, the modulation of pro-inflammatory cytokines as used herein refers to the following cytokines: IL-1α, TNF- a, IL-6 and IL-17. In more specific embodiments, the methods may lead to reduction in the levels of pro-inflammatory cytokines, specifically, to reduction in the levels of at least one of IL-1α, TNF-α, IL-6 and IL-17.

In yet some further embodiments, the methods may be used for modulating the levels of anti-inflammatory cytokines in a cell. The anti-inflammatory cytokines are a series of immuno-regulatory molecules that control the pro-inflammatory cytokines response and act in concert with specific cytokine inhibitors and soluble cytokine receptors to down regulate the inflammatory immune response. Major anti-inflammatory cytokines include interleukin IL-1 receptor antagonist, IL-4, IL-10, IL-11, and IL-13. Leukemia inhibitory factor, interferon-alpha and transforming growth factor TGF-I3 are categorized as either anti-inflammatory or pro-inflammatory cytokines, under various circumstances. Specific cytokine receptors for IL-1, TNF-α, and IL-18 also function as inhibitors for pro-inflammatory cytokines. In some specific embodiments, the methods of the invention may lead to modulation of the levels of at least one of IL-13, IL-10 and IL-4.

The methods may lead to elevation of the levels of at least one anti-inflammatory cytokine in a cell and more specifically, the levels of at least one of IL-13, IL-4 and IL-10.

By further embodiments the present disclosure provides methods for modulating cytokine levels in a subject. More specifically, in some embodiments, the method may comprise the step of administering to said subject an effective amount of at least one complex of nocardamine with at least one metal thereof and any compositions or any vehicle, matrix, nano- or micro-particle comprising the same. It should be appreciated that in certain embodiments, this method may use any complex described by the invention, any combinations thereof with other metal-nocardamine complex/s or any compositions comprising the same, specifically, those described by the invention.

Thus, in yet some specific embodiments, the disclosure provides methods for modulating pro-inflammatory cytokine/s levels in a subject. In further specific embodiments, the methods may reduce the level of at least one pro-inflammatory cytokine in a subject. In more specific embodiments, such pro-inflammatory cytokines may be at least one of IL-la, IL-6, TNF-α and IL-17.

Still further, in some specific embodiments, the disclosure provides methods for modulating anti-inflammatory cytokine/s levels in a subject. In more specific embodiments, the methods may elevate the level of at least one anti-inflammatory cytokine in a subject. In more specific embodiments, such anti-inflammatory cytokines may be at least one of IL-13, IL-4 and IL-10.

Reducing the biological activities of pro-inflammatory cytokines, or alternatively or additionally, elevating the levels or activities of anti-inflammatory cytokines, can reduce the severity of attack of diseases mediated by pro-inflammatory cytokines. Thus, reducing the biological activities of IL-1α and TNF-α may be accomplished by several different, but highly specific, strategies, which involve neutralizing antibodies, soluble receptors, receptor antagonist, and inhibitors of proteases that convert inactive precursors to active, mature molecules. Blocking IL-1α or TNF-α has been highly successful in patients with psoriasis, rheumatoid arthritis, inflammatory bowel disease, or graft-versus-host disease.

Thus, in certain embodiments, the methods may be particularly applicable for subjects suffering from a disorder associated with at least one of elevated levels of pro-inflammatory cytokine/s and reduced levels of anti-inflammatory cytokine/s or from any other immune-mediated or related disorder.

More specifically, the present invention is appropriate for a subject suffering from a disorder such as inflammatory, infectious, proliferative, neuro-degenerative, ischemic, metabolic, spinal cord injury, trauma, autoimmune disorders and acute or chronic wound or injury.

It should be noted that in some embodiments, the common denominator of these groups of disorders may be their relationship to pro-inflammatory cytokines, namely, IL-1α, IL-6, TNF-α and IL-17 that are elevated and/or anti-inflammatory cytokines, specifically, any one of IL-13, IL-10 or IL-4, that are reduced while a disorder is active, and they return to base line level following a specific treatment.

As shown in Example 5, the methods of the disclosure may lead to a clear reduction in the levels of pro-inflammatory cytokines, specifically of at least one of IL-1α, IL-6, TNF-α and IL-17. According to some embodiments, wherein indicate "decreasing" or "reducing" the expression or the levels of a pro-inflammatory cytokine, for example, any one of IL-la, IL-6, TNF-α and IL-17, it is meant that such decrease or reduction may be a reduction or inhibition of between about 10% to 100% of the expression of such cytokines. The terms "decrease", "attenuation" and "elimination" as used herein relate to the act of becoming progressively smaller in size, amount, number, or intensity. Particularly, a decrease of 10%, 15%, 20%, 25%, 30%, 35%, 40%, 45%, 50%, 55%, 60%, 65%, 70%, 75%, 80%, 85%, 90%, 95% or 100% of the expression as compared to a suitable control. It should be further noted that decrease or reduction may be also a reduction of about 2 to 100 folds. Still further, it should be appreciated that the decrease in the levels or expression of said pro-inflammatory cytokine/s may be either in the transcription, translation or the stability of said cytokine. With regards to the above, it is to be understood that, where provided, percentage values such as, for example, 10%, 50%, 120%, 500%, etc., are interchangeable with "fold change" values, i.e., 0.1, 0.5, 1.2, 5, etc., respectively.

In some embodiments, *Interleukin-1 alpha* (IL-1α) as used herein, also known as hematopoietin-1 is a protein of the interleukin 1 family that in humans is encoded by the *IL1A* gene. In general, Interleukin 1 is responsible for the production of inflammation, as well as the promotion of fever and sepsis. IL-1α inhibitors are being developed to interrupt those processes and treat diseases.

IL-1α is produced mainly by activated macrophages, as well as neutrophils, epithelial cells, and endothelial cells. A wide variety of other cells upon stimulation can produce the precursor form of IL-1α. Among them are fibroblasts, macrophages, granulocytes, eosinophils, mast cells and basophils, endothelial cells, platelets, monocytes and myeloid cell lines, blood T-lymphocytes and B-lymphocytes, astrocytes, kidney mesangial cells, Langerhans cells, dermal dendritic cells, natural killer cells, large granular lymphocytes, microglia, neutrophils, lymph node cells, maternal placental cells and several other cell types. IL-1α possesses metabolic, physiological, haematopoietic activities, and plays one of the central roles in the regulation of the immune responses. It binds to the interleukin-1 receptor and, thereby, initiates the pathway that activates tumor necrosis factor-alpha.

Still further, *Interleukin-6* (IL-6), as used herein is an interleukin, acting as a classic pro-inflammatory cytokine. It is an important mediator of the acute phase response. It can be secreted by macrophages, neutrophils, adipocytes and muscle cells in response to a number of stress mediators, thus, stimulating further production of neutrophils and initiating several cellular stress-induced mechanisms. IL-6 stimulates inflammatory and auto-immune processes in numerous diseases, such as psoriasis, diabetes of both types, rheumatoid arthritis, atopic dermatitis, irritant contact dermatitis and sepsis.

*Tumor necrosis factor-alpha* (*TNF-α*)*,* also known as cachexin, or cachectin is a cytokine involved in systemic inflammation. TNF-α is a member of a group of cytokines that stimulate the acute phase reaction. It is predominantly produced by activated macrophages, but can be secreted also by neutrophils, mast cells, eosinophils, myocytes, endothelial cells, fibrobasts, and natural killer cells. The main function, but not the only one, of this cytokine is to regulate the immune cells. Following activation by a different stress-related stimuli, it can induce fever, inflammation, and apoptotic cell death, using several different mechanisms in numerous types of cells. An increase in TNF-α concentration has been implicated in a huge variety of inflammatory diseases. It was demonstrated that a local increase in concentration of TNF-α will induce the typical signs of inflammation: heat, swelling, redness, pain and loss of function. There are multiple reports about a cross-talk and mutual activation of cytokines such as IL-1 super family, IL-6 and TNF-α.

*Interleukin 17A* (IL-17 or IL-17A) as used herein, originally identified as a transcript from a rodent T-cell hybridoma, is the founding member of a group of cytokines called the IL-17 family. Known as CTLA8 in rodents, IL-17 shows high homology to viral IL-17 encoded by an open reading frame of the T-lymphotropic rhadinovirus *Herpesvirus saimiri.* Interleukin 17 is a cytokine that acts as a potent mediator in delayed-type reactions by increasing chemokine production in various tissues to recruit monocytes and neutrophils to the site of inflammation, similar to Interferon gamma. IL-17 is produced by T-helper cells and is induced by IL-23, which results in destructive tissue damage in delayed-type reactions. Interleukin 17 functions as a pro-inflammatory cytokine that responds to the invasion of the immune system by extracellular pathogens and induces destruction of the pathogen's cellular matrix. Interleukin 17 acts synergistically with tumor necrosis factor and interleukin-1. To elicit its functions, IL-17 binds to a type I cell surface receptor called IL-17R of which there are at least three variants IL17RA, IL17RB, and IL17RC.

Numerous immune regulatory functions have been reported for the IL-17 family of cytokines, presumably due to their induction of many immune signaling molecules. The most notable role of IL-17 is its involvement in inducing and mediating pro-inflammatory responses. IL-17 is commonly associated with allergic responses. IL-17 induces the production of many other cytokines (such as IL-6, G-CSF, GM-CSF, IL-1β, TGF-β, TNF-α), chemokines (including IL-8, GRO-α, and MCP-1), and prostaglandins (e.g., PGE₂) from many cell types (fibroblasts, endothelial cells, epithelial cells, keratinocytes, and macrophages). The release of cytokines causes many functions, such as airway remodeling, a characteristic of IL-17 responses. The increased expression of chemokines attracts other cells including neutrophils but not eosinophils. IL-17 function is also essential to a subset of CD4+ T-Cells called T helper 17 (Tₕ17) cells. As a result of these roles, the IL-17 family has been linked to many immune/autoimmune related diseases including rheumatoid arthritis, asthma, lupus, allograft rejection, anti-tumour immunity and recently psoriasis and multiple sclerosis. Notably, among this family, IL-17F has been well characterized both in vitro and in vivo, and has been shown to have a pro-inflammatory role in asthma and its expression level is correlated with disease severity. Hence, IL-17F may have a crucial role in allergic airway inflammation, and have important therapeutic implications in asthma. Because of its involvement in immune regulatory functions, IL-17 inhibitors are being investigated as possible treatments for autoimmune diseases such as rheumatoid arthritis, psoriasis and inflammatory bowel disease.

Recently, new treatments for inflammatory disorders have been developed where at least one of the rationales was to reduce the levels of pro-inflammatory cytokines. For example, as TNF-α is known to promote inflammatory response, monoclonal antibodies have been developed to inhibit this cytokine in order to treat diseases associated with inflammation, such as psoriasis, rheumatoid arthritis, ulcerative colitis, Crohn's disease, and ankylosing spondylitis. Another TNF-α inhibitor, the circulating receptor fusion protein has been in use for treatment of psoriasis, rheumatoid arthritis and ankylosing spondylitis.

Reducing the biological activities of IL-1 and TNF is accomplished by several different, but highly specific, strategies, which involve neutralizing antibodies, soluble receptors, receptor antagonist, and inhibitors of proteases that convert inactive precursors to active, mature molecules. Blocking IL-1 or TNF has proven a highly successful treatment of patients with rheumatoid arthritis, inflammatory bowel disease, or graft-versus-host disease. Likewise, using an inhibitor of IL-17 has also been a strategy for treatment of other disorders associated with inflammation. Based on emerging evidence from animal models, IL-17 has been suggested as a target for anti-inflammatory therapies to improve recovery post-stroke and to reduce the formation of skin cancer. IL-17 has also been implicated in multiple sclerosis. IL-17A, IL-17F and IL-17A/F-receptor inhibitors: brodalumab, ixekizumab and secukinumab received recently an approval for the treatment of psoriasis. Thus, the disclosed metal-DFO E complexes, compositions, kits and methods of the invention provide novel tools for treating and preventing any disorder associated with elevated levels of the pro-inflammatory cytokines, specifically, the disorders mentioned herein above.

Still further, as shown in Example 6, the methods of the invention may lead to an increase in the levels of anti-inflammatory cytokines, specifically of at least one of IL-13 IL-10 and IL-4. According to some embodiments, wherein indicate "increasing" or "enhancing" the expression of the levels of an anti-inflammatory cytokine, for example, any one of IL-13, IL-10 and IL-4, specifically of IL-13, it is meant that such increase or enhancement may be an increase or elevation of between about 10% to 100% of the expression of such cytokines. The terms "increase", "augmentation" and "enhancement" as used herein relate to the act of becoming progressively greater in size, amount, number, or intensity. Particularly, an increase of 10%, 15%, 20%, 25%, 30%, 35%, 40%, 45%, 50%, 55%, 60%, 65%, 70%, 75%, 80%, 85%, 90%, 95% or 100% of the expression as compared to a suitable control. It should be further noted that increase or elevation may be also an increase of about 2 to 100 folds. Still further, it should be appreciated that the increase of the levels or expression of said IL-13 cytokine may be either in the transcription, translation or the stability of said cytokine. With regards to the above, it is to be understood that, where provided, percentage values such as, for example, 10%, 50%, 120%, 500%, etc., are interchangeable with "fold change" values, i.e., 0.1, 0.5, 1.2, 5, etc., respectively.

*Interleukin-13* (IL-13), as used herein is an example for anti-inflammatory cytokine. IL-13 is secreted by many cell types, but especially by the T-helper Type 2 (Th2) cells. Several publications describe it as a mediator of allergic *"inflammation"* and disease, while on the other hand it has also been described as a mediator playing a potent anti-inflammatory role. Additionally, IL-13 was shown to induce several changes in the gut that create an environment hostile to parasites (helminthes), that ultimately lead to detachment of the parasitic organisms from the gut wall and their removal.

*Interleukin-10* (IL-10) is a major anti-inflammatory cytokine found within the human immune response. It is a potent inhibitor of Th1 cytokines, including both IL-2 and IFN-γ. This activity accounts for its initial designation as cytokine synthesis inhibition factor. In addition to its activity as a Th2 lymphocyte cytokine, IL-10 is also a potent deactivator of monocyte/macrophage pro-inflammatory cytokine synthesis. After engaging its high-affinity 110-kd cellular receptor, IL-10 inhibits monocyte/macrophage-derived TNF-alpha, IL-1, IL-6, IL-8, IL-12, granulocyte colony-stimulating factor, MIP-1a, and MIP-2a.

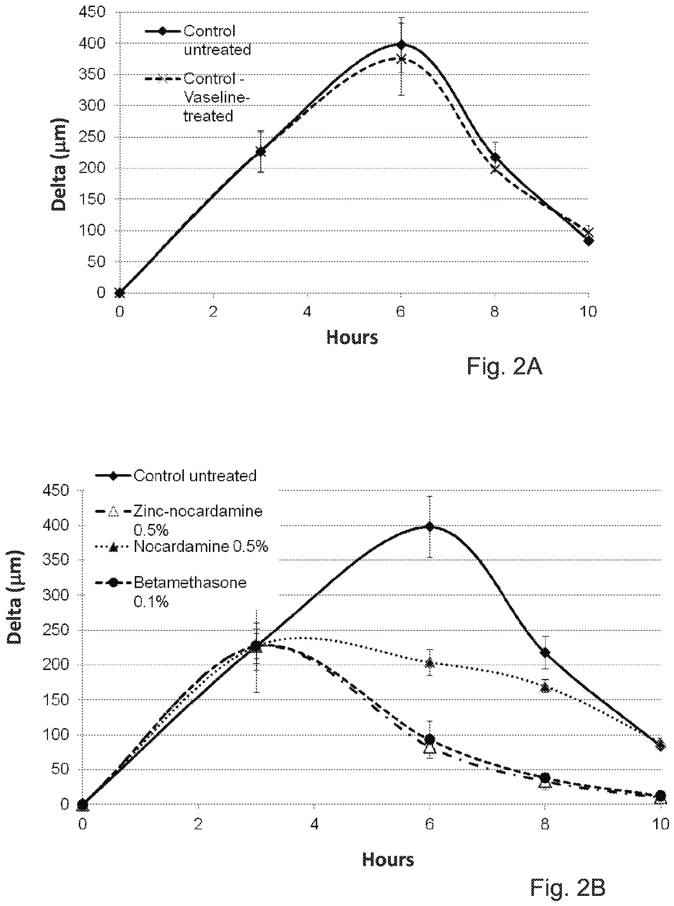

The results presented by the present invention in Examples 5 and 6 (specifically, Figures 2-7) clearly demonstrate the therapeutic potential of the zinc-nocardamine complex in reducing the signs of inflammation in a murine model of inflammation-related dermatitis. Thus, the pharmaceutical composition of the invention may be specifically suitable for treating disorders associated with inflammation.

Based on the insufficient availability of specific anti-inflammatory treatments and the awareness of the roles of pro-inflammatory cytokines in the pathogenesis of several groups of disorders, the development of new anti-inflammatory treatments is anticipated. Thus, there is a current need for the employment of the present invention in therapy.

Thus, another aspect of the present disclosure concerns a method of treating, preventing, inhibiting, reducing, eliminating, protecting or delaying the onset of a pathological condition or a disorder in a subject in need thereof. In some specific embodiments, the method comprises the step of administering to the treated subject a therapeutically effective amount of at least one complex of nocardamine with at least one metal or any combinations thereof with other metal-nocardamine complex/s, or any pharmaceutical compositions, carriers, matrix or vehicles comprising the same. In some specific embodiments, these disorders and/or diseases may be characterized by having a clinical beneficial effect when the labile iron or copper are chelated, and more specifically, when pro-inflammatory cytokines associated with these disorders are reduced (down regulated) and/or when anti-inflammatory cytokines associated with these disorders are elevated (up regulated). In more specific embodiments, the present disclosure provides a method for treating, preventing, reducing, attenuating, inhibiting and eliminating a disorder associated with elevated pro-inflammatory cytokines by administering to said a subject in need thereof a therapeutically effective amount of at least one complex of nocardamine with at least one metal, carriers, matrix or vehicles comprising the same.

The metal of the complexes used by the methods may be selected from the group consisting of lanthanide/s, actinide/s, post-transition metal/s or transition metal/s.

The metal of the complexes used by the methods may be selected from the group consisting of zinc, gallium, aluminum, silver, gold, cobalt, molybdenum, vanadium, or any ionic forms thereof.

According to the invention, the metal ion of the complexes used by the methods may be selected from the group consisting of Zn²⁺, Ga³.

The metal ion of the complexes used by the methods may be Zn²⁺. The methods may use at least one complex of nocardamine with Zn²⁺ as described herein before.

In alternative, the metal ion of the complexes used by the methods may be Ga³⁺.

Further, the methods may use at least one complex of nocardamine with Ga³⁺.

As disclosed earlier herein, according to specific embodiments, the disclosure provides a method of treating, preventing, inhibiting, reducing, eliminating, protecting or delaying the onset of a disease or a pathological condition or a disorder, wherein said disorder is associated with at least one of elevated levels of pro-inflammatory cytokine/s and reduced levels of anti-inflammatory cytokines. Specifically, the pro-inflammatory cytokines may be at least one of IL-1α, TNF-a, IL-6 and IL-17. In some further embodiments, the anti-inflammatory cytokines may be at least one of IL-13, IL-10 and IL-4.

As noted above, in some embodiments, the present disclosure relates to a method of treating, preventing, inhibiting, reducing, eliminating, protecting or delaying the onset of a disease or a disorder, which is associated with at least one of elevated levels of proinflammatory cytokine/s and reduced level/s of anti-inflammatory cytokine/s In more specific embodiments, the disorder may be at least one of inflammatory, infectious, proliferative, neuro-degenerative, ischemic, metabolic, spinal cord injury, trauma, autoimmune disorders and acute or chronic wound or injury. The present disclosure provides methods for treating inflammatory disorders in a subject in need thereof, comprising the step of administering to said subject a therapeutically effective amount of at least one complex of nocardamine with at least one metal, carriers, matrix or vehicles comprising the same, wherein inflammatory disorder relates to any disorder/disease, condition selected from inflammatory skin disorder, inflammatory bowel disease (IBD), specifically ulcerative colitis and Crohn's disease, arthritis and inflammatory respiratory disorders, specifically, asthma.

The general term "inflammatory disorder" relates to disorders where an inflammation is a main response to harmful stimuli, such as pathogens, damaged cells, or irritants. Inflammation is a protective response that involves immune cells, blood vessels, and molecular mediators, as well as the end result of long-term oxidative stress.

"Inflammatory disorders" are a large group of disorders that underlie a vast variety of human diseases. Also, the immune system can be involved in inflammatory disorders, stemming from abnormal immune response of the organism against substances of its own, or initiation the inflammatory process for unknown reason, i.e. autoimmune and auto-inflammatory disorders, respectively. Non-immune diseases with etiological origins in inflammatory processes include cancer, atherosclerosis, and ischemic heart disease.

The purpose of inflammation is to eliminate the initial cause of cell injury, clear out necrotic cells and tissues and to initiate tissue repair. The classical physiological signs of acute inflammation are pain, heat, redness, swelling, and loss of function. A series of biochemical events propagates and matures the inflammatory response, involving the local vascular system, the immune system, and various cells within the injured tissue. Prolonged inflammation, known as "chronic inflammation ", leads to a progressive shift in the type of cells present at the site of inflammation and is characterized by simultane ous destruction and healing of the tissue from the inflammatory process. Inflammation also induces high systemic levels of specific cytokines designated as proinflammatory cytokines which include IL-1α, IL-6, IL-8, IFN-γ, TNF-a, IL-17 and IL- 18. The inflammatory response must be actively terminated when no longer needed to prevent unnecessary "bystander" damage to tissues. Failure to do so results in chronic inflammation, and cellular destruction. Resolution of inflammation occurs by different mechanisms in different tissues. Acute inflammation normally resolves by mechanisms that have remained somewhat elusive. Emerging evidence now suggests that an active, coordinated program of resolution initiates in the first few hours after an inflammatory response begins. After entering tissues, granulocytes promote the switch of arachidonic acid-derived prostaglandins and leukotrienes to lipoxins, which initiate the termination sequence. Neutrophil recruitment thus ceases, and programmed death by apoptosis (programmed cell death) is engaged. These events coincide with the biosynthesis, from omega-3 polyunsaturated fatty acids, of resolvins and protectins, which critically shorten the period of neutrophil infiltration by initiating apoptosis. As a consequence, apoptotic neutrophils undergo phagocytosis by macrophages, leading to neutrophil clearance and release of anti-inflammatory and reparative cytokines such as transforming growth factor-β1. The anti-inflammatory program ends with the departure of macrophages through the lymphatics.

The term "pathological conditions associated with inflammation" as used herein relates to at least one the following: arthritis (ankylosing spondylitis, systemic lupus erythematosus, osteoarthritis, rheumatoid arthritis, psoriatic arthritis), asthma, atherosclerosis, inflammatory bowel disease (Crohn's disease, ulcerative colitis), dermatitis (including psoriasis).

As shown by the Examples (specifically, Figures 2-6) application of the zinc-nocardamine complex of the invention using the ICD model, showed a marked reduction in inflammation. Moreover, this anti-inflammatory effect of the zinc- nocardamine complex of the invention was accompanied by a significant reduction in pro-inflammatory cytokines IL-1α, TNF-α, IL-6 and IL-17 (Figure 6). Thus, the ability of the zinc-nocardamine complex of the invention to reduce the levels of proinflammatory cytokines together with elimination of the inflammation provides a substantial evidence for its anti-inflammatory property. In line with these results, as demonstrated in Figure 7, an increase in anti-inflammatory cytokine IL-13 was observed following treatment with the zinc-nocardamine complex of the invention. Taken together, it can be concluded that, metal-nocardamine complexes of the invention, could be used as a suitable treatment for disorders in which an inflammation is an underlying mechanism.

As indicated above, using a murine model of inflammation (ICD), the following Examples (specifically, Figs. 2-6) clearly demonstrate the applicability of the complex of the invention, as well as compositions, methods and kits thereof for treating acute and chronic inflammatory skin disorders. Thus, in some embodiments, the method of treatment as detailed in the present invention may be suitable for treating, an inflammatory disorder such as chronic or acute inflammatory skin disorder.

Chronic or acute inflammatory skin pathologic conditions include dermatitis, inflammatory skin injuries, inflammatory-related disturbances of skin pigmentation, for example, vitiligo and eczemas. More specifically, certain embodiments of the invention relates to the use of the complex of the invention, as well as compositions, methods and kits thereof for treating dermatitis. The term "dermatitis" refers to inflammation of the skin, in general. The different kinds usually have in common an allergic reaction to specific allergens. The term may be used to refer to eczema, which is also known as dermatitis eczema or eczematous dermatitis. A diagnosis of eczema often implies atopic dermatitis (childhood eczema), but without proper context, it means nothing more than a "rash", i.e. a transient skin inflammation. In some languages, "dermatitis" and eczema are synonyms, while in other languages "dermatitis" implies an acute condition and "eczema" a chronic one. The two conditions are often classified together. In some specific embodiments, the methods, complexes and compositions of the invention, may be applicable for any type of dermatitis, specifically, atopic dermatitis, contact dermatitis, seborrhoeic dermatitis or seborrheic dermatitis and xerotic eczema. More specifically, as used herein, atopic dermatitis is an allergic disease believed to have a hereditary component, and often runs in families whose members also have asthma. Itchy rash is particularly noticeable on head and scalp, neck, inside of elbows, behind knees, and buttocks. It is very common, and rising, in developed countries. Irritant contact dermatitis is sometimes misdiagnosed as atopic dermatitis. Still further, contact dermatitis, as used herein is of two types: allergic (resulting from a delayed reaction to an allergen, such as poison ivy, nickel, or Balsam of Peru), and irritant (resulting from direct a reaction to a detergent, such as sodium lauryl sulfate, for example). Xerotic eczema, as used herein (aka asteatotic e., e. craquele or craquelatum, winter itch, pruritus hiemalis) is dry skin that becomes so serious it turns into eczema. It worsens in dry winter weather, and limbs and trunk are most often affected. The itchy, tender skin resembles a dry, cracked, river bed. Finally, the methods of the invention may be used for treating seborrhoeic dermatitis or Seborrheic dermatitis ("cradle cap" in infants), that as used herein, is a condition sometimes classified as a form of eczema that is closely related to dandruff. It causes dry or greasy peeling of the scalp, eyebrows, and face, and sometimes trunk. The condition is harmless except in severe cases of cradle cap. In newborns it causes a thick, yellow, crusty scalp rash called cradle cap, which seems related to lack of biotin and is often curable. It should be appreciated that the methods of the invention may be applicable for any form of dermatitis disclosed herein.

It should be appreciated that based on its anti-inflammatory qualities the complexes, and compositions of the inventions could be applicable for treatment of another inflammatory skin disorder, specifically, psoriasis.

Psoriasis is also an inflammatory disorder mediated by pro- and anti-inflammatory cytokines. Although the exact causes and pathogenesis of psoriasis are unknown, over-expression of pro-inflammatory, type 1 (Th1) cytokines has been demonstrated in psoriasis and is believed to be of pathophysiological importance. Importantly, a relative deficiency in cutaneous IL-10 mRNA expression compared with other inflammatory dermatoses was demonstrated. Moreover, previous publications demonstrate that patients during established antipsoriatic therapy showed higher IL-10 mRNA expression of peripheral blood mononuclear cells than patients before therapy. This suggested that IL-10 may have antipsoriatic capacity. Indeed, subcutaneous administration of IL-10 produced immunosuppressive effects in patients (depressed monocytic HLA-DR expression, TNF-alpha and IL-12 secretion capacity, IL-12 plasma levels, and responsiveness to recall antigens) as well as a shift toward a type 2 (Th2) cytokine pattern (increasing proportion of IL-4, IL-5, and IL-10 producing T cells, selective increase in IgE serum levels) were observed. More specifically, psoriasis is a common skin condition that features patchy, raised, red areas of skin inflammation with scaling. Psoriasis often affects the tips of the elbows and knees, the scalp, the navel, and the area surrounding the genitals or anus. It occurs when the immune system sends out faulty signals that speed up the growth cycle of skin cells. The scaly patches commonly caused by psoriasis, called psoriatic plaques, are areas of inflammation and excessive skin production. Skin rapidly accumulates at these sites which gives it a silvery-white appearance. Plaques frequently occur on the skin of the elbows and knees, but can affect any area including the scalp, palms of hands and soles of feet, and genitals. In contrast to eczema, psoriasis is more likely to be found on the outer side of the joint. The disorder is a chronic recurring condition that varies in severity from minor localized patches to complete body coverage. Fingernails and toenails are frequently affected (psoriatic nail dystrophy) and can be seen as an isolated symptom. Psoriasis can also cause inflammation of the joints, which is known as psoriatic arthritis. Ten to fifteen percent of people with psoriasis develop psoriatic arthritis, which is of chronic recurrent nature. The symptoms of psoriasis can manifest in a variety of forms. Variants include plaque, pustular, guttate and flexural psoriasis. Psoriasis may be classified into nonpustular and pustular types.

Nonpustular psoriasis includes Psoriasis vulgaris and Psoriatic erythroderma. Psoriasis vulgaris (also known as Chronic stationary psoriasis or Plaque-like psoriasis), is the most common form of psoriasis. It affects 80 to 90% of people with psoriasis. Plaque psoriasis typically appears as raised areas of inflamed skin covered with silvery white scaly skin. These areas are called plaques.

Psoriatic erythroderma (Erythrodermic psoriasis) involves the widespread inflammation and exfoliation of the skin over most of the body surface. It may be accompanied by severe itching, swelling and pain. It is often the result of an exacerbation of unstable plaque psoriasis, particularly following the abrupt withdrawal of systemic treatment. This form of psoriasis can be fatal, as the extreme inflammation and exfoliation disrupt the body's ability to regulate temperature and for the skin to perform barrier functions.

In yet another specific embodiment, the methods of the invention may be used for treating Pustular psoriasis. Pustular psoriasis appears as raised bumps that are filled with non-infectious pus (pustules). The skin under and surrounding the pustules is red and tender. Pustular psoriasis can be localized, commonly to the hands and feet (palmoplantar pustulosis), or generalized with widespread patches occurring randomly on any part of the body. Pustular psoriasis subtypes include Generalized pustular psoriasis (Pustular psoriasis of von Zumbusch), Pustulosis palmaris et plantaris (Persistent palmoplantar pustulosis, Pustular psoriasis of the Barber type, Pustular psoriasis of the extremities), Annular pustular psoriasis, Acrodermatitis continua and Impetigo herpetiformis.

It should be appreciated that the methods of the invention may be also applicable for treating any additional types of psoriasis, for example, drug-induced psoriasis, inverse psoriasis, or flexural psoriasis. The latter, appears as smooth inflamed patches of skin in skin folds, particularly around the genitals (between the thigh and groin), the armpits, under an overweight stomach (pannus), and under the breasts (inframammary fold). It is aggravated by friction and sweat, and is vulnerable to fungal infections.

Still further, the method of the invention may be used for treating guttate psoriasis. This type of psoriasis is characterized by numerous small, scaly, red or pink, teardrop-shaped lesions. These numerous spots of psoriasis appear over large areas of the body, primarily the trunk, but also the limbs, and scalp. Guttate psoriasis is often preceded by a streptococcal infection, typically streptococcal pharyngitis.

Nail psoriasis that may be also treated by the method of the invention produces a variety of changes in the appearance of finger and toe nails. These changes include discoloring under the nail plate, pitting of the nails, lines going across the nails, thickening of the skin under the nail, and the loosening (onycholysis) and crumbling of the nail.

In yet another embodiment, the method of the invention may be used for treating psoriatic arthritis. Psoriatic arthritis involves joint and connective tissue inflammation. Psoriatic arthritis can affect any joint but is most common in the joints of the fingers and toes. This can result in a sausage-shaped swelling of the fingers and toes known as dactylitis. Psoriatic arthritis can also affect the hips, knees and spine (spondylitis). About 10-15% of people who have psoriasis also have psoriatic arthritis.

In some embodiments, treatment of a subject suffering from psoriasis may improve the physiological state of the subject, for example, smoothing skin that was rough due to the disease. In preferred embodiments, such psychological improvement can be achieved by topical application of the metal-complexes of the invention, which does not irritate the skin and does not promote inflammation.

It should be appreciated that, the complex of the invention, as well as compositions, and methods thereof, may be applicable for treating psoriasis, as well as the extra-dermatological features of psoriasis including psoriatic arthritis.

Acne is another non-limiting example for skin inflammatory disorders that may be treated by the method of the invention. Acne is a general term used for eruptive disease of the skin. It is sometimes used as a synonym for acne vulgaris. However, there are several different types of acne. These include Acne vulgaris, acne conglobata, acne miliaris necrotica, tropical acne, infantile acne/neonatal acne, excoriated acne, acne fulminans, drug-induced acne/acne medicamentosa (steroid acne), halogen acne (iododerma, bromoderma, chloracne), oil acne, tar acne, acne cosmetica, occupational acne, acne aestivalis, acne keloidalis nuchae, acne mechanica, acne with facial edema, pomade acne, acne necrotica, blackhead, and lupus miliaris disseminatus faciei.

Inflammation-related skin disorder that may be treated by the method of the invention may include also insect bites and stings. During the sting insects inject formic acid and/or toxins. These can cause an immediate skin reaction, often resulting in redness and swelling, in the injured area. The sting from Hymenoptera order are usually painful, and may stimulate, in patients at risk, a life-threatening systemic allergic reaction, called anaphylaxis. Systemic allergic sting reactions often result in cutaneous, vascular or respiratory symptoms and signs, either alone or in any combination with possible involvement of other less common target tissues. Cardiac anaphylaxis can also cause bradycardia, arrhythmias, angina or myocardial infarction. Bites from mosquitoes, fleas, and mites are more likely to cause itching than pain. The skin reaction to insect bites and stings usually lasts for up to a few days. However, in some cases the local reaction can last for up to two years. The reaction to a sting is of three types. The normal reaction involves the area around the bite with redness, itchiness, and pain. A large local reaction occurs when the area of swelling is greater than five cm (in diameter). Systemic reactions are when symptoms occur in areas besides that of the bites.

In yet another embodiment, the method of the invention may be applicable for treating vitiligo. Vitiligo as used herein is a chronic disorder that causes depigmentation of patches of skin. It occurs when melanocytes, die or are unable to function. The cause of vitiligo is unknown, but research suggests that is may arise from autoimmune, genetic, oxidative stress, neural, or viral causes. The incidence worldwide is less than 1%, with the most common form being non-segmental vitiligo. Symptoms usually begin between ages 10 years and age 30 years, including whitening or graying of hair, loss of skin color inside the mouth and loss of eye color. The most notable symptom of vitiligo is depigmentation of patches of skin that occurs on the extremities. In non-segmental vitiligo (NSV), there is usually some form of symmetry in the location of the patches of depigmentation. New patches also appear over time and can be generalized over large portions of the body or localized to a particular area. Vitiligo where little pigmented skin remains is referred to as vitiligo universalis. NSV can come about at any age, unlike segmental vitiligo which is far more prevalent in teenage years. Classes of non-segmental vitiligo include generalized vitiligo, universal vitiligo, focal vitiligo, acrofacial vitiligo and muscosal vitiligo. Segmental vitiligo (SV) differs in appearance, aetiology and prevalence from associated illnesses. Its treatment is different from that of NSV. It tends to affect areas of skin that are associated with dorsal roots from the spine. It spreads much more rapidly than NSV and, without treatment, it is much more stable/static in course and is not associated with auto-immune diseases, being a treatable condition that responds to topical treatment.

It should be appreciated that the metal-nocardamine complexes of the invention, compositions, complexes, kits and methods described by the invention, may be applicable for any form of inflammatory skin disorder, specifically, any form of dermatitis or psoriasis disclosed herein.

Still further, it should be appreciated that in certain embodiments, the methods of the invention may be applicable for treating an acute or chronic wound. In yet some further embodiments, the methods of the invention may be applicable for treating acute or chronic injury caused by a chemical or thermal burn, and by mechanical hit/blow. As such, the present invention provides methods for wound healing.

The term *wound* as used herein is a type of injury which happens relatively quickly in which a skin is torn, cut, or punctured (an *open* wound), or where blunt force trauma causes a contusion (a *closed* wound). In pathology, it specifically refers to a sharp injury which damages the dermis of the skin. The wounds can be further classified as penetrating and non-penetrating. Penetrating wounds result from a trauma that breaks through the full thickness of the skin; reaching down to the underlying tissue and organs. Non-penetrating wounds are usually the result of blunt trauma or friction with other surfaces and this kind of wound does not break through the skin. Miscellaneous wounds may include, but are not limited to: *Thermal wounds:* Extreme temperatures, either hot or cold, can result in thermal injuries (like burns, sunburns and frostbite). Thermal burn is a type of burn resulted from making contact with heated objects, such as boiling water, steam, hot cooking oil, fire, and hot objects. Scalds are the most common type of thermal burn suffered by children, while for adults thermal burn is most commonly caused by fire. Burn are generally classified by severity from first degree up to fourth degree, but the American Burn Association (ABA) has categorized thermal burns as minor, moderate, and major, based almost solely on the depth and size of the burn. *Chemical wounds:* These result from contact with or inhalation of chemical materials that cause skin or lung damage. A chemical burn occurs when a living tissue is exposed to a corrosive substance such as a strong acid or base. Chemical burns follow standard burn classification and may cause extensive tissue damage. The main types of irritant and/or corrosive products are: acids, bases, oxidizers/reducing agents, solvents, and alkylants. Additionally, chemical burns can be caused by some types of chemical weapons, e.g., vesicants such as mustard gas and Lewisite, or urticants such as phosgene oxime. *Bites and Stings:* Bites can be from humans, dogs, bats, rodents, snakes, scorpions, spiders and ticks. *Electrical wounds:* These usually present with superficial burn-like or sting-like wounds secondary to the passage of high-voltage electrical currents through the body, and may include more severe internal damage. Depending on the healing time of a wound, it can be classified as acute or chronic. Those classified as acute wounds heal uneventfully (with no complications) in the predicted amount of time. Those classified as chronic wounds take a longer time to heal and might have some complications. Factors that contribute to non-healing chronic wounds are diabetes, venous or arterial disease, infection, and metabolic deficiencies of old age.

Non-healing wounds of the diabetic foot are considered one of the most significant complications of diabetes, representing a major worldwide medical, social, and economic burden that greatly affects patient quality of life. Associated with inadequate circulation, poorly functioning veins, and immobility, non-healing wounds occur most frequently in the elderly and in people with diabetes - populations that are sharply rising as the nation ages and chronic diseases increase.

Although diabetes can ravage the body in many ways, non-healing ulcers on the feet and lower legs are common outward manifestations of the disease. Also, diabetics often suffer from nerve damage in their feet and legs, allowing small wounds or irritations to develop without awareness. Given the abnormalities of the microvasculature and other side effects of diabetes, these wounds take a long time to heal and require a specialized treatment approach for proper healing.

*Pressure ulcers,* also known as *pressure sores, bedsores* and *decubitus ulcers,* are localized injuries to the skin and/or underlying tissue that usually occur over a bony prominence as a result of pressure, or pressure in combination with shear and/or friction. The most common sites are the skin overlying the sacrum, coccyx, heels or the hips, but other sites such as the elbows, knees, ankles or the back of the cranium can be affected.

Pressure ulcers occur due to pressure applied to soft tissue resulting in completely or partially obstructed blood flow to the soft tissue. Shear is also a cause, as it can pull on blood vessels that feed the skin. Pressure ulcers most commonly develop in individuals who are not moving about, such as those being bedridden or confined to a wheelchair. It is widely believed that other factors can influence the tolerance of skin for pressure and shear, thereby increasing the risk of pressure ulcer development. These factors are protein-calorie malnutrition, microclimate (skin wetness caused by sweating or incontinence), diseases that reduce blood flow to the skin, such as arteriosclerosis, or diseases that reduce the sensation in the skin, such as paralysis or neuropathy. The healing of pressure ulcers may be slowed by the age of the person, medical conditions (such as arteriosclerosis, diabetes or infection), smoking or medications such as anti-inflammatory drugs.

As indicated above, in some embodiments, the present invention further provides methods for wound healing. *Wound healing* is an intricate process where the skin or other body tissue repairs itself, or is being repaired after injury. In normal skin, the epidermis (surface layer) and dermis (deeper layer) form a protective barrier against the external environment. When the barrier is broken, an orchestrated cascade of biochemical events is quickly set into motion to repair the damage. This process is divided into predictable phases: blood clotting (hemostasis), inflammation, the growth of new tissue (proliferation), and the remodeling of tissue (maturation).

It should not be overlooked that the composition of the invention, particularly when used for treating inflammatory skin disorders, may be acceptable as topically applied composition. Specific embodiments contemplate skin inflammatory conditions, specifically, psoriasis treatment by topical administration of the affected skin areas of an ointment, cream, suspensions, paste, lotions, powders, solutions, oils, encapsulated gel, liposomes containing the complexes, any nano-particles containing the complexes of the invention, or sprayable aerosol or vapors containing a combination of these complexes. Conventional pharmaceutical carriers, aqueous, powder or oily bases, thickeners and the like may be necessary or desirable. The term "topically applied" or "topically administered" means that the ointment, cream, emollient, balm, lotion, solution, salve, unguent, or any other pharmaceutical form is applied to some or all of that portion of the skin of the patient skin that is, or has been, affected by, or shows, or has shown, one or more symptoms of psoriasis or other skin lesion.

By analogy, the metal-nocardamine complexes or the invention or any pharmaceutical compositions thereof, as well as methods of the invention may be applicable for preventing, treating, ameliorating or inhibiting another inflammation/immune-mediated disorder, e.g. inflammatory bowel disease (IBD), specifically, ulcerative colitis and Crohn's disease.

Inflammatory bowel diseases (IBD) are common gastrointestinal disorders, that can be perceived as being the result of a dysbalance between Th1-pro-inflammatory and Th2-anti-inflammatory subtypes of immune responses. IBD is a group of inflammatory conditions of the colon and small intestine. The major types of IBD are Crohn's disease and ulcerative colitis (UC). Other forms of IBD account for far fewer cases. These are collagenous colitis, lymphocytic colitis, ischaemic colitis, diversion colitis, and indeterminate colitis, in cases where it is impossible to make a definitive diagnosis distinguishing Crohn's disease from ulcerative colitis.

The main difference between Crohn's disease and UC is the location and nature of the inflammatory changes. Crohn's disease can affect any part of the gastrointestinal tract, from mouth to anus (skip lesions), although a majority of the cases start in the terminal ileum. Ulcerative colitis, in contrast, is restricted to the colon and the rectum. Microscopically, ulcerative colitis is restricted to the mucosa (epithelial lining of the gut), while Crohn's disease affects the whole bowel wall. Finally, Crohn's disease and ulcerative colitis present with extra-intestinal manifestations (such as liver problems, arthritis, skin manifestations and eye problems) in different proportions. Crohn's disease and ulcerative colitis share the same symptoms such as diarrhea, vomiting, weight loss, fever and abdominal pain.

A recent hypothesis posits that IBD may be caused by an over-active immune system attacking various tissues of the digestive tract, because of the lack of traditional targets such as parasites and worms. The number of people being diagnosed with IBD has increased as the number of infections by parasites such as roundworm, hookworm and human whipworms has fallen, and the condition is still rare in countries where parasitic infections are common.

There are several extra-intestinal manifestations that accompany IBD, for example: autoimmune phenomena, wherein immune complexes have a role in target organ damage. Patients with IBD have antibodies against components of colon cells and several different bacterial antigens. These antigens gain access to the immune system as a consequence of epithelial damage. Abnormalities of T cell-mediated immunity, such as diminished responsiveness to T cell stimuli, have also been described in these patients. In addition, changes in mucosal cell mediated immunity were identified, including increased concentrations of mucosal IgG cells and changes in T cells subsets, suggesting antigen stimulation. Exposure of target antigens after infectious, immune, or toxic damage, leads to activation of mucosal immune cells resulting in cytokines that lead to mucosal inflammatory response. Secretion of pro-inflammatory cytokines such as IFN-γ, contributes to an increase in mucosal permeability, and has been described in animal models of IBD.

Crohn's disease, like many other chronic, inflammatory diseases, can cause a variety of systemic symptoms. Among children, growth failure is common. Many children are first diagnosed with Crohn's disease (pediatric Crohn's disease) based on inability to maintain growth. In addition to systemic and gastrointestinal involvement, Crohn's disease can affect many other organ systems. Inflammation of the interior portion of the eye, known as uveitis, can cause eye pain, especially when exposed to light (photophobia). Inflammation may also involve the white part of the eye (sclera), a condition called episcleritis. Both episcleritis and uveitis can lead to loss of vision if untreated.

Crohn's disease is associated with a type of rheumatologic disease known as seronegative spondyloarthropathy. This group of diseases is characterized by inflammation of one or more joints (arthritis) or muscle insertions (enthesitis). The arthritis can affect larger joints such as the knee or shoulder or may exclusively involve the small joints of the hand and feet. The arthritis may also involve the spine, leading to ankylosing spondylitis if the entire spine is involved or simply sacroiliitis if only the lower spine is involved. The symptoms of arthritis include painful, warm, swollen, stiff joints and loss of joint mobility or function.

A colonoscopy is the best test for making the diagnosis of Crohn's disease as it allows direct visualization of the colon and the terminal ileum, identifying the pattern of disease involvement. Finding a patchy distribution of disease, with involvement of the colon or ileum but not the rectum, is suggestive of Crohn's disease.

Currently there is no cure for Crohn's disease and remission may not be possible or prolonged if achieved. Treatment for Crohn's disease is indicated only when the disease is active and involves first treating the acute problem, then maintaining remission.

Ulcerative colitis is another chronic inflammation of the lining of the gastrointestinal tract. Ulcerative colitis occurs in 35-100 people for every 100,000 in the United States, or less than 0.1% of the population. The disease is more prevalent in northern countries of the world, as well as in northern areas of individual countries or other regions. The incidence of ulcerative colitis in North America is 10-12 new cases per 100,000 per year, with a peak incidence of ulcerative colitis occurring between the ages of 15 and 25. Prevalence is 1 per 1000. There is thought to be a bimodal distribution in age of onset, with a second peak in incidence occurring in the 6th decade of life. The disease affects females more than males. The geographic distribution of ulcerative colitis and Crohn's disease is similar worldwide, with highest incidences in the United States, Canada, the United Kingdom, and Scandinavia. Higher incidences are seen in northern locations compared to southern locations in Europe and the United States.

As with Crohn's disease, the prevalence of ulcerative colitis is greater among Ashkenazi Jews and decreases progressively in other groups of Jewish descent, non-Jewish Caucasians, Africans, Hispanics, and Asians.

The clinical presentation of ulcerative colitis depends on the extent of the disease process. Patients usually present with diarrhea mixed with blood and mucus, of gradual onset. They also may have signs of weight loss, and blood on rectal examination. The disease is usually accompanied with different degrees of abdominal pain, from mild discomfort to severely painful cramps.

Ulcerative colitis is usually confined to the colon (large bowel), with the rectum almost universally being involved. The lining of the affected colon becomes inflamed and is characterized by open sores or ulcers, which bleed and produce pus. Inflammation in the colon also causes the colon to empty frequently, causing diarrhea mixed with blood. Ulcerative colitis is an intermittent disease, with periods of exacerbated symptoms, and periods that are relatively symptom-free. Although the symptoms of ulcerative colitis can sometimes diminish on their own, the disease usually requires treatment to enter a remission.

Ulcerative colitis is associated with a general inflammatory process that affects many parts of the body. Sometimes these associated extra-intestinal symptoms are the initial signs of the disease, such as painful, arthritic knees in a teenager. The presence of the disease cannot be confirmed, however, until the onset of intestinal manifestations.

About half of the people diagnosed with ulcerative colitis have mild symptoms. Others suffer frequent fevers, bloody diarrhea, nausea, and severe abdominal cramps. Ulcerative colitis may also cause problems such as arthritis (seronegative arthritis, ankylosing spondylitis, sacroiliitis), inflammation of the eye (iritis, uveitis, episcleritis), liver disease, and osteoporosis. These complications may be the result of inflammation triggered by the immune system because people with ulcerative colitis have abnormalities of the immune system.

According to some embodiments, the method of the invention may be particularly applicable for preventing, treating, ameliorating or inhibiting inflammatory arthritis.

For arthritis, a related conditions may include, by way of example, all types of primary inflammatory arthritis, for example, rheumatoid arthritis, psoriatic arthritis, ankylosing spondylitis (previously known as Bechterew's disease or Bechterew syndrome), juvenile idiopathic arthritis (JIA) and gout (metabolic arthritis). In addition to all primary forms of arthritis indicated, the condition treated by the invention may include all secondary forms of arthritis, for example, lupus erythematosus, Henoch-Schonlein purpura, haemochromatosis, hepatitis, Wegener's granulomatosis (and many other vasculitis syndromes), Lyme disease and familial mediterranean fever.

Since an inflammatory process is a mainstay of immune-mediated disorders, the anti-inflammatory effect, of compositions of the invention make them appropriate for the treatment of subjects suffering from an immune-mediated disorders, for example, various forms of arthritis.

It should be appreciated that various forms of arthritis may be generally grouped into two main categories, inflammatory arthritis, and degenerative arthritis, each with different causes. Therefore, according to one specific embodiment, the metal-nocardamine complexes of the invention or any pharmaceutical compositions thereof may be specifically intended for the treatment and/or amelioration of an inflammatory disorder, for example, an inflammatory arthritis.

Inflammatory arthritis is characterized by synovitis, bone erosions, osteopenia, soft-tissue swelling, and uniform joint space narrowing. More specifically, the hallmarks of joint inflammation are synovitis and erosion of bone. The latter will initially appear as a focal discontinuity of the thin, white, subchondral bone plate. Normally, this subchondral bone plate can be seen even in cases of severe osteopenia, whereas its discontinuity indicates erosion. Although it is true that periarticular osteopenia and focal subchondral osteopenia can appear prior to true bone erosion, it is the presence of bone erosion that indicates definite joint inflammation. As the bone erosion enlarges, osseous destruction extends into the trabeculae within the medullary space. One important feature of inflammatory arthritis relates to the concept of marginal bone erosion. This term is given to bone erosion that is located at the margins of an inflamed synovial joint. This specific location represents that portion of the joint that is intra-articular but not covered by hyaline cartilage. Therefore, early joint inflammation will produce marginal erosions prior to erosions of the subchondral bone plate beneath the articular surface. When looking for bone erosions, multiple views of a joint are essential to profile the various bone surfaces. A second important characteristic of an inflammatory joint process is uniform joint space narrowing. This occurs because destruction of the articular cartilage is uniform throughout the intra-articular space. A third finding of inflammatory joint disease is soft-tissue swelling.

It should be appreciated that inflammatory arthritis may be further divided into several subgroups, and therefore, the metal-nocardamine complexes, compositions, and the methods of the invention described herein, may be applicable for treating every inflammatory arthritis including an arthritis of the different subgroup.

A systemic arthritis is characterized by involvement of multiple joints, and includes two main categories, rheumatoid arthritis and seronegative spondyloarthropathy.

According to one embodiment, the metal-nocardamine complexes, compositions as well as methods, of the invention may be used for the treatment and/or amelioration of rheumatoid arthritis. Rheumatoid arthritis (RA) is a chronic, systemic autoimmune disorder that most commonly causes inflammation and tissue damage in joints (arthritis) and tendon sheaths, together with anemia. It can also produce diffuse inflammation in the lungs, pericardium, pleura, and the sclera of the eye, and also nodular lesions, most common in subcutaneous tissue. It can be a disabling and painful condition, which can lead to substantial loss of functioning and mobility. Serologic markers such as rheumatoid factor and antibodies to cyclic citrullinated peptide are important indicators of rheumatoid arthritis. The radiographic features of rheumatoid arthritis are those of joint inflammation and include particular osteopenia, uniform joint space loss, bone erosions, and soft-tissue swelling. Because of the chronic nature of the inflammation, additional findings such as joint subluxation and subchondral cysts may also be evident. The seronegative spondyloarthropathy category includes psoriatic arthritis, reactive arthritis, and ankylosing spondylitis, and is characterized by signs of inflammation, multiple joint involvement, and distal involvement in the hands and feet with added features of bone proliferation. Thus, according to one embodiment, the metal-nocardamine complexes, compositions and methods of the invention may be used for the treatment and/or amelioration of any condition of the seronegative spondyloarthropathy category.

More specifically, according to embodiment, the metal-nocardamine complexes, compositions and methods of the invention may be used for preventing, treating, ameliorating or inhibiting any type of arthritis, including psoriatic arthritis. Psoriatic arthritis is a chronic disease characterized by inflammation of the skin (psoriasis) and joints (arthritis).

Males and females are equally likely to suffer from psoriasis. For psoriatic arthritis, males are more likely to have the spondylitic form (in which the spine is affected), and females are more likely to have the rheumatoid form (in which many joints may be involved). Psoriatic arthritis usually develops in people aged 35-55 years. However, it can develop in people of almost any age. Psoriatic arthritis shares many features with several other arthritic conditions, such as ankylosing spondylitis, reactive arthritis, and arthritis associated with Crohn's disease and ulcerative colitis. All of these conditions can cause inflammation in the spine and joints, in the eyes, skin, mouth, and various organs.

According to another embodiment, the metal-nocardamine complexes, compositions, and methods, of the invention may be used for preventing, treating, ameliorating or inhibiting ankylosing spondylitis.

Ankylosing spondylitis (AS, previously known as Bechterew's disease, Bechterew syndrome, Marie-Strümpell disease and a form of spondyloarthritis), is usually a chronic and progressive form of arthritis, caused due to inflammation of multiple joints, characteristically the spinal facet joints and the sacroiliac joints at the base of the spine. While ankylosing spondylitis tends to affect these joints and the soft tissues around the spine, other joints may also be affected, as well as tissues surrounding the joints (entheses, where tendons and ligaments attach to bone). Ankylosing spondylitis may also involve areas of the body other than the joints, such as the eyes, heart, and lungs. This disorder frequently results in bony ankylosis (or fusion), hence the term ankylosing, which is derived from the Greek word ankylos, meaning stiffening of a joint. Spondylos means vertebra (or spine) and refers to inflammation of one or more vertebrae.

The disease is estimated to affect approximately 0.1-0.2% of the general population. Ankylosing spondylitis primarily affects young males. Males are four to ten times more likely to have ankylosing spondylitis than females. Most people with the disease develop it at age 15-35 years, with an average age of 26 years at onset.

Although the exact cause is unknown, ankylosing spondylitis is believed to be due to the combination of a genetic influence and a triggering environmental factor. Approximately 90-95% of patients with ankylosing spondylitis have the tissue antigen Human Leukocyte Antigen B27 (HLA-B27), compared to 7% in the general population. People with ankylosing spondylitis often have a family history of the disease.

In yet another embodiment, the metal-nocardamine complexes, compositions, as well as methods of the invention, may be used for preventing, treating, ameliorating or inhibiting reactive arthritis (ReA). Reactive arthritis, another type of seronegative spondyloarthropathy, is an autoimmune condition that develops in response to an infection in another part of the body. Coming into contact with bacteria and developing an infection can trigger reactive arthritis. It has symptoms similar to various other conditions collectively known as "arthritis," such as rheumatism. It is caused by another infection and is thus "reactive", i.e., dependent on the other condition. The "trigger" infection has often been cured or is in remission in chronic cases, thus making determination of the initial cause difficult.

The symptoms of reactive arthritis very often include a combination of three seemingly unlinked symptoms, an inflammatory arthritis of large joints, inflammation of the eyes (conjunctivitis and uveitis), and urethritis. It should be indicated that ReA is also known as Reiter's syndrome, after the German physician Hans Reiter, it is also known as arthritis urethritica, venereal arthritis and polyarteritis enterica.

It should be appreciated that there are many other forms of inflammatory arthritis, including juvenile idiopathic arthritis, gout and pseudo gout, as well as arthritis associated with colitis or psoriasis. It should be therefore appreciated that the metal-nocardamine complexes, compositions, as well as methods of the present invention are also applicable for these conditions.

Therefore, the metal-nocardamine complexes, compositions and methods of the invention, may be used for preventing, treating, ameliorating or inhibiting juvenile idiopathic arthritis (JIA). JIA, is the most common form of persistent arthritis in children (juvenile in this context refers to an onset before age 16, idiopathic refers to a condition with no defined cause, and arthritis is the inflammation of the synovium of a joint). JIA is a subset of arthritis seen in childhood, which may be transient and self-limited or chronic. It differs significantly from arthritis commonly seen in adults (rheumatoid arthritis), and other types of arthritis that can present in childhood which are chronic conditions (e.g. psoriatic arthritis and ankylosing spondylitis).

Still further, in some specific embodiments for inflammatory disorders, any inflammatory-respiratory disease, such as asthma can benefit from the treatment with the metal-nocardamine complexes, composition of the invention, where a therapeutically effective amount of the composition thereof is administered to a subject suffering from the disease. Thus, in specific embodiments, the method of the invention may be used for the prophylaxis, treatment and/or amelioration of respiratory disorders, specifically, asthma.

Asthma is a common chronic inflammatory disease of the airways characterized by variable and recurring symptoms, reversible airflow obstruction and bronchospasm. Common symptoms include wheezing, coughing, chest tightness, and shortness of breath.

Asthma is thought to be caused by a combination of genetic and environmental factors. Its diagnosis is usually based on the pattern of symptoms, response to therapy over time and spirometry. It is clinically classified according to the frequency of symptoms, forced expiratory volume in one second (FEV1), and peak expiratory flow rate. Asthma may also be classified as atopic (extrinsic) or non-atopic (intrinsic) where atopy refers to a predisposition toward developing type 1 hypersensitivity reactions.

Treatment of acute symptoms of asthma is usually with an inhaled short-acting beta-2 agonists (such as salbutamol) and oral corticosteroids. In very severe cases, intravenous corticosteroids, magnesium sulfate, and hospitalization may be required. Symptoms can be prevented by avoiding triggers, such as allergens and irritants, and by the use of inhaled corticosteroids. Long-acting beta agonists (LABA) or antileukotriene agents may be used in addition to inhaled corticosteroids if asthma symptoms remain uncontrolled.

Asthma is the result of chronic inflammation of the airways which subsequently results in increased contractability of the surrounding smooth muscles. This among other factors leads to bouts of narrowing of the airway and the classic symptoms of wheezing. The narrowing is typically reversible with or without treatment. Occasionally the airways themselves change. Typical changes in the airways include an increase in eosinophils and thickening of the lamina reticularis. Chronically the airways' smooth muscle may increase in size along with an increase in the numbers of mucous glands. Other cell types involved include: T lymphocytes, macrophages, and neutrophils. There may also be involvement of other components of the immune system including: cytokines, chemokines, histamine, and leukotrienes.

While there is no cure for asthma, symptoms can typically be improved. A specific, customized plan for proactively monitoring and managing symptoms should be created. This plan should include the reduction of exposure to allergens, testing to assess the severity of symptoms, and the usage of medications. The treatment plan should be written down and advise adjustments to treatment according to changes in symptoms.

Importantly, the metal-siderophore, specifically, metal-nocardamine complexes of the invention provide an additional therapeutic dimension to the current available medications, as they do not only serve as preventive measures against the development of the described respiratory disorder pathologies, but also diminish the ensuing tissue damage.

It should be noted that medications for asthma and other respiratory-associated disorders are typically provided as metered-dose inhalers (MDIs) in combination with an asthma spacer or as a dry powder inhaler. The spacer is a plastic cylinder that mixes the medication with air, making it easier to receive a full dose of the drug. A nebulizer may also be used. The metal-DFO E complexes of the invention may be therefore administered using such MDIs, and may be also combined with any other asthma medications, specifically those indicated above.

As indicated above, the present invention contemplates methods for the treatment of various immune-related respiratory diseases. In addition to asthma, such respiratory diseases may include any other acute allergy manifestations in airways, chronic rhinosinusitis (CRS), allergic rhinitis, COPD, nasal polyposis (NP), vasomotor rhinitis, airways hyper-responsiveness, cystic fibrosis and lung fibrosis, or allergic sinusitis. The invention therefore provides methods, combined compositions and kits for preventing, treating, ameliorating or inhibiting any of the respiratory diseases described above.

Thus, in certain embodiments, the invention provides methods, compositions and kits for treating sinusitis. Sinusitis is inflammation of the paranasal sinuses, which may be due to infection, allergy or autoimmune issues. Most cases are due to a viral infection and resolve over the course of 10 days. It is a common condition with more than 24 million cases occurring in the United States annually.

Chronic sinusitis, by definition, lasts longer than three months and can be caused by many different diseases that share chronic inflammation of the sinuses as a common symptom. Chronic sinusitis cases are subdivided into cases with polyps and cases without polyps. When polyps are present, the condition is called chronic hyperplastic sinusitis; however, the causes are poorly understood and may include allergy, environmental factors such as dust or pollution, bacterial infection, or fungus (either allergic, infective, or reactive). Non-allergic factors, such as vasomotor rhinitis, can also cause chronic sinus problems.

Allergic rhinitis, pollinosis or hay fever that may be treated by the method of the invention, is an allergic inflammation of the nasal airways. It occurs when an allergen such as pollen or dust is inhaled by an individual with a sensitized immune system, and triggers antibody production. These antibodies mostly bind to mast cells, which contain histamine. When the mast cells are stimulated by pollen and dust, histamine (and other chemicals) is released. This causes itching, swelling and mucus production. Symptoms vary in severity between individuals. Very sensitive individuals can experience hives or other rashes.

Chronic obstructive pulmonary disease (COPD), also known as chronic obstructive lung disease (COLD), chronic obstructive airway disease (COAD), chronic airflow limitation (CAL) and chronic obstructive respiratory disease (CORD), refers to chronic bronchitis and emphysema, a pair of commonly co-existing diseases of the lungs in which the airways become narrowed. This leads to a limitation of the flow of air to and from the lungs causing shortness of breath. In contrast to asthma, the limitation of airflow is poorly reversible and usually gets progressively worse over time. COPD is caused by noxious particles or gas, most commonly from tobacco smoking, which triggers an abnormal inflammatory response in the lung. The inflammatory response in the larger airways is known as chronic bronchitis, which is diagnosed clinically when people regularly cough up sputum. In the alveoli, the inflammatory response causes destruction of the tissues of the lung, a process known as emphysema. The natural course of COPD is characterized by occasional sudden worsening of symptoms called acute exacerbations, most of which are caused by infections or air pollution. The methods, combined compositions and kits of the invention are applicable for treating COAD and COPD.

Still further, the method of the invention may be used for treating nasal polyps. Nasal polyps are polypoidal masses arising mainly from the mucous membranes of the nose and paranasal sinuses. They are overgrowths of the mucosa that frequently accompany allergic rhinitis. They are freely moveable and non-tender. Nasal polyps are usually classified into antrochoanal polyps and ethmoidal polyps. Antrochoanal polyps arise from the maxillary sinuses and are the much less common, ethmoidal polyps arise from the ethmoidal sinuses. Antrochoanal polyps are usually single and unilateral whereas ethmoidal polyps are multiple and bilateral.

Non-allergic rhinitis refers to runny nose that is not due to allergy. Non-allergic rhinitis can be classified as either non-inflammatory or inflammatory rhinitis. One very common type of non-inflammatory, non-allergic rhinitis that is sometimes confused with allergy is called vasomotor rhinitis, in which certain non-allergic triggers such as smells, fumes, smoke, dusts, and temperature changes, cause rhinitis. It is thought that these non-allergic triggers cause dilation of the blood vessels in the lining of the nose, which results in swelling, and drainage. Vasomotor rhinitis can coexist with allergic rhinitis, and this is called "mixed rhinitis." Vasomotor rhinitis appears to be significantly more common in women than men, leading some researchers to believe that hormones play a role. In general, age of onset occurs after 20 years of age, in contrast to allergic rhinitis which can be developed at any age. Individuals suffering from vasomotor rhinitis typically experience symptoms year-round, though symptoms may exacerbate in the spring and autumn when rapid weather changes are more common. An estimated 17 million United States citizens have vasomotor rhinitis. The antihistamine azelastine has been shown to be effective for allergic, mixed and vasomotor rhinitis.

Airway hyperresponsiveness (or other combinations with bronchial or hyper-reactivity) is a state characterized by easily triggered bronchospasm (contraction of the bronchioles or small airways). Airway hyperresponsiveness can be assessed with a bronchial challenge test. This most often uses products like metacholine or histamine. These chemicals trigger bronchospasm in normal individuals as well, but people with bronchial hyperresponsiveness have a lower threshold. Bronchial hyperresponsiveness is a hallmark of asthma but also occurs frequently in people suffering from chronic obstructive pulmonary disease (COPD).

Cystic fibrosis (also known as CF) that is another example for conditions that may be treated by the method of the invention is a common disease which affects the entire body, causing progressive disability and often early death. The name cystic fibrosis refers to the characteristic scarring (fibrosis) and cyst formation within the pancreas. Difficulty breathing is the most serious symptom and results from frequent lung infections that are treated, though not cured, by antibiotics and other medications. A multitude of other symptoms, including sinus infections, poor growth, diarrhea, and infertility result from the effects of CF on other parts of the body. CF is caused by a mutation in the gene for the protein cystic fibrosis transmembrane conductance regulator (CFTR), and is considered as an autosomal recessive disease.

Pulmonary fibrosis is the formation or development of excess fibrous connective tissue (fibrosis) in the lungs. It can be described as "scarring of the lung". Pulmonary fibrosis involves gradual replacement of normal lung parenchyma with fibrotic tissue. Thickening of scar tissue causes irreversible decrease in oxygen diffusion capacity. In addition, decreased compliance makes pulmonary fibrosis a restrictive lung disease. It is the main cause of restrictive lung disease that is intrinsic to the lung parenchyma.

Some embodiments relate to methods according to the invention, particularly for treating respiratory diseases. According to one embodiment, such compositions may be particularly adapted for pulmonary delivery by oral or nasal inhalation. More specifically, pulmonary delivery may require the use of liquid nebulizers, aerosol-based metered dose inhalers (MDI's), or dry powder dispersion devices.

In further embodiments, the present invention provides a method of treating or preventing an infectious disease or condition in a mammalian subject caused by any pathogen, specifically, at least one of, bacterial pathogen, a viral pathogen and a parasite. More specifically, the method comprises the step of administering to a subject in need the effective amount of the metal-nocardamine complexes of the invention or any compositions thereof.

*"Infection"* as used herein, is the invasion of an organism's body tissues by disease-causing agents, their multiplication, and the reaction of host tissues to these organisms and the toxins they produce. Infectious disease, also known as transmissible disease or communicable disease, is illness resulting from an infection. It should be appreciated that an infectious disease as used herein also encompasses any infectious disease caused by a pathogenic agent. Pathogenic agents include bacteria, viruses, prokaryotic microorganisms, lower eukaryotic microorganisms, complex eukaryotic organisms, prions, parasites, yeasts, nematodes such as parasitic roundworms and pinworms, arthropods such as ticks, mites, fleas, and lice, fungi such as ringworm, and other macroparasites such as tapeworms and other helminths.

Prokaryotic microorganisms includes bacteria as detailed herein after, for example, Gram positive, Gram negative, Gram variable bacteria, acid fast organisms and intracellular bacteria.

A lower eukaryotic organism includes a yeast or fungus such as but not limited to *Pneumocystis carinii, Candida albicans,* Aspergillus, *Histoplasma capsulatum, Blastomyces dermatitidis, Cryptococcus neoformans, Trichophyton* and *Microsporum.*

A complex eukaryotic organism includes worms, insects, arachnids, nematodes, aemobe, *Entamoeba histolytica, Giardia lamblia, Trichomonas vaginalis, Trypanosoma brucei gambiense, Trypanosoma cruzi, Balantidium coli, Toxoplasma gondii, Cryptosporidium* or *Leishmania.*

The term "viruses" is used in its broadest sense to include viruses of the families adenoviruses, papovaviruses, herpesviridae (simplex, varicella zoster, Epstein-Barr, CMV), hepatitis A, hepatitis B, hepatitis C, influenza viruses A and B, pox viruses: smallpox, vaccinia, rhinoviruses, poliovirus, rubella virus, arboviruses, rabies virus, flaviviruses, measles virus, mumps virus, HIV, HTLV I and II.

The term "fungi" includes for example, fungi that cause diseases such as ringworm, histoplasmosis, blastomycosis, aspergillosis, cryptococcosis, sporotrichosis, coccidioidomycosis, paracoccidio-idoinycosis, and candidiasis.

The term "parasite" includes, but is not limited to, infections caused by somatic tapeworms, blood flukes, tissue roundworms, ameba, and Plasmodium, Trypanosoma, Leishmania, and Toxoplasma species.

It should be noted that the complex of nocardamine with at least one metal may chelate labile iron and/or copper, reducing the availability of labile iron for synthetic and proliferative purposes of the microorganism, thereby inhibiting growth of pathogenic microorganisms, and diminishing the extent of infection/inflammation.

Thus, in some embodiments, the present invention provides methods for treating an infectious disease caused by a bacterial pathogen.

In some specific embodiments, the bacterial pathogen may be at least one of enteropathogenic *Escherichia coli (EPEC), Pseudomonas aeruginosa* and *Staphylococcus aureus.*

Bacterial infection is an example for inflammation-related disorder. The present invention further provides the method for use of the complexes of the invention or any combinations thereof, as well as pharmaceutical compositions thereof, for preventing, treating, ameliorating or inhibiting bacterial infections. As presented in Figures 8 and 9, the anti-bacterial effect of zinc-nocardamine was demonstrated on the following types of pathogenic bacteria: *E.coli, P. aeruginosa* and *S. aureus.* More specifically, a growth inhibition of *E.coli, P. aeruginosa* and *S. aureus* was demonstrated by the bactericidal activity (inhibition of bacterial viability) of the complex of the invention.

More specifically, the term *"bacterial infections"* relates to infection caused by bacteria. The term *"bacteria"* (in singular a "bacterium") in this context refers to any type of a single celled microbe. This term encompasses herein bacteria belonging to general classes according to their basic shapes, namely spherical (cocci), rod (bacilli), spiral (spirilla), comma (vibrios) or corkscrew (spirochaetes), as well as bacteria that exist as single cells, in pairs, chains or clusters.

In more specific embodiments, the term *"bacteria"* specifically refers to Gram positive, Gram negative or acid fast organisms. The Gram-positive bacteria can be recognized as retaining the crystal violet stain used in the Gram staining method of bacterial differentiation, and therefore appear to be purple-colored under a microscope. The Gram-negative bacteria do not retain the crystal violet, making positive identification possible. In other words, the term "bacteria" applies herein to bacteria with a thicker peptidoglycan layer in the cell wall outside the cell membrane (Gram-positive), and to bacteria with a thin peptidoglycan layer of their cell wall that is sandwiched between an inner cytoplasmic cell membrane and a bacterial outer membrane (Gram-negative).

The present invention further concerns the biological activities of the zinc-nocardamine complex: it acts as an anti-inflammatory agent, as a bacteriostatic and/or bacteriocidal agent, and as a strong chelator of labile and redox-active iron.

In some embodiments, examples of bacteria contemplated herein include the species of the genera *Treponema sp., Borrelia sp., Neisseria sp., Legionella sp., Bordetella sp., Escherichia sp., Salmonella sp., Shigella sp., Klebsiella sp., Yersinia sp., Vibrio sp., Hemophilus sp., Rickettsia sp., Chlamydia sp., Mycoplasma sp., Staphylococcus sp., Streptococcus sp., Bacillus sp., Clostridium sp., Corynebacterium sp., Proprionibacterium sp., Mycobacterium sp., Ureaplasma sp.* and *Listeria sp.* In yet some more specific embodiments, bacterial pathogens in the context of the invention may include but are not limited to enteropathogenic *Escherichia coli (EPEC), Pseudomonas aeruginosa, Staphylococcus aureus, Streptococcus pyogenes, Clostidium difficile, Enterococcus faecium, Klebsiella pneumonia, Acinetobacter baumanni and Enterobacter species, Mycobacterum tuberculosis, Alcaligenes faecalis, Neisseria meningitis, Prevotella intermedia, Porphyromonas gingivalis, species of Salmonella, Shigella, Proteus, Providencia, Enterobacter* and *Morganella.*

Thus, the composition of the invention exhibits substantial antibacterial effect, which makes it applicable for the treatment of infection-mediated disorders. It should be also appreciated that the beneficial antibacterial effect of the metal-DFO E complexes of the invention or any compositions of the invention may be enhanced by combination thereof with other known anti-bacterial agents.

In yet some further embodiments, the methods of the invention may be particularly applicable for treating and/or preventing infectious diseases caused by a viral pathogen, specifically, at least one of herpesviruses, (simplex, varicella-zoster), hepatitis A, hepatitis B, hepatitis C, influenza viruses A and B, adenoviruses, papovaviruses, Epstein-Barr, CMV, pox viruses: smallpox, vaccinia, hepatitis B, rhinoviruses, poliovirus, rubella virus, arboviruses, rabies virus, flaviviruses, measles virus, mumps virus, HIV, HTLV I and II.

In yet some embodiments, the methods of the invention may be applicable for treating proliferative disorder in a subject in need thereof. In some specific embodiments, the method of the invention may comprise the step of administering to the subject a therapeutically effective amount of at least one complex of nocardamine with at least one metal, or any pharmaceutical compositions as disclosed by the invention, carriers, matrix or vehicles comprising the same. It should be further appreciated that the method of the invention may be specifically relevant for proliferative disorders that are malignancies associated with at least one of elevated pro-inflammatory cytokines and reduced levels of anti-inflammatory cytokines.

The term *"proliferative disorder"* means cell division and growth that is not part of normal cellular turnover, metabolism, growth, or propagation of the whole organism. Unwanted proliferation of cells is seen in tumors and other pathological proliferation of cells, it does not serve normal function, and for the most part will continue unbridled at a growth rate exceeding that of cells of a normal tissue in the absence of outside intervention. A pathological state that ensues because of the unwanted proliferation of cells is referred herein as a "hyper proliferative disease" or "hyper proliferative disorder." It should be noted that the term "proliferative disorder", "cancer", "tumor" and "malignancy" all relate equivalently to a hyperplasia of a tissue or organ. In general, the compositions and methods of the present invention may be used in the treatment of non-solid and solid tumors.

Still further, malignancy, as contemplated in the present invention may be any one of carcinomas, melanomas, lymphomas, leukemias, myeloma and sarcomas.

Carcinoma as used herein refers to an invasive malignant tumor consisting of transformed epithelial cells. Alternatively, it refers to a malignant tumor composed of transformed cells of unknown histogenesis, but which possess specific molecular or histological characteristics that are associated with epithelial cells, such as the production of cytokeratins or intercellular bridges.

Melanoma as used herein is a malignant tumor of melanocytes. Melanocytes are cells that produce the dark pigment, melanin, which is responsible for the color of skin. They predominantly occur in skin, but are also found in other parts of the body, including the bowel and the eye. Melanoma can occur in any part of the body that contains melanocytes.

Leukemia refers to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease-acute or chronic; (2) the type of cell involved; myeloid (myelogenous), lymphoid (lymphogenous), or monocytic; and (3) the increase or non-increase in the number of abnormal cells in the blood-leukemic or aleukemic (subleukemic).

Sarcoma is a cancer that arises from transformed connective tissue cells. These cells originate from embryonic mesoderm, or middle layer, which forms the bone, cartilage, and fat tissues. This is in contrast to carcinomas, which originate in the epithelium. The epithelium lines the surface of structures throughout the body, and is the origin of cancers in the breast, colon, and pancreas.

Myeloma as mentioned herein is a cancer of plasma cells, a type of white blood cell normally responsible for the production of antibodies. Collections of abnormal cells accumulate in bones, where they cause bone lesions, and in the bone marrow where they interfere with the production of normal blood cells. Most cases of myeloma also feature the production of a paraprotein, an abnormal antibody that can cause kidney problems and interferes with the production of normal antibodies leading to immunodeficiency. Hypercalcemia (high calcium levels) is often encountered.

Lymphoma is a cancer in the lymphatic cells of the immune system. Typically, lymphomas present as a solid tumor of lymphoid cells. These malignant cells often originate in lymph nodes, presenting as an enlargement of the node (a tumor). It can also affect other organs in which case it is referred to as extranodal lymphoma. Non limiting examples for lymphoma include Hodgkin's disease, non-Hodgkin's lymphomas and Burkitt's lymphoma.

Further malignancies that may find utility in the present invention can comprise but are not limited to hematological malignancies (including lymphoma, leukemia and myeloproliferative disorders, as described above), hypoplastic and aplastic anemia (both virally induced and idiopathic), myelodysplastic syndromes, all types of paraneoplastic syndromes (both immune mediated and idiopathic) and solid tumors (including GI tract, colon, lung, liver, breast, prostate, pancreas and Kaposi's sarcoma. The invention may be applicable as well for the treatment or inhibition of solid tumors such as tumors in lip and oral cavity, pharynx, larynx, paranasal sinuses, major salivary glands, thyroid gland, esophagus, stomach, small intestine, colon, colorectum, anal canal, liver, gallbladder, extrahepatic bile ducts, ampulla of Vater (hepatopancreatic duct), exocrine pancreas, lung, pleural mesothelioma, bone, soft tissue sarcoma, carcinoma and malignant melanoma of the skin, breast, vulva, vagina, cervix uteri, corpus uteri, ovary, fallopian tube, gestational trophoblastic tumors, penis, prostate, testis, kidney, renal pelvis, ureter, urinary bladder, urethra, carcinoma of the eyelid, carcinoma of the conjunctiva, malignant melanoma of the conjunctiva, malignant melanoma of the uvea, retinoblastoma, carcinoma of the lacrimal gland, sarcoma of the orbit, brain, spinal cord, vascular system, hemangiosarcoma and Kaposi's sarcoma

In some further embodiments the present invention provides methods for treatment of neurodegenerative disorder in a subject in need thereof, comprising the step of administering to said subject a therapeutically effective amount of at least one complex of nocardamine with at least one metal, or any combinations thereof or any pharmaceutical compositions, carriers, matrix or vehicles comprising the same. In some embodiments, neurodegenerative disorder is at least one of a group of "neurodegenerative diseases" characterized by neurodegenerative processes, and include at least one of the following: amyotrophic lateral sclerosis, Parkinson's, Alzheimer's, and Huntington's. Such diseases are incurable, resulting in progressive degeneration and/or death of neuron cells.

The term *"Neurodegeneration"* is the general term for the progressive loss of structure or function of neurons, leading to their death. The greatest risk factor for neurodegenerative diseases is aging. Mitochondrial DNA mutations as well as oxidative stress both contribute to aging. Many of these diseases are late-onset, meaning there is some factor that change as a person ages, for each disease. One constant factor is that in each disease, neurons gradually lose function as the disease progresses with age.

In yet some further embodiments, the methods of the invention may be applicable for treating spinal cord injury and trauma.

Spinal cord injury (SCI) is the damage to the spinal cord that causes changes in its function. Injuries can occur at any level of the spinal cord and can be classified as *complete injury,* a total loss of sensation and muscle function, or *incomplete,* meaning some nervous signals are able to travel past the injured area of the cord. Depending on the location and severity of damage along the spinal cord, the symptoms can vary widely, from mild such as pain or numbness to severe, such as paralysis and incontinence.

Spinal cord injury can be traumatic or non-traumatic, and can be classified into three types based on cause: mechanical forces, toxic, and ischemic (from insufficient blood supply). The damage can also be divided into primary and secondary injury: in primary injury, the cell death that occurs immediately at the original injury; in secondary injury, biochemical cascades that are initiated by the original insult can cause further tissue damage including activation of the ischemic cascade, inflammation, swelling, cell suicide, and neurotransmitter imbalances. They can take place for minutes or weeks following the injury.

Usually the damage results from physical trauma such as car accidents, gunshots, falls, or sports injuries, but it can also result from other causes such as infection, insufficient blood flow, or pressure from a tumor.

Non-physical-traumatic SCI ranges from 30 to 80% of all SCI. SCI may occur in infection, intervertebral disc disease, and spinal cord vascular disease. Spontaneous bleeding can occur within or outside of the protective membranes that line the cord, and intervertebral disks can herniate. Damage can result from dysfunction of the blood vessels, as in arteriovenous malformation, or when a blood clot becomes lodged in a blood vessel and cuts off blood supply to the cord. When systemic blood pressure drops, blood flow to the spinal cord may be reduced, potentially causing a loss of sensation and voluntary movement in the areas supplied by the affected level of the spinal cord. Congenital conditions and tumors that compress the cord can also cause SCI, as can vertebral spondylosis and ischemia. Multiple sclerosis is a disease that can damage the spinal cord, as can infectious or inflammatory conditions such as tuberculosis, herpes zoster or herpes simplex, meningitis, myelitis, and syphilis. In developed countries, the most common cause of non-traumatic SCI is degenerative diseases, followed by tumors; in many developing countries the leading cause is infection such as HIV and tuberculosis.

SCI is also classified by the degree of impairment. The *International Standards for Neurological Classification of Spinal Cord Injury* (ISNCSCI), published by the American Spinal Injury Association (ASIA), is widely used to document sensory and motor impairments following SCI. It is based on neurological responses, touch and pinprick sensations tested in each dermatome, and strength of the muscles that control key motions on both sides of the body.

The prognosis ranges widely, from full recovery to permanent paralysis of the four limbs, called tetraplegia (also called quadriplegia) in injuries at the level of the neck, and paraplegia (paralysis of two limbs) in lower injuries. Complications that can occur in the short and long term after injury include muscle atrophy, pressure sores, infections, and respiratory problems.

Swelling can cause further damage to the spinal cord by reducing the blood supply and causing ischemia, which can give rise to an ischemic cascade with a release of toxins that damage neurons.

It should be further appreciated that the method of the invention may be applicable for any of the spinal injuries described herein and for any related conditions or complications.

In further embodiments the present invention provides methods for treatment of ischemic disorders in a subject in need thereof, comprising the step of administering to said subject a therapeutically effective amount of at least one complex of nocardamine with at least one metal, any combinations thereof, or any pharmaceutical compositions, carriers, matrix or vehicles comprising the same, wherein ischemic disorder is at least one of: atherosclerosis of arteries in the heart, brain, arms, legs, pelvis, and kidneys.

The term *"ischemia"* means a "reduced blood supply". The term *"ischemic disorder"* as used herein refers to Ischemic Vascular Disease - a group of disorders characterized by a waxy substance called plaque that builds up inside blood vessels, and thereby restricts the normal flow of blood. When plaque builds up in the arteries, the condition is called *"atherosclerosis".* Atherosclerosis can affect any artery in the body, including arteries in the heart, brain, arms, legs, pelvis, and kidneys. As a result, different diseases may develop based on which arteries are affected.

*"Ischemic Vascular Disease"* (IVD) is a term that includes a group of diseases caused by the build-up of plaque. Following are the specific non limiting examples of IVD:
Still further, in some embodiments, the methods of the invention may be applicable for Coronary Heart Disease (CHD). Coronary heart disease is where atherosclerosis affects the coronary arteries in the heart. If the flow of oxygen-rich blood to the heart muscle is reduced or blocked, angina (pain) or a heart attack, respectively, may occur.

In some embodiments, the methods of the invention may be applicable for heart attack. A heart attack occurs if the flow of oxygen-rich blood to a section of heart muscle is blocked. If blood flow isn't restored quickly, the section of heart muscle begins to die.

Without quick treatment, a heart attack can lead to serious problems and even death. Still further, in some embodiments, the methods of the invention may be applicable for Carotid Artery Disease (CAD). Carotid artery disease occurs if plaque builds up in the arteries on any side of the carotid arteries. These arteries supply oxygen-rich blood to the brain. Thus, if blood flow to the brain is reduced or blocked, even for a few minutes, the lack of oxygen may cause damage, or death of brain cells, which is called stroke. If brain cells die or are damaged because of a stroke, symptoms occur in the parts of the body that these brain cells control. Examples of stroke symptoms include sudden weakness; paralysis or numbness of the face, arms, or legs; trouble speaking or understanding speech; and trouble seeing.

In yet some further embodiments, the methods of the invention may be applicable for Peripheral Arterial Disease (PAD). PAD is a disease in which plaque builds up in the arteries of the legs or arms. Blocked blood flow to the legs can cause pain and numbness. It also can raise the risk of getting an infection in the affected limbs. If severe enough, blocked blood flow can cause gangrene (tissue death).

As mentioned above, all types of ischemic vascular disease are caused by atherosclerosis. Atherosclerosis may start when certain factors damage the inner layers of the arteries. These factors include: smoking, high amounts of certain fats and cholesterol in the blood, high blood pressure and high amounts of sugar in the blood due to insulin resistance or diabetes.

In some further embodiments the present invention provides methods for treatment of metabolic disorders in a subject in need thereof, comprising the step of administering to said subject a therapeutically effective amount of at least one complex of nocardamine with at least one metal, any combinations thereof, or any pharmaceutical compositions, carriers, matrix or vehicles comprising the same. In some embodiments, the methods of the invention may be specifically relevant for metabolic disorder, specifically, at least one of diabetes mellitus type I, diabetes mellitus type II and any diabetic related conditions.

Metabolism is the process by which the body uses food to get or make energy. Food is made up of proteins, carbohydrates, and fats. Chemicals in the digestive system break the food parts down into sugars and acids, and the body can use these break down products right away, or it can store the energy in body tissues, such as liver, muscles, and body fat.

A *"metabolic disorder"* occurs when abnormal chemical reactions disrupt this process. An individual can develop a metabolic disorder when some organs, such as liver or pancreas, become diseased or do not function normally. Diabetes is a non-limited example of metabolic disorder. Diabetes mellitus (DM), commonly referred to as diabetes, is a group of metabolic diseases in which there are high blood sugar levels over a prolonged period. If left untreated, diabetes can cause many complications. Acute complications include diabetic ketoacidosis and nonketotic hyperosmolar coma. Serious long-term complications include cardiovascular disease, stroke, chronic kidney failure, foot ulcers, and damage to the eyes. Diabetes is due to either not producing enough insulin by the pancreas (diabetes mellitus type I), or the lack of proper response by the cells of the body to the insulin produced (diabetes mellitus type II).

There is growing evidence for a link between inflammation and the pathogenesis of Type 2 diabetes. This evolving concept, which suggests that insulin resistance and type 2 diabetes may have an immune component, provides a new avenue for anti-inflammatory therapy for type 2 diabetes. Therefore, based on its anti-inflammatory effects, the composition, as well as methods of the invention may be used for the treatment and/or amelioration of an autoimmune disorder, such as diabetes.

Diabetes mellitus, is a syndrome characterized by disordered metabolism and inappropriately high blood sugar (hyperglycemia) resulting from either low levels of the hormone insulin or from abnormal resistance to insulin's effects coupled with inadequate levels of insulin secretion to compensate. The characteristic symptoms are excessive urine production (polyuria), excessive thirst and increased fluid intake (polydipsia), and blurred vision; these symptoms are likely absent if the blood sugar is only mildly elevated.

There are three main forms of diabetes: type 1, type 2 and gestational diabetes (occurs during pregnancy). Type 1 diabetes mellitus is characterized by loss of the insulin-producing beta cells of the islets of Langerhans in the pancreas, leading to a deficiency of insulin. The main cause of this beta cell loss is a T-cell mediated autoimmune attack. There is no known preventative measure that can be taken against type 1 diabetes. Most affected people are otherwise healthy and of a healthy weight when onset occurs. Sensitivity and responsiveness to insulin are usually normal, especially in the early stages. Type 1 diabetes can affect children or adults and was traditionally termed "juvenile diabetes" as it represents a majority of cases of diabetes affecting children.

In yet another specific embodiment, the composition of the invention may be used for preventing, treating, ameliorating or inhibiting diabetes type 2. Diabetes mellitus type 2, or non-insulin-dependent diabetes mellitus (NIDDM) or adult-onset diabetes, is a metabolic disorder that is characterized by high blood glucose in the context of insulin resistance and relative insulin deficiency. As the condition progresses, medications may be needed. Long-term complications from high blood sugar include an increased risk of heart attacks, strokes, amputation, and kidney failure. There are many factors which can potentially give rise to or exacerbate type 2 diabetes. These include obesity, hypertension, elevated cholesterol (combined hyperlipidemia), and with the condition often termed metabolic syndrome (it is also known as Syndrome X, Reavan's syndrome, or CHAOS). Other causes include acromegaly, Cushing's syndrome, thyrotoxicosis, pheochromocytoma, chronic pancreatitis, cancer and drugs. Additional factors found to increase the risk of type 2 diabetes include aging, high-fat diets and a less active lifestyle.

Insulin resistance means that body cells do not respond appropriately when insulin is present. Unlike type 1 diabetes mellitus, insulin resistance is generally "post-receptor", meaning it is a problem with the cells that respond to insulin rather than a problem with the production of insulin. Severe complications can result from improperly managed type 2 diabetes, including renal failure, erectile dysfunction, blindness, slow healing wounds (including surgical incisions), and arterial disease, including coronary artery disease. The onset of type 2 has been most common in middle age and later life, although it is being more frequently seen in adolescents and young adults due to an increase in child obesity and inactivity.

It should be further appreciated that the methods, kits and complexes of the invention may be applicable for treating diabetes-related conditions. It is understood that the interchangeably used terms "associated" and "related", when referring to pathologies herein, mean diseases, disorders, conditions, or any pathologies which at least one of: share causalities, co-exist at a higher than coincidental frequency, or where at least one disease, disorder condition or pathology causes the second disease, disorder, condition or pathology. Such conditions may include for example, eye related complications (cataract, glaucoma, retinopathy), neuropathy, nephropathy, cardiomyopathy, stroke, hyper tension, peripheral arterial disease and sores and any ulcers, chronic ulcers or any slow healing ulcers or skin or organ injuries associated with a diabetic condition. In accordance with the present invention the undesired side effect treated or prevented is preferably an undesired side effect related to the eye and/or vision such as cataract.

The methods of treatment and uses of the invention may also be utilized for the benefit of subjects suffering from diabetes-related or associated diseases or disorders, comprising hyperinsulinaemia, dyslipidaemia, hypercholesterolemia, impaired glucose tolerance, hypertension, cardiovascular disease, diabetic cardiomyopathy, diabetic cardiac dysrhytmia, atherosclerosis, diabetic nephropathy, glomerulonephritis, glomerular sclerosis, nephrotic syndrome, hypertensive nephrosclerosis, end stage renal disease, microalbuminuria and albuminuria.

Hyperinsulinemia, or hyperinsulinaemia, as used herein, is a condition in which there are excess levels of circulating insulin in the blood. Also known as pre-diabetes, insulin resistance, and syndrome X, it is commonly associated with PCOS (Polycystic Ovarian Syndrome) in females. Hyperinsulinemia is often mistaken for diabetes or hypoglycaemia, both of which are separate conditions. Hyperinsulinemia can develop into diabetes if unmonitored and untreated, and may remain present when diabetes occurs. It is not caused by diabetes, as is commonly believed. Hyperinsulinemia may cause hypoglycaemia in some patients.

Dyslipidemia as used herein is a disruption in the amount of lipids in the blood. In societies of developed countries, most dyslipidemias are hyperlipidemias; that is, an elevation of lipids in the blood, often due to diet and lifestyle. The prolonged elevation of insulin levels can lead to dyslipidemia. Increased levels of O-GlcNAc transferase (OGT) are known to cause dyslipidaemia.

Impaired glucose tolerance (IGT) is a pre-diabetic state of dysglycemia that is associated with insulin resistance and increased risk of cardiovascular pathology. IGT may precede type II diabetes mellitus by many years.

Hypertension (HTN) or high blood pressure as used herein is a chronic medical condition in which the blood pressure in the arteries is elevated. It is the opposite of hypotension. It is classified as either primary (essential) or secondary. About 90-95% of cases are termed "primary hypertension", which refers to high blood pressure for which no medical cause can be found. The remaining 5-10% of cases (Secondary hypertension) are caused by other conditions that affect the kidneys, arteries, heart, or endocrine system. Persistent hypertension is one of the risk factors for strokes, heart attacks, heart failure and arterial aneurysm, and is a leading cause of chronic kidney failure.

Cardiomyopathy, as used herein is deterioration of myocardium functioning e.g. a clinical or sub-clinical condition diagnosed when ventricular dysfunction develops in patients with diabetes in the absence of coronary atherosclerosis and hypertension. It is characterized functionally by ventricular dilation, myocyte hypertrophy, interstitial fibrosis, and decreased or preserved systolic function in the presence of a diastolic dysfunction.

Atherosclerosis (also known as arteriosclerotic vascular disease or ASVD) is a condition in which an artery wall thickens as the result of a build-up of fatty materials such as cholesterol. It is a syndrome affecting arterial blood vessels, a chronic inflammatory response in the walls of arteries, in large part due to the accumulation of macrophage white blood cells and promoted by low-density lipoproteins (plasma proteins that carry cholesterol and triglycerides) without adequate removal of fats and cholesterol from the macrophages by functional high density lipoproteins (HDL). It is commonly referred to as a hardening or furring of the arteries. It is caused by the formation of multiple plaques within the arteries.

Diabetic nephropathy (nephropatia diabetica), also known as Kimmelstiel-Wilson syndrome, or nodular diabetic glomerulosclerosis and intercapillary glomerulonephritis, is a progressive kidney disease caused by angiopathy of capillaries in the kidney glomeruli. It is characterized by nephrotic syndrome and diffuse glomerulosclerosis. It is due to longstanding diabetes mellitus, and is a prime indication for dialysis in many Western countries.

Glomerulonephritis, also known as glomerular nephritis (GN), is a renal disease characterized by inflammation of the glomeruli, or small blood vessels in the kidneys. It may present with isolated hematuria and/or proteinuria (blood and/or protein presence in the urine); or as a nephrotic syndrome, a nephritic syndrome, acute renal failure, or chronic renal failure. They are categorized into several different pathological patterns, which are broadly grouped into non-proliferative or proliferative types. Primary causes are ones which are intrinsic to the kidney, whilst secondary causes are associated with certain infections (bacterial, viral or parasitic pathogens), drugs, systemic disorders (SLE, vasculitis) or diabetes.

Glomerular sclerosis refers to a hardening of the glomerulus in the kidney. It is a general term to describe scarring of the kidney glomeruli. Proteinuria (large amounts of protein in urine) is one of the signs of glomerulosclerosis. Diabetes is a frequent cause of glomerular sclerosis.

Nephrotic syndrome is a nonspecific disorder in which the kidneys are damaged, causing them to leak large amounts of protein (proteinuria at least 3.5 grams per day per 1.73m² body surface area) from the blood into the urine.

Kidneys affected by nephrotic syndrome have small pores in the podocytes, large enough to permit proteinuria (and subsequently hypoalbuminemia, because some of the protein albumin has gone from the blood to the urine) but not large enough to allow cells through (hence no hematuria). By contrast, in nephritic syndrome, RBCs pass through the pores, causing hematuria. Diabetes is often an underlying cause of nephrotic syndrome.

Hypertensive nephropathy, or hypertensive nephrosclerosis, or hypertensive renal disease, is a medical condition referring to damage to the kidney due to chronic high blood pressure. In the kidneys, as a result of benign arterial hypertension, hyaline (pink, amorphous, homogeneous material) accumulates in the wall of small arteries and arterioles, producing the thickening of their walls and the narrowing of the lumina - hyaline arteriolosclerosis. Consequent ischemia will produce tubular atrophy, interstitial fibrosis, glomerular alterations (smaller glomeruli with different degrees of hyalinization - from mild to sclerosis of glomeruli) and periglomerular fibrosis. In advanced stages, renal failure will occur. Functional nephrons have dilated tubules, often with hyaline casts in the lumens. Additional complications often associated with hypertensive nephropathy include glomerular damage resulting in proteinuria and hematuria.

End-stage renal disease is an advanced stage of chronic kidney disease (CKD), also known as chronic renal disease. CKD manifests as a progressive loss in renal function over a period of months or years. The symptoms of worsening kidney function are unspecific, and might include feeling generally unwell and experiencing a reduced appetite. Recent professional guidelines classify the severity of chronic kidney disease in five stages, with stage 1 being the mildest and usually causing few symptoms and stage 5 being a severe illness with poor life expectancy if untreated. Stage 5 CKD is also called established chronic kidney disease and is synonymous with the now outdated terms end-stage renal disease (ESRD), chronic kidney failure (CKF) or chronic renal failure (CRF).

As indicated above, the methods of the invention may be used for treating any immune-related disorder. It should be noted that an "Immune-related disorder" is a condition that is associated with the immune system of a subject, either through activation or suppression of the immune system, or that can be treated, prevented or diagnosed by targeting a certain component of the immune response in a subject, such as the adaptive or innate immune response. Such disorder may be any one of an inflammatory disease or an autoimmune disease.

According to one specific embodiment, the method of the invention may be specifically suitable for treating an inflammatory disease or an inflammatory-associated condition. The terms "inflammatory disease" or "inflammatory-associated condition" refers to any disease or pathologically condition which can benefit from the reduction of at least one inflammatory parameter, for example, induction of an inflammatory cytokine such as IFN-gamma and IL-2. The condition may be caused (primarily) from inflammation, or inflammation may be one of the manifestations of the diseases caused by another physiological cause.

Examples of other immune-related disorders that may be treated by the methods, and kits of the invention include, but are not limited to, ulcerative colitis, Crohn's disease, irritable bowel disease (IBD), alopecia areata, lupus, anlcylosing spondylitis, Meniere's disease, antiphospholipid syndrome, mixed connective tissue disease, autoimmune Addison's disease, multiple sclerosis, autoimmune hemolytic anemia, myasthenia gravis, autoimmune hepatitis, pemphigus vulgaris, Behcet's disease, pernicious anemia, bullous pemphigoid, polyarthritis nodosa, cardiomyopathy, polychondritis, dermatitis herpetiformis, polyglandular syndromes, chronic fatigue syndrome (CFIDS), polymyalgia rheumatica, chronic inflammatory demyelinating, polymyositis and dermatomyositis, chronic inflammatory polyneuropathy, primary agammaglobulinemia, Churg-Strauss syndrome, primary biliary cirrhosis, cicatricial pemphigoid, psoriasis, CREST Syndrome, Raynaud's phenomenon, cold agglutinin disease, Reiter's syndrome, rheumatic fever, discoid lupus, rheumatoid arthritis, essential mixed cryoglobulinemia, sarcoidosis, fibromyalgia, scleroderma, Grave's disease, Sjogren's syndrome, Guillain Barre disease, Stiff person syndrome, Hashimoto's thyroiditis, Takayasu arteritis, idiopathic pulmonary fibrosis, temporal arteritis/giant cell arteritis, idiopathic thrombocytopenia purpura (ITP), IgA nephropathy, uveitis, vasculitis, lichen planus, and vitiligo. The metal-nocardamine complexes described herein can be administered to a subject to treat or prevent disorders associated with an abnormal or unwanted immune response associated the above diseases.

The invention further encompasses the use of the compositions of the invention for treating any condition related to the disorders described above. It is understood that the interchangeably used terms "associated" and "related", when referring to pathologies herein, mean diseases, disorders, conditions, or any pathologies which at least one of: share causalities, co-exist at a higher than coincidental frequency, or where at least one disease, disorder, condition or pathology causes a second disease, disorder, condition or pathology.

Still further, it should be appreciated that there are numerous potential clinical applications for these complexes of the invention, including, but not limited to, the treatment of amyotrophic lateral sclerosis, age-related macular degeneration, cataract, sepsis, wound healing, injury due to exposure to chemical agents, heat or cold, ischemia and reperfusion injury, stroke, ionizing irradiation damage, injury associated with thallasemia, and hemochromatosis and Wilson disease.

Still further, it should be noted that in certain embodiments, the method of the invention may be applicable for treating any disorder associated with elevated levels of IL-1α. Examples for such disorders include, but are not limited to any one of sepsis, rheumatoid arthritis, Crohn's disease, inflammatory bowel disease, myocardial infarction, coronary artery disease, atherosclerosis, inflammatory cardiomyopathy, cardiac hypertrophy, osteoarthritis, CAPS, Schnitzler syndrome, ankylosing spondylitis, systemic lupus erythematosus, familiar mediterranean fever, systemic-onset juvenile idiopathic arthritis, Muckle-Wells syndrome, gout, pseudogout, type 2 diabetes, psoriatic arthritis, psoriasis, antisynthetase syndrome, anterior cruciate knee ligament tear, relapsing polychondritis, hemochromatosis-related arthritis of the hands, chronic recurrent multifocal osteomyelitis, erosive osteoarthritis of the hand, heart failure, diabetes-induced cardiomyopathy, smoldering myeloma, giant cells arteritis, dry eye disease, metabolic syndrome, hidradenitis suppurativa, acne, Behcett's disease, Blau syndrome/granulomatous arthritis, mevalonate kinase deficiency, Majeed syndrome, Henoch-Schonlein purpura, idiopathic recurrent pericarditis, macrophage activation syndrome, Sweet's syndrome/neutrophilic dermatoses, neutrophilic panniculitis, Erdheim-Chester/histiocytoses, multicentric Castleman disease, Jessner-Kanof disease, Kawasaki disease, colitis in chronic granulomatous disease, PAPA syndrome, SAPHO syndrome, hepatocellular carcinoma, pancreatic ductal adenocarcinoma, fibrosarcoma, type 1 diabetes, stroke, acute brain injury, Alzheimer's disease, colorectal cancer, breast cancer, melanoma, glioma, prostate, cervix, lung, and bladder cancer, Paget's disease, tumor necrosis factor-associated periodic syndrome.

In other embodiments, the method of the invention may be applicable for treating any disorder associated with elevated levels of TNF-α. Non-limiting examples include septic shock, inflammatory bowel disease, ulcerative colitis, juvenile idiopathic arthritis, Takayasu arteritis, pathological corneal hemangiogenesis and lymphangiogenesis, sarcoidosis, amyloidosis, post-operative cognitive dysfunction, ovarian cancer, stroke, rheumatoid arthritis, ankylosing spondylitis, Crohn's disease, inflammatory bowel disease, psoriasis, psoriatic arthritis, pediatric Crohn's disease, pediatric ulcerative colitis, colorectal cancer, idiopathic pulmonary fibrosis, cystic fibrosis, retinitis pigmentosa, pancreatic cancer, Paget's disease, tumor necrosis factor-associated periodic syndrome.

Still further, it should be noted that in certain embodiments, the method of the invention may be applicable for treating any disorder associated with elevated levels of IL-6. Such disorders include rheumatoid arthritis, Crohn's disease, inflammatory bowel disease, ulcerative colitis, periodontitis, pneumonitis, idiopathic pulmonary fibrosis, depression, colorectal cancer, uveitis, Castleman's disease, systemic juvenile idiopathic arthritis, systemic lupus, systemic sclerosis, polymyositis, vasculitis syndrome, spondyloarthritis, relapsing polychondritis, acquired haemophilia A, autoimmune haemolytic anaemia, adult-onset Still's disease, amyloid A amyloidosis, polymyalgia rheumatica, remitting seronegative symmetrical synovitis with pitting oedema, Behçett's disease, *graft-versus-*host disease, tumour necrosis factor-associated periodic syndrome, pulmonary arterial hypertension, atopic dermatitis, sciatica, relapsing polychondritis, type 2 diabetes, obesity, Grave's ophthalmopathy, cardiovascular disease in rheumatic arthritis, giant cells arteritis, non-ST elevated myocardial infarction, schizophrenia, ovarian cancer, fibrous dysplasia of bone, primary Sjögren's syndrome, ankylosing spondylitis, Erdheim Chester disease, ANCA-associated vasculitis, neuromyelitis optica, chronic glomerulonephritis, Takayasu arteritis, prostate cancer, renal cell carcinoma, multiple myeloma, lymphoma, cytokines release syndrome, listeriosis, asthma, COPD, psoriasis, multiple sclerosis, osteoarthritis, sepsis, interstitial lung disease, intraocular inflammation, endometriosis, Alzheimer's disease, cerebral trauma, breast cancer, pancreatic cancer, AIDS-related pathologies, cutaneous and systemic plasmacytosis, gastric cancer, cervical cancer, melanoma, hepatocellular carcinoma, leukemia, glioblastoma, uteral carcinoma, cystic fibrosis, oral cancer, Paget's disease.

In certain embodiments, the method of the invention may be applicable for treating any disorder associated with elevated levels of IL-17. Examples for such disorders may include inflammatory bowel disease, Crohn's disease, ulcerative colitis, psoriasis, rheumatoid arthritis, psoriatic arthritis, ankylosing spondyloarthritis, multiple sclerosis, asthma, colorectal cancer, lung cancer, hepatocellular carcinoma, liver fibrosis, esophageal squamous cell carcinoma, glioblastoma, ovarian cancer, breast cancer, melanoma, gastric cancer, head and neck cancers, nasopharyngeal cancer, COPD, SAPHO syndrom, lung fibrosis, cardiac fibrosis, renal fibrosis, Paget's disease, osteosarcoma, gastric ulcer, bladder cancer, systemic juvenile idiopathic arthritis, systemic lupus erythematosus, airway neutrophilia, cystic fibrosis, allergic rhinitis, alopecia areata, atopic dermatitis, cutaneous T-cell lymphomas, mastocytosis.

As used herein the term *"method"* refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, *"disease", "disorder", "condition"* and the like, as they relate to a subject's health, are used interchangeably and have meanings ascribed to each and all of such terms.

The term *"treatment"* as used herein refers to the administering of a therapeutic amount of the composition of the present invention which is effective to ameliorate undesired symptoms associated with a disease, to prevent the manifestation of such symptoms before they occur, to slow down the progression of the disease, slow down the deterioration of symptoms, to enhance the onset of remission period, slow down the irreversible damage caused in the progressive chronic stage of the disease, to delay the onset of said progressive stage, to lessen the severity or cure the disease, to improve survival rate or more rapid recovery, or to prevent the disease from occurring or a combination of two or more of the above.

The *"effective amount"* for purposes disclosed herein is determined by such considerations as may be known in the art. The amount must be effective to achieve the desired therapeutic effect as described above, depending, *inter alia,* on the type and severity of the disease to be treated and the treatment regime. The effective amount is typically determined in appropriately designed clinical trials (dose range studies) and the person versed in the art will know how to properly conduct such trials in order to determine the effective amount. As generally known, an effective amount depends on a variety of factors including the distribution profile within the body, a variety of pharmacological parameters such as half life in the body, on undesired side effects, if any, on factors such as age and gender, etc.

More specifically, the compositions containing the metal-nocardamine complexes of the present invention, or any combination, mixture or cocktail thereof can be administered for prophylactic and/or therapeutic treatments. In therapeutic application, compositions are administered to a patient already affected by an inflammation or immune-related disorder or specifically, a disorder associated with at least one of elevated levels of pro-inflammatory cytokines and reduced levels of anti-inflammatory cytokines, in an amount sufficient to cure or at least partially arrest the condition and its complications. An amount adequate to accomplish this is defined as a "therapeutically effective dose." Amounts effective for this use will depend upon the severity of the condition and the general state of the patient's own immune system, but generally range from about 0.01 to about 10,000 mg/kg, specifically, about 0.01 to about 1000, 500, 250, 100, 90, 80, 70, 60, 50, 40, 30, 20, 10, 9, 8, 7, 6, 5, 4, 3, 2, 1, 0.1 mg/kg. It should be noted that in certain embodiments, the effective amount may be 1, 1.5, 2, 2.5, 3, 3.5, 4, 4.5, 5, 5.5, 6, 6.5, 7, 7.5, 8, 8.5, 9, 9.5, 10 mg/kg or more. In yet some further embodiments, the effective amount may be 2.5 mg/kg of the complexes of the invention or of any combinations thereof. Single or multiple administrations on a daily, weekly or monthly schedule can be carried out with dose levels and pattern being selected by the treating physician. More specific embodiments relate to the use of typically 2-3 doses per week.

The term *"prophylaxis"* refers to prevention or reduction the risk of occurrence of the biological or medical event, specifically, the occurrence or re occurrence of disorders associated with at least one of elevated levels of pro-inflammatory cytokine/s and reduced level/s of anti-inflammatory cytokine/s, that is sought to be prevented in a tissue, a system, an animal or a human being, by a researcher, veterinarian, medical doctor or other clinician, and the term "prophylactically effective amount" is intended to mean that amount of a pharmaceutical composition that will achieve this goal. Thus, in particular embodiments, the methods of the invention are particularly effective in the prophylaxis, i.e., prevention of conditions associated with infectious disease. Thus, subjects administered with said compositions are less likely to experience symptoms associated with said infectious condition that are also less likely to re-occur in a subject who has already experienced them in the past.

The term *"amelioration"* as referred to herein, relates to a decrease in the symptoms, and improvement in a subject's condition brought about by the compositions and methods according to the invention, wherein said improvement may be manifested in the forms of inhibition of pathologic processes associated with the immune-related disorders described herein, a significant reduction in their magnitude, or an improvement in a diseased subject physiological state.

The term *"inhibit"* and all variations of this term is intended to encompass the restriction or prohibition of the progress and exacerbation of pathologic symptoms or a pathologic process progress, said pathologic process symptoms or process are associated with.

The term *"eliminate"* relates to the substantial eradication or removal of the pathologic symptoms and possibly pathologic etiology, optionally, according to the methods of the invention described below.

The terms *"delay", "delaying the onset", "retard"* and all variations thereof are intended to encompass the slowing of the progress and/or exacerbation of a pathologic disorder or an infectious disease and their symptoms slowing their progress, further exacerbation or development, so as to appear later than in the absence of the treatment according to the invention.

More specifically, treatment or prevention include the prevention or postponement of development of the disease, prevention or postponement of development of symptoms and/or a reduction in the severity of such symptoms that will or are expected to develop. These further include ameliorating existing symptoms, preventing- additional symptoms and ameliorating or preventing the underlying metabolic causes of symptoms. It should be appreciated that the terms "inhibition", "moderation", "reduction" or "attenuation" as referred to herein, relate to the retardation, restraining or reduction of a process by any one of about 1% to 99.9%, specifically, about 1% to about 5%, about 5% to 10%, about 10% to 15%, about 15% to 20%, about 20% to 25%, about 25% to 30%, about 30% to 35%, about 35% to 40%, about 40% to 45%, about 45% to 50%, about 50% to 55%, about 55% to 60%, about 60% to 65%, about 65% to 70%, about 75% to 80%, about 80% to 85% about 85% to 90%, about 90% to 95%, about 95% to 99%, or about 99% to 99.9%.

The present invention relates to the treatment of subjects, or patients, in need thereof. By *"patient"* or *"subject in need"* it is meant any organism who may be infected by the above-mentioned pathogens, and to whom the preventive and prophylactic kit/s, system/s and methods herein described is desired, including humans, domestic and non-domestic mammals such as canine and feline subjects, bovine, simian, equine and murine subjects, rodents, domestic birds, aquaculture, fish and exotic aquarium fish. It should be appreciated that the treated subject may be also any reptile or zoo animal. More specifically, the kit/s and method/s of the invention are intended for preventing pathologic condition in mammals. By *"mammalian subject*" is meant any mammal for which the proposed therapy is desired, including human, equine, canine, and feline subjects, most specifically humans. It should be noted that specifically in cases of non-human subjects, the method of the invention may be performed using administration via injection, drinking water, feed, spraying, oral lavage and directly into the digestive tract of subjects in need thereof.

Another aspect of the present invention concerns a complex of nocardamine with at least one metal.

The complex may be of nocardamine with at least one metal wherein at least one metal is selected from the group consisting of lanthanides, actinides, post-transition metals or transition metals.

The disclosure relates to the complex of nocardamie with at least one metal, wherein the metal is selected from the group consisting of zinc, gallium, aluminum, silver, gold, cobalt, molybdenum, and vanadium.

According to the invention, the complex is of nocardamine with at least one metal, wherein the metal is selected from the group consisting of Zn²⁺ and Ga³⁺.

The complex is of nocardamine with Zn²⁺.

In yet some other embodiments, the complex of the invention may be nocardamine with Ga³⁺.

In some specific embodiment the invention relates to a composition of nocardamine with at least one metal, for use in a method of treating, preventing, inhibiting, reducing, eliminating, protecting or delaying the onset of a pathological condition or a disorder in a subject in need thereof.

The invention also relates as a further aspect, to combining separate pharmaceutical compositions in a kit form. In a disclosure, the kit includes at least two separate pharmaceutical compositions: (i) at least one of lanthanide/s, actinide/s, post-transition metal/s or transition metal/s, or any combinations thereof, in any form of salts, esters and amides thereof, and a pharmaceutically acceptable carrier or diluent, and (ii) nocardamine.

The metal contained in the kit of the invention may be at least one of zinc, gallium, aluminum, silver, gold, molybdenum and vanadium.

The kit of the invention may comprise zinc. In yet some further disclosure, the kits of the invention may comprise gallium. Still further, in some embodiments, the kits of the invention may comprise zinc and gallium.

More specifically, the kit includes container means for containing both separate compositions, such as a divided bottle or a divided foil packet. However, the separate compositions may also be contained within a single, undivided container. Typically the kit includes directions for the administration of the separate components. The kit form is particularly advantageous when the separate components are preferably administered in different dosage forms (e.g., parenteral vs. topical), are administered at different dosage intervals, or when titration of the individual components of the combination is desired by the prescribing physician.

The kit of the invention may further comprise an acid in a separated container. In some embodiments, the acid is selected from the list consisting of: 1-hydroxy-2-naphthoic acid, 2,2-dichloroacetic acid, 2-oxoglutaric acid, 4-acetamidobenzoic acid, 4-aminosalicylic acid, acetic acid, alkanesulphonic acid, alkenesulphonic acid, alkynesulphonic acid with any substitutions, adipic acid, ascorbic acid, aspartic acid, benzoic acid, camphoric acid, camphor-10-sulfonic acid, decanoic acid, hexanoic acid, octanoic acid, carbonic acid, cinnamic acid, citric acid, cyclamic acid, dodecylsulfuric acid, formic acid, fumaric acid, galactaric acid, gentisic acid, glucoheptonic acid, gluconic acid, glucuronic acid, glutamic acid, glutaric acid, glycerophosphoric acid, glycolic acid, hippuric acid, hydrobromic acid, hydrochloric acid, isobutyric acid, lactic acid, lactobionic acid, lauric acid, maleic acid, malic acid, malonic acid, mandelic acid, methanesulfonic acid or its derivates, or a substituted hydrocarbyl sulphonic acid, for example a hydroxy-, alkoxy-, acyloxy-, alkoxycarbonyl-, halogen-, aromatic- or amino-substituted alkylsulphonic acid, nicotinic acid, nitric acid, oleic acid, oxalic acid, palmitic acid, pamoic acid, phosphoric acid, propionic acid, pyroglutamic acid, salicylic acid, sebacic acid, seleninic acid, selenonic acid, or any seleninic or selenonic analogue of the previously mentioned sulfonic compounds, stearic acid, succinic acid, sulfuric acid, tartaric acid, thiocyanic acid, undecylenic acid.

In some embodiments the kit of the invention will enable the use of the active ingredients in a method of treating, preventing, inhibiting, reducing, eliminating, protecting or delaying the onset of a pathological condition or a disorder in a subject in need thereof.

In yet some other particular and non-limiting embodiments, the invention further provides therapeutic methods for reducing the levels of IL-1α, TNF-α, IL-6 and IL-17 in a subject suffering from a disorder accompanied by elevated IL-1α, TNF-α, IL-6 and IL-17, thereof comprising of the step of administering a therapeutically effective amount of the kit of the invention, optionally in combination with at least one therapeutic compound, to a subject suffering from a pathological disorder or disease as outlined above. It should be further noted that the application of the kit of the invention or any component thereof, may form a complementary treatment regimen for subjects suffering from a pathological disorder or disease as outlined above.

Still further, the kits and the complexes of the invention and any components thereof may be applied as a single daily dose or multiple daily doses, preferably, every 1 to 7 days. It is specifically contemplated that such application may be carried out once, twice, thrice, four times, five times or six times daily, or may be performed once daily, once every 2 days, once every 3 days, once every 4 days, once every 5 days, once every 6 days, once every week, two weeks, three weeks, four weeks or even a month. The application of the kits of the invention or of any component thereof may last up to a day, two days, three days, four days, five days, six days, a week, two weeks, three weeks, four weeks, a month, two months three months or even more. Specifically, application may last from one day to one month. Most specifically, application may last from one day to 7 days.

The present disclosure further concerns a method for the production of a complex of nocardamine with either cobalt, or molybdenum, or vanadium, including the form of vanadyl, or zinc, or aluminum, or silver or gold. The method comprising: mixing nocardamine (Desferrioxamine E) with cobalt, or molybdenum, vanadium, zinc, silver or gold when contained at a molar ratio of metal to nocardamine between 1 :0.01 to 1 : 100, typically, 1 : 1, under the conditions, described below.

By one option the mixing is in the presence of an acid, for example from the following list consisting of non limiting examples of I-hydroxy-2-naphthoic acid, 2,2- dichloroacetic acid, 2-oxoglutaric acid, 4-acetamidobenzoic acid, 4-aminosalicylic acid, acetic acid, alkanesulphonic acid, alkenesulphonic acid, alkynesulphonic acid with any substitutions, adipic acid, ascorbic acid, aspartic acid, benzoic acid, camphoric acid, camphor- 10-sulfonic acid, decanoic acid, hexanoic acid, octanoic acid, carbonic acid, cinnamic acid, citric acid, cyclamic acid, dodecylsulfuric acid, formic acid, fumaric acid, galactaric acid, gentisic acid, glucoheptonic acid, gluconic acid, glucuronic acid, glutamic acid, glutaric acid, glycerophosphoric acid, glycolic acid, hippuric acid, hydrobromic acid, hydrochloric acid, isobutyric acid, lactic acid, lactobionic acid, lauric acid, maleic acid, malic acid, malonic acid, mandelic acid, methanesulfonic acid or its derivates, or a substituted hydrocarbyl sulphonic acid, for example a hydroxy-, alkoxy-, acyloxy-, alkoxycarbonyl-, halogen-, aromatic- or amino- substituted alkylsulphonic acid, nicotinic acid, nitric acid, oleic acid, oxalic acid, palmitic acid, pamoic acid, phosphoric acid, propionic acid, pyroglutamic acid, salicylic acid, sebacic acid, seleninic acid, selenonic acid, or any seleninic or selenonic analogue of the previously mentioned sulfonic compounds, stearic acid, succinic acid, sulfuric acid, tartaric acid, thiocyanic acid, undecylenic acid, at a molar ratio of acid to nocar-damme between 1 :0.01 to 1 : 100, typically 1 : 1.

Another aspect of the disclosure encompasses a process of preparation of the complex of nocardamine with a metal ion, the process comprising: contacting nocardamine with a metal ion, selected from the group, consisting of cobalt, or molybdenum, or vanadium, including the form of vanadyl, or zinc, aluminum, silver or gold when contained at a molar ratio of metal to nocardamine, between 1:0.01 to 1:100.

In some selective embodiments, the stoichiometric ratio of metal to nocardamine is 1:1.

In some embodiments the process for the production of complexes of nocardamine with a metal ion comprises the following steps: contacting nocardamine (Desferoxamine E) with a metal ion, selected from the group, consisting of cobalt, molybdenum, vanadium, including the form of vanadyl, zinc, aluminum, silver or gold wherein either nocardamine and/or the metal ion is in the form of a salt, when contained at a molar ratio of metal to nocardamine, between 1 :0.01 to 1 :100 in the presence of an acid, wherein the acid is selected from the group consisting of : I-hydroxy-2-naphthoic acid, 2,2-dichloroacetic acid, 2-oxoglutaric acid, 4-acetamidobenzoic acid, 4- aminosalicylic acid, acetic acid, alkanesulphonic acid, alkenesulphonic acid, alkynesulphonic acid with any substitutions, adipic acid, ascorbic acid, aspartic acid, benzoic acid, camphoric acid, camphor- 10-sulfonic acid, decanoic acid, hexanoic acid, octanoic acid, carbonic acid, cinnamic acid, citric acid, cyclamic acid, dodecylsulfuric acid, formic acid, fumaric acid, galactaric acid, gentisic acid, glucoheptonic acid, gluconic acid, glucuronic acid, glutamic acid, glutaric acid, glycerophosphoric acid, glycolic acid, hippuric acid, hydrobromic acid, hydrochloric acid, isobutyric acid, lactic acid, lactobionic acid, lauric acid, maleic acid, malic acid, malonic acid, mandelic acid, methanesulfonic acid or its derivates, or a substituted hydrocarbyl sulphonic acid, for example a hydroxy-, alkoxy-, acyloxy-, alkoxycarbonyl-, halogen-, aromatic- or amino- substituted alkylsulphonic acid, nicotinic acid, nitric acid, oleic acid, oxalic acid, palmitic acid, pamoic acid, phosphoric acid, propionic acid, pyroglutamic acid, salicylic acid, sebacic acid, seleninic acid, selenonic acid, or any seleninic or selenonic analogue of the previously mentioned sulfonic compounds, stearic acid, succinic acid, sulfuric acid, tartaric acid, thiocyanic acid, undecylenic acid, at a molar ratio of acid to nocardamine between 1:0.01 to 1:100.

Other purposes and advantages of the invention will become apparent as the description proceeds.

The present invention as defined by the claims, the contents of which are to be read as included within the disclosure of the specification, and will now be described by way of example with reference to the accompanying Figures.

All scientific and technical terms used herein have meanings commonly used in the art unless otherwise specified. The definitions provided herein are to facilitate understanding of certain terms used frequently herein and are not meant to limit the scope of the present disclosure.

As used herein the term "about" refers to ± 10 %. The terms "comprises", "comprising", "includes", "including", "having" and their conjugates mean "including but not limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The term "about" as used herein indicates values that may deviate up to 1%, more specifically 5%, more specifically 10%, more specifically 15%, and in some cases up to 20% higher or lower than the value referred to, the deviation range including integer values, and, if applicable, non-integer values as well, constituting a continuous range. As used herein the term "about" refers to ± 10 %.

Throughout this specification and the Examples and claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

It should be noted that various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible sub ranges as well as individual numerical values within that range. For example, description of a range such as from 1 to 6 should be considered to have specifically disclosed sub ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub combination or as suitable in any other described embodiment of the invention. Certain features described in the context of various embodiments are not to be considered essential features of those embodiments, unless the embodiment is inoperative without those elements.

Various embodiments and aspects of the present invention as delineated herein above and as claimed in the claims section below find experimental support in the following examples.

Disclosed and described, it is to be understood that this invention is not limited to the particular examples, methods steps, and compositions disclosed herein as such methods steps and compositions may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

The following examples are representative of techniques employed by the inventors in carrying out aspects of the present invention. It should be appreciated that while these techniques are exemplary of preferred embodiments for the practice of the invention, those of skill in the art, in light of the present disclosure, will recognize that numerous modifications can be made without departing from the spirit and intended scope of the invention.

### EXAMPLES

Without further elaboration, it is believed that one skilled in the art can, using the preceding description, utilize the present invention to its fullest extent. The following preferred specific embodiments are, therefore, to be construed as merely illustrative, and not limitative of the claimed invention in any way.

Standard molecular biology protocols known in the art not specifically described herein are generally followed essentially as in Sambrook et al., Molecular cloning: A laboratory manual, Cold Springs Harbor Laboratory, New-York (1989,1992), and in Ausubel et al., Current Protocols in Molecular Biology, John Wiley and Sons, Baltimore, Maryland (1988).

Standard organic synthesis protocols known in the art not specifically described herein are generally followed essentially as in Organic syntheses : Vol. 1-79, editors vary, J. Wiley, New York, (1941-2003); Gewert et al., Organic synthesis workbook, Wiley-VCH, Weinheim (2000); Smith & March, Advanced Organic Chemistry, Wiley-Interscience ; 5th edition (2001).

Standard medicinal chemistry methods known in the art not specifically described herein are generally followed essentially as in the series "Comprehensive Medicinal Chemistry", by various authors and editors, published by Pergamon Press.

Without further elaboration, it is believed that one skilled in the art can, using the preceding description, utilize the present invention to its fullest extent. The following preferred specific embodiments are, therefore, to be construed as merely illustrative, and not limitative of the claimed invention in any way.

Standard molecular biology protocols known in the art not specifically described herein are generally followed essentially as in Vanderkerken K The 5T2MM murine model of multiple myeloma: maintenance and analysis [Methods Mol. Med. 113:191-205 (2005); Epstein J. The SCID-hu myeloma model. Methods Mol. Med. 113:183-90 (2005)].

### Animals

* For the Irritant contact dermatitis (ICD) model Balb\c female mice at 12 weeks of age were used. Irritant contact dermatitis was induced by epicutaneous application of 15 µl of 1% croton oil solution in acetone and olive oil (4:1), to the mouse right ear.
* For the Adjuvant-Induced Arthritis model, six to eight week old female inbred Lewis rats (Harlan Laboratories, Israel) are injected intradermally at the base of the tail with 1 mg of Mycobacterium Tuberculosis (MT) H37Ra (Difco, Detroit, MI) in CFA (Difco)
* For the Collagen-Induced Arthritis model, Nine week old male DBA/1 mice (Harlan Laboratories, Israel) are used. Mice are injected s.c. at the base of the tail with 200 µg collagen type II emulsified in CFA (Difco). Three weeks later mice are boosted s.c. with the same concentration of collagen II.
* For the TNBS colitis model, Balb/C mice are sensitized with 160 µL of the haptenizing agent TNBS (Sigma-Aldrich, St. Louis, MO) at a concentration of 2.5% in 50% ethanol by skin painting.
* Sprague-Dawley (SD) male rats, purchased from Harlan-Israel facilities, treated with streptozotocin (70 mg/kg) for use as diabetes type I model.
* 8-week-old female C57BL/6 mice by injecting s.c. into the left para-lumbar region 125 µg of myelin oligodendrocyte glycoprotein 35-55 peptide (MOG35-55) emulsified in complete Freund's adjuvant (CFA) containing 5 mg/ml heat-killed *Mycobacterium tuberculosis* for use as an MS model.

### Experimental procedures

### Nocardamine isolation

The applied method, described by Meiwes et al. (18) was modified by the inventors. S. *olivaceus* was seeded on HA medium contained per liter of tap water 4 g glucose, 10 g malt extract, 4 g yeast extract, 20 g agar at pH 7.3, and incubated at 27°C for 10 days. Pre-culture medium containing per liter of deionized water: 20 g soybean meal and 20 g mannitol at pH 7.5 was inoculated with spores of S. *olivaceus* and incubated for 48 hours on a rotary shaker (120 rpm) at 27°C. These cultures were used for inoculation of production medium. The basic production medium contained per liter of deionized water: 30 g sucrose, 3 g (NH₄)₂SO₄, 0.5 g NaCl, 0.2 g MgSO₄*7H₂O, 0.1 g CaCl₂-2H₂0, 2 mg ZnSO₄-7H₂0, 1 g KH₂PO₄. 100 mM N-tris(hydroxymethyl)methyl-2-aminoethanesulphonic acid (TES), 5 g lysine, and the pH was adjusted to 7.5 with 3 N NaOH. The concentration of iron in each one of the media was reduced to nanomolar level using Chelex 100 beads and conalbumin. Flasks with production medium were incubated for 48 h under the same conditions as the pre-cultures. The medium was filtered and the liquid phase was lyophilized. Extraction of the aqueous phase with 1-buthanol was performed. The buthanolic extract was evaporated, and the residue was triturated with ether, which was removed. Then the residue was dissolved in hot methanol, and activated carbon was added. After 48 h of incubation at -20°C a precipitate was formed, which was further filtered, and dried. The obtained dry precipitate was purified by column chromatography on silica gel (0.040-0.063 mm), using mixture of methanol with dichloromethane as mobile phase. The eluate was dried out using rotary evaporator, and the purity of yielded nocardamine was tested by spectrophotometric titration (λ=435 nm). Nocardamine was authenticated by mass spectrometry, which was identical to nocardamine bought from Alexis Biochemicals (San Diego, CA, USA).

The metal-nocardamine complexes other than zinc-nocardamine complex and gallium-nocardamine complex, provided in the examples below, fall outside the scope of the claims and do not form part of the invention.

### Zinc-nocardamine complex preparation - method I

To obtain zinc-nocardamine complex, for structural/chemical studies, equimolar aqueous solutions of zinc chloride and nocardamine were prepared. To form the complex, equal volumes of the solutions were mixed, then cooled to 4°C, titrated to pH 2.96 using formic acid 98%, and then the pH was brought to 7.06, initially by NH₄OH, 25% and later with NH₄OH, 5% solutions. The mixture was incubated for 30 min at 45°C and cooled to room temperature. The solution was lyophilized and its purity was measured spectrophotometrically (at λ=435 nm). Assuming that the binding of nocardamine to zinc is complete, the formation of the zinc-nocardamine complex was verified by mass-spectrometry.

### Zinc-nocardamine complex preparation - method II

To obtain zinc-nocardamine complex, for pharmaceutical use, equimolar aqueous solutions of zinc chloride and nocardamine were prepared. To form the complex, equal volumes of the solutions were mixed at room temperature. To facilitate the complex formation the pH of the mixture was brought to 3 using IN HCl and then to 7, with NaOH solution. The mixture was incubated for 30 min at 45°C and cooled to room temperature. The solution was lyophilized and its purity was measured spectrophotometrically following the reaction with ferric iron (at λ=435 nm), and assuming that a formation of the zinc-nocardamine complex is complete.

### Zinc-nocardamine ointment preparation

To prepare 3 g of Vaseline-based ointment containing 0.5% zinc-nocardamine complex, 15 mg of pure nocardamine were used; for that (16.7 mg of 89.8% pure nocardamine were weighed). The nocardamine powder was dissolved in 465 µl DDW, in a glass tube. Seventeen µl of 192 mg/ml Zn²⁺ (as ZnCl₂ aqueous solution) were added, and the tube was vortexed and sonicated again. The pH was lowered to 3 using hydrochloric acid and then brought to 7 with NaOH solution. Then, 135 µl of Tween 80 were added. The tube was vortexed for 2 min and sonicated. In a separate glass tube (tube B) 2.4 g Vaseline was melted over a water bath pre-heated to 65°C, and tube A containing the zinc-nocardamine complex aqueous solution was poured into tube B. Tube B was stirred for 15 sec at 65°C, and put on ice. An amount of 3 g unguent was obtained.

### Gallium-nocardamine complex preparation - method I

To obtain gallium-nocardamine complex, for structural/chemical studies, equimolar aqueous solutions of gallium chloride (99.999% pure) and nocardamine (89.8 % pure) were prepared. Equal volumes of the solutions were mixed, then cooled to 10°C, titrated to pH 3.2 using formic acid (98%), and then the pH was brought to 7.1, initially by NH₄OH, 25% and later with NH₄OH, 5% solutions. The mixture was incubated for 30 min at 45°C and cooled to room temperature. The solution was lyophilized and its purity was measured spectrophotometrically (at λ=435 nm). The formation of the gallium-nocardamine complex was verified by mass-spectrometry.

### Gallium-nocardamine complex preparation - method II

To obtain gallium-nocardamine complex, for pharmaceutical use, equimolar aqueous solutions of gallium chloride and nocardamine were prepared. Equal volumes of the solutions were mixed at room temperature. The pH of the mixture was brought to 3.2 using IN HCl and then to 7, with NaOH solution. The mixture was incubated for 30 min at 45°C and cooled to room temperature. The solution was lyophilized and its purity was measured spectrophotometrically, following the reaction with ferric iron (at λ=435 nm).

### Silver-nocardamine complex preparation

Silver-nocardamine complex was prepared as follows: 1 mmole (203 mg) of silver methanesulfonate was dissolved in 100 ml doubly distilled water (DDW). An equivalent amount of nocardamine (1 mmole - 600 mg) was dissolved in 80 ml DDW. The solutions were mixed, and the combined solution was kept in the dark under continuous stirring at 50°C for 10 min. Then the pH was adjusted to 6.8-7.4. The mixture was cooled down to room temperature and lyophilized, yielding grey-white powder.

### Gold-nocardamine complex preparation - method I

Gold-nocardamine complex was prepared as follows: 20 µmole of gold chloride (as [HAuCl₄·4H₂O] MW = 412, gold content 48%) (8.2 mg) was dissolved in 5ml DDW. An equivalent amount of nocardamine (20 µmole - 12 mg) was dissolved in 2 ml DDW. The two solutions were mixed, the pH was brought to 3, and the combined solution was kept in the dark under continuous stirring at 50°C for 10 min. Then the pH was adjusted to 6.8-7.4. The mixture was lyophilized, yielding yellow powder.

### Gold-nocardamine complex preparation - method II

Gold-nocardamine mesylate complex was prepared as follows: 20 µmole of AuCl₃ (6.0 mg) and 20 µmole of sodium methanesulfonate (2.2 mg) were dissolved in 5 ml DDW. Equivalent amount of nocardamine (20 µmole = 12.0 mg) was dissolved in 1.6 ml DDW.

The solutions were mixed together, the pH was adjusted to 6.8-7.4, in the dark, under continuous stirring at 50°C for 10 minutes and cooled down to room temperature. The mixture was lyophilized, yielding yellow powder.

### Gold-nocardamine complex preparation - method III

Gold-nocardamine mesylate complex was prepared as follows: Solutions of 20 µmole of AuCl₃ (6.0 mg) in 3 ml DDW, and of equivalent amount of nocardamine (20 µmole = 12.0 mg) in 1.6 ml DDW were mixed. Twenty µmole of sodium methanesulfonate (2.2 mg) was dissolved in 400 µl DDW and was combined with the gold chloride and nocardamine already mixed solution. The pH was adjusted to 5 and maintained at 5 for 20 min; then the pH was adjusted to 6.8 and kept in the dark under continuous stirring at 50°C for 10 min. Then it was cooled down to room temperature and lyophilized, yielding yellow powder.

### Mass-spectrometric analysis

The mass-spectrometric analysis was performed on Thermo Scientific TSQ MS apparatus (MA, USA) by direct injection (Electrospray positive ionization; vaporizer temperature 200°C; capillary temperature 100°C; flow: 0.005 ml/min (100% water); injection volume: 5 µl).

### Anti-bacterial effect

The anti-bacterial effect of zinc-nocardamine was assessed on the following types of pathogenic bacteria: *E.coli* (Gram-negative), *P. aeruginosa* (Gram-negative) and S. *aureus* (Gram-positive) (isolated in Hadassah-Ein Karem Hospital). More specifically, the experiment was performed as following: Each bacterial strain was grown overnight at 37°C, under aeration of 200 rpm, in the appropriate medium (EPEC (*E. coli)*) and *P. aeruginosa* in LB broth, *S. aureus* in BHI broth. Both media were purchased from BD (NJ, USA). Series of three-fold consecutive dilutions were performed in a 96-well plate, and bacteria were then added to the wells at a final OD₆₀₀ of 0.01. The experiments with all three strains were performed in duplicate. Zinc-nocardamine 10 mg/ml aqueous solution was prepared in advance; prior to the experiment it was diluted in the appropriate media in a ratio of 1:2, to obtain a stock concentration of 5 mg/ml. This stock was further diluted to 1.7, 0.5, 0.18, 0.06, 0.02, 0.002, 0.0007, and 0.0002 mg/ml and added to appropriate wells. Then the plate was incubated in a plate reader for 24 h, and the kinetics of OD measurements were recorded every 20 minutes by Eon plate reader (BioTek, VE,, USA). Bacterial growth curves were analyzed for each strain - the data presented demonstrates mean values of the duplicates, corresponding to dilutions yielding the following concentrations of zinc-nocardamine (in mg/ml) 0, 5, 1.7 and 0.5, where an effect on growth was observed. Bacterial viability experiments were performed on Petri dishes with appropriate medium.

### Measuring cytokine levels

The samples were homogenized, and protein concentration was measured by Bradford procedure. The concentration of cytokines was determined by ELISA method, using commercially available kits according to the Manufacturer's protocols (RayBiotech, GA, USA).

### Irritant contact dermatitis (ICD) model

Croton oil-induced irritant contact dermatitis (ICD) model is a well established and broadly used model for skin inflammation (19). Balb\c female mice at 12 weeks of age were used. ICD was induced by epicutaneous application of 15 µl of 1% croton oil solution in acetone and olive oil (4:1), to the right ear, if not mentioned otherwise. Ear thickness was measured using an engineer's micrometer, immediately before, and at 3, 6, 8, and 10 hours after the application of the croton oil. Ear swelling was measured and expressed by subtracting pre-exposure thickness value from the post-exposure thickness values.

### Induction and clinical assessment of Adjuvant-Induced Arthritis

Six to eight week old female inbred Lewis rats are injected intradermally at the base of the tail with 1 mg of Mycobacterium tuberculosis (MT) H37Ra (Difco, Detroit, MI) in CFA (Difco). Severity of arthritis (arthritis index) is assessed every other day by a blinded observer as follows: 0, no arthritis; 1, redness of the joint; 2, redness and swelling of the joint. The ankle and tarsal-metatarsal joints of each paw are scored. A maximum score of 16 can be obtained.

### Histopathology assessment in Adjuvant-Induced Arthritis

Rats are euthanized and hind legs are removed and fixed in formalin. Joints are stained with hematoxylin and eosin (H&E) and evaluated by a specialized veterinary pathologist

### Induction and clinical assessment of Collagen-Induced Arthritis

Nine week old male DBA/1 mice are injected s.c. at the base of the tail with 200 µg collagen type II emulsified in CFA (Difco). Three weeks later mice are boosted s.c. with the same concentration of collagen II. The severity of arthritis is assessed daily by measuring feet diameter of hind and fore paws by caliper by a blinded observer. As controls 4 mice of the same age that have not been injected with collagen are used. The mean of the healthy mice served as a "cut-off" measurement. Starting from day 5 after the boost injection, every mouse with a score value above the average of the healthy mice (in one paw or more) is assigned to one of the treatment groups.

### Induction and clinical assessment of Hapten-Mediated (TNBS) Colitis

Balb/C mice are sensitized with 160µL of the haptenizing agent TNBS at a concentration of 2.5% in 50% ethanol by skin painting. A week later, 120 µl of 1% TNBS in 50% ethanol is administered intrarectally via a 3.5-French catheter. Mice are sacrificed 3 days after the intrarectal TNBS administration. Animals are weighed at the time of sensitization, intrarectal administration and every day following such until sacrifice. Clinical assessment is carried out by evaluating weight loss and histopathology of the colon tissue post mortem.

### Histopathology assessment in TNBS colitis

Tissues are fixed in phosphate buffered saline containing 4% formalin and embedded in paraffin. Sections (5 µm) are stained with hematoxylin and eosin. The degree of inflammation is assessed by a blinded pathologist using the following scoring system from 0-4: 0 - no signs of inflammation; 1 - low level of leucocyte infiltration; 2 - moderate level of leucocyte infiltration; 3 - high level of leucocyte infiltration, high vascular density, thickening of bowel wall; 4 - transmural infiltrations, loss of goblet cells, high vascular density, strong bowel wall thickening, edema.

### Experimental Autoimmune Encephalomyelitis (EAE) model for MS

EAE is induced in 8-week-old female C57BL/6 mice by injecting, s.c. into the left para-lumbar region, 125 µg of myelin oligodendrocyte glycoprotein 35-55 peptide (MOG35-55) emulsified in complete Freund's adjuvant (CFA) containing 5 mg/ml heat-killed *Mycobacterium tuberculosis.* Immediately thereafter, and, again, at 48 h, the mice are inoculated i.p. with 0.5 ml of pertussis toxin (400 ng). 7 days later the mice are further challenged with an additional injection of MOG35-55 peptide in CFA injected into the right para-lumbar region. Mice are treated with the nocardamine-metal complex of the invention or the indicated appropriate controls.

EAE clinical score is evaluated as follows:
0 - without clinical disease;
1- tail weakness;
2 - hind limb weakness sufficient to impair righting;
3 - one limb plagic;
4 - paraplegia with forelimb weakness;
5 - quadriplegia;
6- death.

### Example 1

### Comparative mass-spectrometric (MS) analysis of nocardamine, zinc-nocardamine and gallium-nocardamine complexes

Zinc-nocardamine complex prepared as described in the experimental procedures was studied using MS. The family of peaks around m/z = 663-667 reflect the complex of nocardamine with the naturally abundant isotopes of zinc, demonstrating the existence of this complex (Figure IB). The family of peaks around m/z = 726.9 represent the Zn₂-nocardamine, formed within the MS instrument during the experiment (Figure IB). Peak of 623.21 represents the sodium-nocardamine species, formed within the MS instrument during the experiment. The peak of 601.16 represents the singly protonated form of nocardamine alone (Figure 1A).

Gallium-nocardamine complex was prepared as described in the experimental procedures, and was studied by MS. The peaks around m/z = 667 reflect the complex of nocardamine with the 2 naturally abundant gallium isotopes, demonstrating the existence of this complex (Figure 1C). Evidently, all the nocardamine molecules are bound, mostly to gallium.

### Example 2

### Anti-inflammatory effect of the zinc-nocardamine complex on skin inflammation using the irritant contact dermatitis (ICD) model

The therapeutic activity of zinc-nocardamine complex, as an anti-inflammatory agent, was tested in the murine ICD model.

In order to confirm that the therapeutic effect of the ointment stems from the zinc-nocardamine complex, and not from the Vaseline-based vehicle, a proper preliminary experiment was performed. Sixteen mice were divided into 2 groups, eight mice per group. The two groups of mice were exposed to the irritant - croton oil. At 3 hours after the exposure the first group was treated with ointment containing the vehicle (Vaseline) only, while the second group was not treated at all. No difference was observed between these groups, demonstrating that the vehicle does not provide beneficial therapeutic effect (Figure 2A).

In order to test whether the therapeutic effect stems from the zinc-nocardamine complex, the inventors prepared an ointment containing zinc-nocardamine complex (0.5%; w/w) as outlined in experimental procedures. This complex was applied with fingertip to the right ear of a mouse at 3 hours after the exposure to croton oil, and the anti-inflammatory effect of this treatment was compared among the following experimental groups: Group 1 - exposed to croton oil but not treated - control (n=8); Group 2 - exposed and treated with ointment containing nocardamine (0.5%: w/w); Group 3 - exposed and treated with ointment containing zinc-nocardamine (0.5%; w/w); and Group 4 - exposed and treated with a commercial betamethasone valerate ointment 0.1%. The animals were exposed to the irritant at t = 0, and treated by applying a fingertip of the respective ointment on the exposed ear at t = 3 h after the exposure to the irritant; the examination was conducted at t = 6 h after the exposure.

As shown in Figure 2B, at 3 hours after the exposure to the irritant, the exposed ears in both groups were swollen to the same extent, as expected. Single treatment of the exposed ear with zinc-nocardamine ointment demonstrated high efficacy against ICD similar to that observed with the commercial betamethasone ointment (Figure 2B). The treatment with the zinc-nocardamine ointment caused a rapid and marked reduction of the edema. Furthermore, this experiment has shown a substantial difference between the therapeutic efficacy of zinc-nocardamine complex as compared to that of nocardamine alone. While the animals treated with nocardamine alone recovered better than the untreated ones, their recovery was much slower than the recovery observed in mice that received zinc-nocardamine complex (Figure 2B).

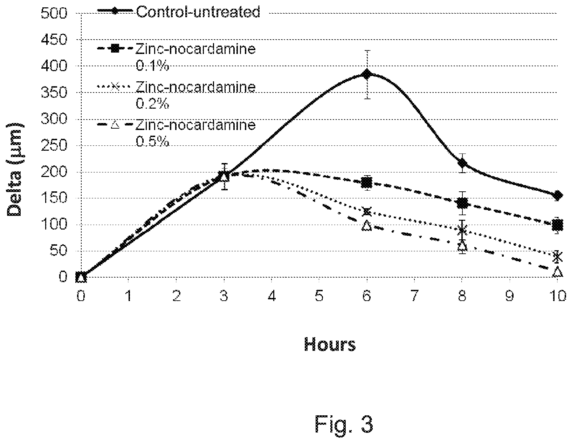

In order to establish the optimal concentration of zinc-nocardamine in Vaseline-based ointment, increasing concentrations of zinc-nocardamine were applied on the right ear of mice at 3 h following the exposure to the irritant. The results of this dose-response experiment show that the optimal concentration of zinc-nocardamine complex in Vaseline-based ointment is 0.5% (Figure 3). Therefore, this concentration was employed in upcoming experiments.

### Example 3

### Anti-inflammatory effect of zinc-nocardamine complex on the ICD at late stage of the inflammation

The inventors next tested whether a treatment of experimental ICD at a later stage of inflammatory process (at 6 h after the exposure) will still be beneficial (Figure 4). In addition, the preventive effect of zinc-nocardamine complex was studied by applying the complex containing the ointment at 1 h before the exposure to the croton oil (Figure 4). The results of this experiment revealed that treatment with zinc-nocardamine ointment at 1 h prior to the exposure to the irritant nearly abolishes the inflammatory injurious effect. In addition, it was shown that zinc-nocardamine ointment is effective even if applied at an advanced (late) stage of the inflammatory process (Figure 4). Also, a rapid decrease in the edema was observed when the treatment was applied at 6 h after the exposure to the irritant (Figure 4).

### Example 4

### Assessment of the systemic therapeutic effect of topically applied zinc-nocardamine complex using the ICD model

This experiment was conducted in order to compare a local effect of the topically applied zinc-nocardamine ointment with its assumable systemic effect this treatment provides. As shown in Figure 5, ear edema induced by ICD decreased significantly even when zinc-nocardamine ointment was applied to the contra-lateral ear. Likewise, reduced ear edema was observed when the ointment was applied to the mouse tail. The results of this experiment show that zinc-nocardamine complex exhibits a marked therapeutic efficacy against irritant contact dermatitis even if it is not applied directly on the site of the irritated skin (Figure 5). These findings demonstrate the high ability of the zinc-nocardamine ointment to pass through cellular membranes and to reach the blood flow, achieving therapeutically active concentrations.

### Example 5

### Effect of zinc-nocardamine on pro-inflammatory cytokines

In order to study the underlying mechanism of the zinc-nocardamine complex, its assumed anti-inflammatory activity was tested by measuring the concentrations of various pro-inflammatory cytokines, in the ears, along the experimental protocol, specifically, IL-1α, IL-6, IL-17 and TNF-α. The concentration of these cytokines were measured in ear tissue that had been exposed to croton oil (at t = 0) and was left untreated to t = 6 h. The concentration was compared to the concentrations at t = 6 h, in ears which were exposed at t = 0, and treated with the complex at t = 3 h.

The effect of ICD-induced inflammatory process on the concentration of IL-1α, IL-6, TNF-α and IL-17 within the ear tissue is shown in Figure 6A - 6D. More specifically, twenty two mice were divided into four groups as follows: Group 1 (n=6) - Control mice, unexposed to irritant and untreated; Group 2 (n=6): Exposed to croton oil, untreated, sacrificed at 6 h after the exposure, while the inflammation is expected to reach its peak; Group 3 (n=6): Ears were exposed to croton oil (t = 0) and treated topically (at t = 6 h) with a fingertip of ointment containing zinc-nocardamine (0.5%), ; Group 4 (n=4): Ears were exposed to croton oil, left untreated, and sacrificed at t = 3 h after the exposure, thus, assessing the dynamics of the inflammatory process. The concentration of the cytokines was determined by ELISA method, using commercially available kits, according to the Manufacturer protocol (RayBiotech, GA, USA)

The concentrations of IL-1α, IL-6, TNF-α at 3 h after the exposure to croton oil noticeably increased, by 2.8-fold for IL-1α and 1.8-fold for IL-6, 1.8-fold for TNF-α. However, the changes in the concentration of IL-17 at 3 h after the exposure to the irritant were not statistically significant (Figure 6D). Six hours after the exposure to the irritant further significant increases, in each cytokine, within the inflamed ear tissue, were observed. The magnitude of the increases was 7.9-fold for IL-1α, 3.2-fold for IL-6, 3.7-fold for TNF-α, and 2.1-fold for IL-17. Treatment with zinc-nocardamine (0.5%) truncated the inflammatory effect, and reduced the concentration of the cytokines to nearly the basal level.

Examining the pattern of change of the inflammatory cytokines in mice ears that had been exposed to the irritant, without consequent treatment, it can be seen that the degree of edema reaches its peak t = 6 h after the exposure. Three hours after the exposure the ears swell to an extent of half of their peak.

The decrease in the protein levels of the cytokines IL-1, IL-6 IL-17 and TNF-α following treatment provides an additional evidence for an anti-inflammatory effect of zinc-nocardamine complex, and thereby demonstrates the feasibility of using the metal-nocardamine complexes of the invention in conditions were the decrease in pro-inflammatory cytokines is desired.

### Example 6

### The effect of the zinc-nocardamine complex on anti-inflammatory cytokines

The assumed anti-inflammatory activity of the zinc-nocardamine complex was tested by measuring the concentrations of various anti-inflammatory cytokines, specifically, IL-4, IL-10 and IL-13, in ear tissue of mice. The concentrations of these cytokines were measured and their pattern of change along the experiment was compared for each: at baseline t = 0 (no exposure), at t = 3 h after exposure to croton oil, at t = 6 h after exposure to croton oil, and at 6 h after exposure to croton oil where these ears had been treated with the zinc-nocardamine complex at t = 3 h after the exposure to the croton oil. The levels of IL-13, along the experimental protocol, within the ear tissue, are shown in Figure 7. More specifically, twenty two mice were divided into four groups as follows: Group 1 (n=6) - Control mice (unexposed and untreated); Group 2 (n=6) - Ears were exposed to croton oil and not treated, sacrificed at 6 h after the exposure, while the inflammation was expected to reach its maximum; Group 3 (n=6): Ears were exposed to croton oil (at t = 0), topically treated with a fingertip of zinc-nocardamine (0.5% ointment) (at t = 3 h); Group 4 (n=4): Ears were exposed to croton oil (t = 0) not treated and sacrificed at t = 3 h after the exposure. The Figure shows the dynamics of the inflammatory process. The concentration of the cytokines was determined by ELISA method, using commercially available ELISA kits, according to the Manufacturer protocol (RayBiotech, GA, USA; Diaclone, Besançon, France).

The concentration of IL-13 measured at 3 h after the exposure to croton oil decreased 2.5-fold (Figure 7) when compared to the baseline level. Six hours after the exposure to the irritant this decrease was partly repaired, but remained 1.4-fold below the baseline level. Treatment with zinc-nocardamine (0.5% ointment), at t = 3 h, enhanced the anti-inflammatory effect, as indicated by restoring the IL-13 level back to its baseline level, at t = 6 h.

### Example 7

### Anti-bacterial properties of the zinc-nocardamine

Anti-bacterial properties of zinc-nocardamine are shown in Figure 8. The anti-bacterial effect of zinc-nocardamine was assessed on the following types of pathogenic bacteria: *E.coli* (Gram-negative), *P. aeruginosa* (Gram-negative) and *S. aureus* (Gram-positive) (isolated in Hadassah-Ein Karem Hospital). More specifically, the experiment was performed as following: Each bacterial strains was grown overnight as follows: EPEC (*E*. *coli*) and *P. aeruginosa* were grown in LB broth. *S. aureus* was grown in BHI broth. Zinc-nocardamine 10 mg/ml aqueous solution was prepared in advance and prior to the experiment was diluted in the appropriate media in a ratio of 1:2, to obtain a concentration of 5 mg/ml. Series of three-fold consecutive dilutions were performed in a 96-well plate and bacteria were then added to the wells at a final OD₆₀₀ of 0.01. All the experiments with all three strains were performed in duplicate. The plate was then incubated in a plate reader for 24 h, and the kinetics of OD measurements were recorded, every 20 minutes. Bacterial growth curves were analyzed for each strain - the data represented demonstrates mean values of the duplicates, corresponding to dilutions yielding 0, 5, 1.7 and 0.5 mg/ml of zinc-nocardamine, where growth effect was observed. Bacterial viability experiments were performed on Petri dishes with appropriate medium.

As shown in Figure 8, at 1.7 mg/ml of zinc-nocardamine complex a growth inhibition was observed with regard to either *E.coli, P. aeruginosa* (mild inhibition) *or S. aureus.* In addition, *S. aureus* growth inhibition was observed at 0.5 mg/ml of the complex.

Bactericidal activity (inhibition of bacterial viability) experiments were performed on Petri dishes with the appropriate medium and are shown in Figure 9. At 5mg/ml of zinc-nocardamine complex a bactericidal activity was obtained with regard to either *E.coli, P. aeruginosa* and *S*. *aureus.*

### Example 8

### The effect of the zinc-nocardamine complexes on the experimental model of Asthma

The experiment is performed using a murine model of ovalbumin (OVA)-induced asthma. Thirty two BALB/c female 8 weeks old mice are divided into 4 groups (n=8) as follows: (1) control; (2) asthmatic, sham-treated; (3) asthmatic, treated with metal-nocardamine complex intra-peritoneally (i/p) and intra-nasally (i/n); (4) asthmatic treated with metal-nocardamine complex, i/n only. The animals are sensitized to ovalbumin by intraperitoneal 100µl injection (10µg ovalbumin and 3 mg Al(OH)₃ in 0.9% saline) on days 0, 7, and 14 of the experiment. The mice are further exposed to intra-nasal instillations of OVA (50 µl) on days 17, 19, 20, and 23.

Mice of group 3 receive prophylactic treatment by 2 i/p injections (100µl each, containing 1 mg of metal-nocardamine complex per kg body weight, dissolved in saline buffer) 5 days and 1 day before the first sensitization. Subsequently, the i/p injections containing only 1/3 of the dose (0.3 mg/kg) are given one day before and one day after the OVA sensitization, while a dose of 1 mg/kg weight is given on the day of sensitization. From day 15, the complex is given i/n.

Mice of group 4 receive 5 mg/kg of metal-nocardamine complex by i/n only, based on the pattern of administration similar to that of Group 3. Animals in Groups 1 and 2 will receive saline injections and nasal instillations according to the same regime. At the end of the experiments, on day 24, the animals are euthanized with injection of ketamine/xylazine mixture.

Immediately after euthanasia, bronchoalveolar lavage (BAL) with saline solution is performed. BAL fluid is fixed and stained on cytospin slides, and eosinophils and neutrophils in BAL are counted. Following lavage, the lungs are excised and samples from lung tissue are taken for homogenization. Ferritin and ferritin-bound iron is quantified in the homogenate.

Lungs of 5 mice from each group are filled with a 4% paraformaldehyde solution, and sliced longitudinally into 3 parts. The middle third is embedded in paraffin, randomly sliced, and stained with eosin-hematoxylin. The intensity of the peribronchial and perivascular cellular infiltration and mucous content are assessed. Periodic Acid Schiff (PAS) staining is used to assess epithelial cells metaplasia, and Masson's trichrome staining is used to evaluate the presence of fibrous connective tissue.

### Example 9

### The effect of zinc-nocardamine complexes on spinal cord injury model

Contusion spinal cord injury model is studied using Infinite Horizon spinal cord impactor. The injury is performed at force setting of 70 kdyn for 100 ms on exposed T12 vertebra of the anesthetized and immobilized mouse (C57BL 8 weeks old, males). The post-traumatic recovery of mice with or without treatment is assessed on a daily basis for the next 42 days using open field Basso mouse scale (BMS) as follows:
**0** - No ankle movement;
**1** - Slight ankle movement;
**2** - Extensive ankle movement;
**3** -Plantar placing of the paw with or without weight support -OR-Occasional, frequent or consistent dorsal stepping but no plantar stepping;
**4** - Occasional plantar stepping;
**5** - Frequent or consistent plantar stepping, no coordination -OR-Frequent or consistent plantar stepping, some coordination, paws rotated at initial contact and lift off (R/R);
**6** - Frequent or consistent plantar stepping, some coordination, paws parallel at initial contact (P/R, P/P) -OR-Frequent or consistent plantar stepping, mostly coordinated, paws rotated at initial contact and lift off (R/R);
**7** - Frequent or consistent plantar stepping, mostly coordinated, paws parallel at initial contact and rotated at lift off (P/R) -OR-Frequent or consistent plantar stepping, mostly coordinated, paws parallel at initial contact and lift off (P/P), and severe trunk instability;
**8** - Frequent or consistent plantar stepping, mostly coordinated, paws parallel at initial contact and lift off (P/P), and mild trunk instability -OR-Frequent or consistent plantar stepping, mostly coordinated, paws parallel at initial contact and lift off (P/P), and normal trunk stability and tail down or up & down;
**9** - Frequent or consistent plantar stepping, mostly coordinated, paws parallel at initial contact and lift off (P/P), and normal trunk stability and tail always up;
   P - parallel; R - rotated.

### Example 10

### The effect of zinc-nocardamine complexes on Psammomys Obesus model of type II diabetes

Type II diabetes is characterized by insulin resistance and progressive beta-cell failure. Deficient insulin secretion, with increased proportions of insulin precursor molecules, is a common feature of type II diabetes; this could result from inappropriate beta-cell function and/or reduced beta-cell mass. The gerbil *Psammomys obesus* is a good model to address questions related to the role of insulin resistance and beta-cell failure in nutritionally induced diabetes. Upon a change from its natural low-calorie diet to a calorie-rich laboratory food, *P. obesus* develops moderate obesity associated with postprandial hyperglycemia. Once postprandial hyperglycemia develops (nonfasted blood glucose >8.3 mmol/l), progression of diabetes is very rapid, reaching the end-stage of the disease, characterized by severe hypoinsulinemia, hyperlipidemia, and ketosis, within 4-6 weeks of initiating the calorie-rich diet. Hyperglycemia in *P. obesus* is reversible, except for the hypoinsulinemic end stage of the disease; normoglycemia could be re-obtained by limiting the caloric intake. Continued dietary load, superimposed on its innate insulin resistance, results in depletion of pancreatic insulin stores, with increased proportions of insulin precursor molecules in the pancreas and the blood. Inadequate response of the preproinsulin gene to the increased insulin needs is an important cause of diabetes progression. The major culprit is the inappropriate insulin production with depletion of insulin stores as a consequence. Similar mechanisms could operate during the evolution of typeII diabetes in humans. Hyperglycemic gerbils tend to develop microangiopathic complications, resulting in diabetic nephropathy and diabetes-induced cataract.

Thirty two male sand rats (*Psammomys Obesus*), body weight 160 - 190 g, are divided into four groups (n=8 each) - Group 1 - Control animals fed by low-energy diet; Groups 2-4 - Fed on high energy diabetogenic diet. Blood glucose level and body weight are monitored every 3-5 days for 63 days. The animals of Groups 1 & 2 are treated with intraperitoneal injections of 100 µl saline. Animals of Group 3 are treated with zinc-nocardamine (2.5 mg/kg), and Group 4 are treated with gallium-nocardamine (2.5 mg/kg). The treatments are administered, three times per week, for the first two weeks, and twice per week till the end of the experiment.

### Example 11

### The metal-nocardamine complex for the treatment of diabetes using a diabetic mouse model

Encouraged by the beneficial effect of the metal-nocardamine complex of the invention on an inflammatory condition demonstrated using the ICD model, the inventors next examine the potential beneficial effect of the metal-nocardamine complex on another immune-related disorder, using a mouse diabetes models. Thus, the possible use of the metal-nocardamine complex in prophylaxis and/or amelioration of diabetes are next examined using the NOD mouse as a model for diabetes.

The inventors examine the effect of the metal-nocardamine complex using NOD mice by i.v. administering of the metal-nocardamine complexes of the invention on weeks 8 and 12. Control mice receive PBS. Mice are monitored every other week for blood glucose levels and weight.

### Example 12

### The effect of the metal-nocardamine complex on Collagen-Induced Arthritis

The *in vivo* effects of the metal-nocardamine complex are examined using an experimental model of Collagen-Induced Arthritis (CIA) model in mice. Male DBA/1 mice are immunized on day 0 with collagen type II emulsified in CFA to induce arthritis, and a boost dose given on day 21. Most of the mice develop CIA within 2.5 weeks after the boost injection. Upon clinical signs of disease, the mice are treated with a single dose of either the metal-nocardamine complex of the invention or with saline, as the appropriate controls.

### Example 13

### The effect of the metal-nocardamine complex on Established Adjuvant-Induced Arthritis

The *in vivo* effects of a metal-nocardamine complex in an experimental model of established arthritis is examined next. Lewis rats are immunized with MT/CFA on day 0 to induce arthritis; arthritis severity is measured via clinical scoring. On day 14 rats are divided into experimental groups. On day 15, towards the peak of the disease, rats are treated with the metal-nocardamine complex of the invention or with saline as the appropriate control.

### Example 14

### The effect of the metal-nocardamine complex of the invention on TNBS-induced experimental Colitis, an Animal Model of Inflammatory Bowel Disease (IBD)

The effects of the metal-nocardamine complexes of the invention are examined in an additional experimental model of an autoimmune inflammatory disease. The model depicted herein is the TNBS Colitis model, a widely accepted and used animal model of IBD.

Twenty Balb/C mice are sensitized by TNBS skin-painting (designated day -7), followed by intrarectal administration of TNBS, one week later (designated day 0). The metal-nocardamine complex is administered at three time points (-6, -2, +1) via i.p. injection to animals in the treated Group, which are compared with untreated control animals. All the animals are weighed at the time of sensitization, intrarectal administration and at every following day. The animals are sacrificed three days following intrarectal administration (designated day +3) and the colon tissue is removed and subjected to histopathological analysis.

### Example 15

### The metal-nocardamine complex for the treatment of psoriasis in human patient

The human experiments are performed among a sample of 20 patients, 10 male and 10 female, aged 17 to 71. Every form of psoriasis is represented: plaque psoriasis, scalp psoriasis, guttate psoriasis, erythrodermic psoriasis and reversed psoriasis.

The treatment consists of topical applications of the metal-nocardamine complex of the invention using application of a thin layer of the complex on days 1,2,4, 7, 10 and 14. If needed, the treatments are continued for an additional week.

None of the usual treatments for psoriasis are used during this study, in a way that the results obtained can only be attributed to the metal-nocardamine complex/es of the invention.

The treatment is administered as indicated above and smoothening of the treated psoriatic plaques as well as the healing to a normal looking skin are being monitored.

### Example 16

### The metal-nocardamine complex in the treatment and prevention of Experimental Autoimmune Encephalomyelitis (EAE) in mice

To further investigate the effect of the metal-nocardamine complex on immune-related disorders, the EAE as a model for Multiple Sclerosis (MS) is used next. EAE is induced in 8-week-old female C57BL/6 mice by injecting myelin oligodendrocyte glycoprotein 35-55 peptide (MOG35-55) in the first and seventh days of the experiment. Mice are further inoculated with pertussis toxin in the first and second day of experiment as indicated in Experimental Procedures. Four groups of mice (10 mice each) are evaluated for clinical score as detailed in Experimental Procedures. The first group (A) - examining the potential preventive and protective effect of the metal-nocardamine complex, receives weekly treatment, three times per week, of the metal-nocardamine complex. The treatment is initiated three days prior to MOG injection and terminated on day 37, and is followed by treatment with vehicle from day 37 to day 52. Mice of the second group (B) are treated weekly, 3 times per week, with the metal-nocardamine complex starting on the seventh day following MOG injection, throughout the end of the experiment at day 52. The third group (C), receives weekly treatment, 3 time per week, with the metal-nocardamine complex initiated on the first day throughout the end of the experiment at day 52. The control group (D) is treated with saline, as the appropriate control, starting on day 1 throughout the end of the experiment at day 52. The mice are clinically scored according to the criteria set forth in the Experimental Procedures.

### Example 17

### The metal-nocardamine complex for the treatment of Herpes (Cold Sores)

The trial is performed on 8 patients (4 male and 4 females), aged 28 - 38. Herpes simplex virus (HSV) manifestations (herpes labialis) are treated in all patients, by a topical application of an ointment containing the zinc-nocardamine complex (0.5%), of the invention, twice a day, on the lips, for three consecutive days (altogether 6 administrations). No other drug (agent) is used in parallel with the application of the zinc-nocardamine complex ointment, of the invention.

## Claims

1. An effective amount of at least one complex of nocardamine (desferrioxamine E) with at least one metal, any combination/s of said complexes, any pharmaceutical composition/s of the complexes comprising any carrier/s, matrix or vehicle/s, for use in a method for modulating cytokine levels in a subject, wherein said subject is suffering from a disorder associated with at least one of elevated level/s of at least one pro-inflammatory cytokine and reduced level/s of at least one anti-inflammatory cytokine, and wherein said disorder is at least one of inflammatory, infectious, autoimmune, proliferative, ischemic, metabolic, neuro-degenerative disorder, spinal cord injury and trauma, and acute or chronic wound or injury, wherein said complex is a complex of nocardamine with Zn²⁺ion or a complex of nocardamine with Ga³⁺ion.

2. The complex or composition for use according to claim 1, wherein said modulation is at least one of reduction or elevation of cytokine levels in said subject.

3. The complex or composition for use according to claim 2, wherein said cytokine is at least one of: at least one pro-inflammatory cytokine and at least one anti-inflammatory cytokine.

4. The complex or composition for use according to any one of claims 1 to 3, for at least one of:
(a) reducing the level of at least one pro-inflammatory cytokine in said subject, wherein said pro-inflammatory cytokine is at least one of IL-1α, IL-6, TNF-α and IL-17; and
(b) elevating the level of at least one anti-inflammatory cytokine in said subject, wherein said anti-inflammatory cytokine each independently is at least one of IL-13, IL-4, and IL-10.

5. A composition comprising at least one complex of nocardamine with zinc ion (Zn²⁺), said composition optionally further comprises at least one of diluent/s, carrier/s, excipient/s.

6. A kit comprising:
(i) at least one metal selected from lanthanide/s, actinide/s, post-transition metal/s or transition metal/s, or any combinations of said complexes, in any form of salts, esters and amides thereof, or a pharmaceutically acceptable derivative thereof and a pharmaceutically acceptable carrier or diluent, wherein at least one of said metals is a zinc ion (Zn²⁺); and
(ii) nocardamine.

## Patentansprüche

1. Wirksame Menge von wenigstens einem Komplex von Nocardamin (Desferrioxamin E) mit wenigstens einem Metall, (einer) beliebigen Kombination(en) der Komplexe, (einer) beliebigen pharmazeutischen Zusammensetzung(en) der Komplexe, die (eine) beliebige Trägersubstanz(en), eine beliebige Matrix oder (einen) beliebige(n) Trägerstoff(e) umfassen, zur Verwendung in einem Verfahren zum Modulieren von Zytokinspiegeln in einem Subjekt, wobei das Subjekt an einer Erkrankung leidet, die mit (einem) erhöhten Spiegel(n) von wenigstens einem proinflammatorischen Zytokin und/oder (einem) reduzierten Spiegel(n) von wenigstens einem antiinflammatorischen Zytokin im Zusammenhang steht, und wobei die Erkrankung eine inflammatorische, infektiöse, autoimmune, proliferative, ischämische, metabolische, neurodegenerative Erkrankung, eine Rückenmarksverletzung, ein Trauma und/oder eine akute oder chronische Wunde oder Verletzung ist, wobei der Komplex ein Komplex von Nocardamin mit einem Zn²⁺-Ion oder ein Komplex von Nocardamin mit einem Ga³⁺-Ion ist.

2. Komplex oder Zusammensetzung zur Verwendung nach Anspruch 1, wobei die Modulation eine Reduzierung und/oder eine Erhöhung der Zytokinspiegel in dem Subjekt ist.

3. Komplex oder Zusammensetzung zur Verwendung nach Anspruch 2, wobei das Zytokin Folgendes ist: wenigstens ein proinflammatorisches Zytokin und/oder wenigstens ein antiinflammatorisches Zytokin.

4. Komplex oder Zusammensetzung zur Verwendung nach einem der Ansprüche 1 bis 3 für Folgendes:
(a) Reduzieren des Spiegels von wenigstens einem proinflammatorischen Zytokin in dem Subjekt, wobei das proinflammatorische Zytokin IL-1α, IL-6, TNF-α und/oder IL-17 ist; und/oder
(b) Erhöhen des Spiegels von wenigstens einem antiinflammatorischen Zytokin in dem Subjekt, wobei das antiinflammatorische Zytokin jeweils unabhängig IL-13, IL-4 und/oder IL-10 ist.

5. Zusammensetzung, die wenigstens einen Komplex von Nocardamin mit einem Zinkion (Zn²⁺) umfasst, wobei die Zusammensetzung optional ferner (ein) Verdünnungsmittel, (eine) Trägersubstanz(en) und/oder (einen) Arzneistoffträger umfasst.

6. Kit, das Folgendes umfasst:
(i) wenigstens ein Metall, das aus Lanthanid(en), Actinid(en), Nachübergangsmetall(en) oder Übergangsmetall(en) oder beliebigen Kombinationen der Komplexe in beliebiger Form von Salzen, Estern und Amiden davon oder einem pharmazeutisch unbedenklichen Derivat davon und einer pharmazeutisch unbedenklichen Trägersubstanz oder einem pharmazeutisch unbedenklichen Verdünnungsmittel ausgewählt ist, wobei wenigstens eines der Metalle ein Zinkion (Zn²⁺) ist; und
(ii) Nocardamin.

## Revendications

1. Quantité efficace d'au moins un complexe de nocardamine (desferrioxamine E) avec au moins un métal, toute combinaison desdits complexes, toute composition pharmaceutique des complexes comprenant tout support, matrice ou véhicule, à utiliser dans un procédé permettant de moduler les taux de cytokines chez un sujet, ledit sujet souffrant d'un trouble associé à des taux élevés d'au moins une cytokine pro-inflammatoire et/ou des taux réduits d'au moins une cytokine anti-inflammatoire, et ledit trouble étant un trouble inflammatoire et/ou infectieux et/ou auto-immun et/ou prolifératif et/ou ischémique et/ou métabolique et/ou neuro-dégénératif et/ou une lésion et un traumatisme de la moelle épinière et/ou une plaie ou blessure aiguë ou chronique, ledit complexe étant un complexe de nocardamine avec l'ion Zn²⁺ ou un complexe de nocardamine avec l'ion Ga³⁺.

2. Complexe ou composition à utiliser selon la revendication 1, ladite modulation étant la réduction et/ou l'élévation des taux de cytokines chez ledit sujet.

3. Complexe ou composition à utiliser selon la revendication 2, ladite cytokine étant :
au moins une cytokine pro-inflammatoire et/ou au moins une cytokine anti-inflammatoire.

4. Complexe ou composition à utiliser selon l'une quelconque des revendications 1 à 3, pour :
(a) la réduction du taux d'au moins une cytokine pro-inflammatoire chez ledit sujet, ladite cytokine pro-inflammatoire étant IL-la et/ou IL-6 et/ou TNF-α et/ou IL-17 ; et/ou
(b) l'élévation du niveau d'au moins une cytokine anti-inflammatoire chez ledit sujet, ladite cytokine anti-inflammatoire étant chacune indépendamment IL-13 et/ou IL-4 et/ou IL-10.

5. Composition comprenant au moins un complexe de nocardamine avec l'ion zinc (Zn²⁺), ladite composition comprend en outre facultativement un ou des diluant(s) et/ou un ou des support(s) et/ou un ou des excipient(s).

6. Trousse comprenant :
(i) un métal choisi parmi les lanthanides et/ou les actinides et/ou les métaux post-transition et/ou les métaux de transition, ou toute combinaison desdits complexes, sous toute forme de sels, esters et amides de ceux-ci, ou un de leurs dérivés pharmaceutiquement acceptables et un support ou diluant pharmaceutiquement acceptable, au moins l'un desdits métaux étant un ion zinc (Zn²⁺) ; et
(ii) de la nocardamine.
